(19) Europäisches Patentamt / European Patent Office / Office européen des brevets

(11) **EP 3 390 452 B1**

(12) **FASCICULE DE BREVET EUROPEEN**

(45) Date de publication et mention de la délivrance du brevet:
**26.05.2021 Bulletin 2021/21**

(21) Numéro de dépôt: **16826758.1**

(22) Date de dépôt: **16.12.2016**

(51) Int Cl.:
***C07K 16/28*** *(2006.01)* ***A61P 35/00*** *(2006.01)*

(86) Numéro de dépôt international:
**PCT/FR2016/053504**

(87) Numéro de publication internationale:
**WO 2017/103521 (22.06.2017 Gazette 2017/25)**

(54) **NOUVELLE UTILISATION D'UN ANTICORPS DIRIGE CONTRE LA PROTEINE MEMBRANAIRE CD303**

NEUE VERWENDUNG EINES ANTI-CD303-TRANSMEMBRANPROTEIN-ANTIKÖRPERS

NEW USE OF AN ANTI-CD303 TRANSMEMBRANE PROTEIN ANTIBODY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.12.2015 FR 1562545**

(43) Date de publication de la demande:
**24.10.2018 Bulletin 2018/43**

(73) Titulaire: **Laboratoire Français du Fractionnement et des Biotechnologies**
**91940 Les Ulis (FR)**

(72) Inventeurs:
- **CHTOUROU, Abdessatar Sami**
  **78990 Elancourt (FR)**
- **FOURNIER, Nathalie**
  **59193 Erquinghem-Lys (FR)**

(74) Mandataire: **Plasseraud IP**
**66, rue de la Chaussée d'Antin**
**75440 Paris Cedex 09 (FR)**

(56) Documents cités:
**WO-A1-2009/138489 WO-A1-2012/080642**
**WO-A1-2015/095143 WO-A2-01/36487**

- **JAHN P S ET AL: "BDCA-2 signaling inhibits TLR-9-agonist-induced plasmacytoid dendritic cell activation and antigen presentation", CELLULAR IMMUNOLOGY, ACADEMIC PRESS, SAN DIEGO, CA, US, vol. 265, no. 1, 1 janvier 2010 (2010-01-01), pages 15-22, XP027236570, ISSN: 0008-8749 [extrait le 2010-07-06]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

SEQUENCE LISTING

<110> LABORATOIRE FRANCAIS DU FRACTIONNEMENT ET DES BIOTECHNOLOGIES

<120> NOUVELLE UTILISATION D'UN ANTICORPS DIRIGE CONTRE UNE PROTEINE MEMBRANAIRE

<130> WOB 15 CL LFB PDCS

<150> FR15/62545
<151> 2015-12-16

<160> 130

<170> PatentIn version 3.5

<210> 1
<211> 8
<212> PRT
<213> artificial

<220>
<223> CDR1-H-famille 1

<400> 1

```
Gly Tyr Thr Phe Thr Asp Tyr Ser
1               5
```

<210> 2

<211> 8
<212> PRT
<213> artificial

<220>
<223> CDR2-H-famille 1

<220>
<221> misc_feature
<222> (3)..(3)
<223> Xaa can be any naturally occurring amino acid

<220>
<221> misc_feature
<222> (8)..(8)
<223> Xaa can be any naturally occurring amino acid

<400> 2

```
Ile Ser Xaa Tyr Tyr Gly Asp Xaa
1               5
```

<210> 3
<211> 11
<212> PRT
<213> artificial

<220>
<223> CDR3-H-famille 1

<220>
<221> misc_feature
<222> (4)..(6)
<223> Xaa can be any naturally occurring amino acid

<220>
<221> misc_feature
<222> (8)..(10)
<223> Xaa can be any naturally occurring amino acid

<400> 3

```
Ala Arg Asn Xaa Xaa Xaa Tyr Xaa Xaa Xaa Tyr
1               5                   10
```

<210> 4
<211> 6
<212> PRT
<213> artificial

<220>
<223> CDR1-L-famille 1

<220>
<221> misc_feature
<222> (4)..(4)
<223> Xaa can be any naturally occurring amino acid

<400> 4

Gln Asp Ile Xaa Asn Tyr
1 5

<210> 5
<211> 3
<212> PRT
<213> artificial

<220>
<223> CDR2-L-famille 1

<400> 5

Tyr Thr Ser
1

<210> 6
<211> 9
<212> PRT
<213> artificial

<220>
<223> CDR3-L-famille 1

<220>
<221> misc_feature
<222> (4)..(4)
<223> Xaa can be any naturally occurring amino acid

<400> 6

Gln Gln Gly Xaa Thr Leu Pro Trp Thr
1 5

<210> 7
<211> 8
<212> PRT
<213> artificial

<220>
<223> CDR1-H-famille 2

<220>
<221> misc_feature
<222> (7)..(7)
<223> Xaa can be any naturally occurring amino acid

<400> 7

Gly Tyr Thr Phe Thr Asp Xaa Ser
1 5

<210> 8
<211> 8
<212> PRT
<213> artificial

<220>
<223> CDR2-H-famille 2

<220>
<221> misc_feature
<222> (7)..(7)
<223> Xaa can be any naturally occurring amino acid

<400> 8

```
Ile Asn Thr Glu Thr Gly Xaa Pro
1               5
```

<210> 9
<211> 14
<212> PRT
<213> artificial

<220>
<223> CDR3-H-famille 2

<220>
<221> misc_feature
<222> (1)..(1)
<223> Xaa can be any naturally occurring amino acid

<220>
<221> misc_feature
<222> (12)..(12)
<223> Xaa can be any naturally occurring amino acid

<400> 9

```
Xaa Arg Asn Gly Tyr Tyr Val Gly Tyr Tyr Ala Xaa Asp Tyr
1               5                   10
```

<210> 10
<211> 5
<212> PRT
<213> artificial

<220>
<223> CDR1-L-famille 2

<220>
<221> misc_feature
<222> (4)..(4)
<223> Xaa can be any naturally occurring amino acid

<400> 10

```
Ser Ser Val Xaa Tyr
1               5
```

<210> 11
<211> 3
<212> PRT
<213> artificial

<220>
<223> CDR2-L-famille 2

<400> 11

Ser Thr Ser
1

<210> 12
<211> 9
<212> PRT
<213> artificial

<220>
<223> CDR3-L-famille 2

<220>
<221> misc_feature
<222> (8)..(8)
<223> Xaa can be any naturally occurring amino acid

<400> 12

Gln Gln Arg Arg Ser Tyr Pro Xaa Thr
1 5

<210> 13
<211> 8
<212> PRT
<213> artificial

<220>
<223> CDR1-H-122A2

<400> 13

Gly Tyr Thr Phe Thr Asp Tyr Ser
1 5

<210> 14
<211> 8
<212> PRT
<213> artificial

<220>
<223> CDR2-H-122A2

<400> 14

Ile Ser Thr Tyr Tyr Gly Asp Ser
1 5

<210> 15
<211> 11
<212> PRT
<213> artificial

<220>

<223> CDR3-H-122A2

<400> 15

Ala Arg Asn Gly Asn Phe Tyr Val Met Asp Tyr
1 5 10

<210> 16
<211> 6
<212> PRT
<213> artificial

<220>
<223> CDR1-L-122A2

<400> 16

Gln Asp Ile Ser Asn Tyr
1 5

<210> 17
<211> 3
<212> PRT
<213> artificial

<220>
<223> CDR2-L-122A2

<400> 17

Tyr Thr Ser
1

<210> 18
<211> 9
<212> PRT
<213> artificial

<220>
<223> CDR3-L-122A2

<400> 18

Gln Gln Gly Asn Thr Leu Pro Trp Thr
1 5

<210> 19
<211> 8
<212> PRT
<213> artificial

<220>
<223> CDR1-H-102E9

<400> 19

Gly Tyr Thr Phe Thr Asp Tyr Ser
1 5

<210> 20
<211> 8
<212> PRT
<213> artificial

<220>
<223> CDR2-H-102E9

<400> 20

Ile Asn Thr Glu Thr Gly Glu Pro
1               5

<210> 21
<211> 14
<212> PRT
<213> artificial

<220>
<223> CDR3-H-102E9

<400> 21

Thr Arg Asn Gly Tyr Tyr Val Gly Tyr Tyr Ala Met Asp Tyr
1               5                   10

<210> 22
<211> 5
<212> PRT
<213> artificial

<220>
<223> CDR1-L-102E9

<400> 22

Ser Ser Val Ile Tyr
1               5

<210> 23
<211> 3
<212> PRT
<213> artificial

<220>
<223> CDR2-L-102E9

<400> 23

Ser Thr Ser
1

<210> 24
<211> 9
<212> PRT
<213> artificial

<220>

<223> CDR3-L-102E9

<400> 24

Gln Gln Arg Arg Ser Tyr Pro Phe Thr
1 5

<210> 25
<211> 8
<212> PRT
<213> artificial

<220>
<223> CDR1-H-104C12

<400> 25

Gly Tyr Thr Phe Thr Asp Tyr Ser
1 5

<210> 26
<211> 8
<212> PRT
<213> artificial

<220>
<223> CDR2-H-104C12

<400> 26

Ile Ser Pro Tyr Tyr Gly Asp Thr
1 5

<210> 27
<211> 11
<212> PRT
<213> artificial

<220>
<223> CDR3-H-104C12

<400> 27

Ala Arg Asn Asp Asp Tyr Tyr Arg Phe Ala Tyr
1 5 10

<210> 28
<211> 6
<212> PRT
<213> artificial

<220>
<223> CDR1-L-104C12

<400> 28

Gln Asp Ile Asn Asn Tyr
1 5

<210> 29
<211> 3
<212> PRT
<213> artificial

<220>
<223> CDR2-L-104C12

<400> 29

Tyr Thr Ser
1

<210> 30
<211> 9
<212> PRT
<213> artificial

<220>
<223> CDR3-L-104C12

<400> 30

Gln Gln Gly Lys Thr Leu Pro Trp Thr
1               5

<210> 31
<211> 8
<212> PRT
<213> artificial

<220>
<223> CDR1-H-114D11

<400> 31

Gly Tyr Thr Phe Thr Asp Ser Ser
1               5

<210> 32
<211> 8
<212> PRT
<213> artificial

<220>
<223> CDR2-H-114D11

<400> 32

Ile Asn Thr Glu Thr Gly Gly Pro
1               5

<210> 33
<211> 14
<212> PRT
<213> artificial

<220>

<223> CDR3-H-114D11

<400> 33

Ala Arg Asn Gly Tyr Tyr Val Gly Tyr Tyr Ala Leu Asp Tyr
1 5 10

<210> 34
<211> 5
<212> PRT
<213> artificial

<220>
<223> CDR1-L-114D11

<400> 34

Ser Ser Val Phe Tyr
1 5

<210> 35
<211> 3
<212> PRT
<213> artificial

<220>
<223> CDR2-L-114D11

<400> 35

Ser Thr Ser
1

<210> 36
<211> 9
<212> PRT
<213> artificial

<220>
<223> CDR3-L-114D11

<400> 36

Gln Gln Arg Arg Ser Tyr Pro Tyr Thr
1 5

<210> 37
<211> 8
<212> PRT
<213> artificial

<220>
<223> CDR1-H-104E10

<400> 37

Gly Tyr Thr Phe Thr Asp Tyr Ser
1 5

<210> 38
<211> 8
<212> PRT
<213> artificial

<220>
<223> CDR2-H-104E10

<400> 38

```
Ile Asn Thr Glu Thr Gly Glu Pro
1               5
```

<210> 39
<211> 14
<212> PRT
<213> artificial

<220>
<223> CDR3-H-104E10

<400> 39

```
Ala Arg Asn Gly Tyr Tyr Val Gly Tyr Tyr Ala Met Asp Tyr
1               5                   10
```

<210> 40
<211> 5
<212> PRT
<213> artificial

<220>
<223> CDR1-L-104E10

<400> 40

```
Ser Ser Val Ile Tyr
1               5
```

<210> 41
<211> 3
<212> PRT
<213> artificial

<220>
<223> CDR2-L-104E10

<400> 41

```
Ser Thr Ser
1
```

<210> 42
<211> 9
<212> PRT
<213> artificial

<220>

<223> CDR3-L-104E10

<400> 42

Gln Gln Arg Arg Ser Tyr Pro Tyr Thr
1 5

<210> 43
<211> 118
<212> PRT
<213> artificial

<220>
<223> VH-122A2

<400> 43

Gln Val Gln Leu Gln Gln Ser Gly Ala Glu Leu Val Arg Pro Gly Val
1 5 10 15

Ser Val Lys Ile Ser Cys Lys Gly Ser Gly Tyr Thr Phe Thr Asp Tyr
20 25 30

Ser Met His Trp Val Lys Gln Ser His Ala Lys Ser Leu Glu Trp Ile
35 40 45

Gly Val Ile Ser Thr Tyr Tyr Gly Asp Ser Asn Tyr Asn Gln Lys Phe
50 55 60

Lys Gly Lys Ala Thr Met Thr Val Asp Lys Ser Ser Thr Thr Ala Tyr
65 70 75 80

Met Glu Leu Ala Arg Leu Thr Ser Glu Asp Ser Ala Ile Tyr Tyr Cys
85 90 95

Ala Arg Asn Gly Asn Phe Tyr Val Met Asp Tyr Trp Gly Gln Gly Thr
100 105 110

Ser Val Thr Val Ser Ser
115

<210> 44
<211> 121
<212> **PRT**
<213> artificial

<220>
<223> VH-102E9

<400> 44

```
Gln Ile His Leu Val Gln Ser Gly Pro Asp Leu Lys Lys Pro Gly Glu
1               5                   10                  15

Thr Val Lys Ile Ser Cys Lys Ala Ser Gly Tyr Thr Phe Thr Asp Tyr
            20                  25                  30

Ser Met His Trp Val Lys Gln Ala Pro Gly Lys Gly Leu Lys Trp Met
        35                  40                  45

Gly Trp Ile Asn Thr Glu Thr Gly Glu Pro Thr Tyr Ala Asp Asp Phe
    50                  55                  60

Lys Gly Arg Phe Ala Phe Ser Leu Glu Ser Ser Ala Ser Thr Ala Phe
65                  70                  75                  80

Leu Gln Ile Asn Asn Leu Lys Asn Glu Asp Thr Ser Thr Tyr Phe Cys
                85                  90                  95

Thr Arg Asn Gly Tyr Tyr Val Gly Tyr Tyr Ala Met Asp Tyr Trp Gly
            100                 105                 110

Gln Gly Thr Ser Val Thr Val Ser Ser
        115                 120
```

<210> 45
<211> 118
<212> PRT
<213> artificial

<220>
<223> VH-104C12

<400> 45

Gln Val Gln Leu Gln Gln Ser Gly Ala Glu Leu Val Gly Pro Gly Val
1               5                   10                  15

Ser Val Lys Ile Ser Cys Lys Gly Ser Gly Tyr Thr Phe Thr Asp Tyr
            20                  25                  30

Ser Met His Trp Val Lys Gln Ser His Ala Lys Ser Leu Glu Trp Ile
        35                  40                  45

Gly Val Ile Ser Pro Tyr Tyr Gly Asp Thr Asn Tyr Asn Gln Lys Phe
    50                  55                  60

Lys Gly Lys Ala Thr Met Thr Val Asp Lys Ser Ser Ser Thr Ala Tyr
65                  70                  75                  80

Met Glu Leu Ala Ser Leu Thr Ser Glu Asp Ser Ala Ile Tyr Phe Cys
                85                  90                  95

Ala Arg Asn Asp Asp Tyr Tyr Arg Phe Ala Tyr Trp Gly Gln Gly Thr
            100                 105                 110

Leu Val Thr Val Ser Ala
        115

<210> 46
<211> 121
<212> PRT
<213> artificial

<220>
<223> VH-114D11

<400> 46

Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Thr Phe Thr Asp Ser
            20                  25                  30

Ser Met His Trp Val Gln Gln Ala Pro Asn Lys Gly Leu Lys Trp Met

```
            35                  40                  45

Gly Trp Ile Asn Thr Glu Thr Gly Gly Pro Thr Tyr Ala Asp Asp Phe
    50                  55                  60

Lys Gly Arg Phe Ala Phe Ser Leu Glu Thr Ser Ala Arg Thr Ala Tyr
65                  70                  75                  80

Leu Gln Ile Asn Asn Leu Lys Asn Glu Asp Thr Ala Thr Tyr Phe Cys
                85                  90                  95

Ala Arg Asn Gly Tyr Tyr Val Gly Tyr Tyr Ala Leu Asp Tyr Trp Gly
            100                 105                 110

Gln Gly Thr Ser Val Thr Val Ser Ser
        115                 120
```

<210> 47
<211> 121
<212> PRT
<213> artificial

<220>
<223> VH-104E10

<400> 47

Gln Ile Gln Leu Val Gln Ser Gly Pro Glu Leu Lys Lys Pro Gly Glu
1 5 10 15

Thr Val Lys Ile Ser Cys Lys Ala Ser Gly Tyr Thr Phe Thr Asp Tyr
20 25 30

Ser Met His Trp Val Lys Gln Ala Pro Gly Lys Gly Leu Lys Trp Met
35 40 45

Gly Trp Ile Asn Thr Glu Thr Gly Glu Pro Thr Tyr Ala Asp Asp Phe
50 55 60

Lys Gly Arg Phe Ala Phe Ser Leu Glu Thr Ser Ala Thr Thr Ala Tyr
65 70 75 80

Leu Gln Ile Asn Asn Phe Lys Asn Glu Asp Thr Ala Thr Tyr Phe Cys
85 90 95

Ala Arg Asn Gly Tyr Tyr Val Gly Tyr Tyr Ala Met Asp Tyr Trp Gly
100 105 110

Gln Gly Thr Ser Val Thr Val Ser Ser
115 120

<210> 48
<211> 107
<212> PRT
<213> artificial

<220>
<223> VL-122A2

<400> 48

```
Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Ser Asn Tyr
            20                  25                  30

Leu Asn Trp Tyr Gln Gln Lys Pro Asp Gly Thr Val Lys Leu Leu Ile
        35                  40                  45

Tyr Tyr Thr Ser Arg Leu His Ser Gly Val Pro Ser Arg Phe Ser Gly
    50                  55                  60

Ser Gly Ser Gly Thr Asp Tyr Ser Leu Thr Ile Ser Asn Leu Asp Gln
65                  70                  75                  80

Glu Asp Ile Ala Thr Tyr Phe Cys Gln Gln Gly Asn Thr Leu Pro Trp
                85                  90                  95

Thr Phe Gly Gly Gly Thr Lys Leu Glu Ile Lys
            100                 105
```

<210> 49
<211> 106
<212> PRT
<213> artificial

<220>
<223> VL-102E9

<400> 49

```
Gln Ile Val Leu Thr Gln Ser Pro Ala Ile Met Ser Ala Ser Pro Gly
1               5                   10                  15

Glu Lys Val Thr Ile Thr Cys Ser Ala Ser Ser Ser Val Ile Tyr Ile
            20                  25                  30

His Trp Phe Gln Gln Lys Pro Gly Thr Ser Pro Lys Leu Trp Ile Tyr
        35                  40                  45

Ser Thr Ser Tyr Leu Ala Ser Gly Val Pro Ala Arg Phe Ser Gly Ser
```

50 55 60

Gly Ser Gly Thr Ser Tyr Ser Leu Thr Ile Ser Arg Met Glu Ala Glu
65 70 75 80

Asp Ala Ala Thr Tyr Tyr Cys Gln Gln Arg Arg Ser Tyr Pro Phe Thr
85 90 95

Phe Gly Gly Gly Thr Lys Leu Glu Ile Lys
100 105

<210> 50
<211> 107
<212> PRT
<213> artificial

<220>
<223> VL-104C12

<400> 50

Asp Leu Gln Met Thr Gln Thr Pro Ser Ser Leu Ser Ala Ser Leu Gly
1 5 10 15

Asp Arg Val Thr Ile Ser Cys Arg Ala Ser Gln Asp Ile Asn Asn Tyr
20 25 30

Leu Ser Trp Tyr Gln Glu Lys Pro Asp Gly Thr Phe Lys Leu Leu Ile
35 40 45

Tyr Tyr Thr Ser Arg Leu His Ser Gly Val Pro Ser Arg Phe Ser Gly
50 55 60

Ser Gly Ser Gly Thr Asp Tyr Ser Leu Thr Val Arg Asn Leu Glu Gln
65 70 75 80

Glu Asp Ile Gly Thr Tyr Phe Cys Gln Gln Gly Lys Thr Leu Pro Trp
85 90 95

Thr Phe Gly Gly Gly Thr Lys Leu Glu Ile Arg
100 105

<210> 51
<211> 106
<212> PRT
<213> artificial

<220>
<223> VL-114D11

<400> 51

```
Gln Ile Val Leu Thr Gln Ser Pro Ala Ile Met Ser Ala Ser Pro Gly
1               5                   10                  15

Glu Lys Val Thr Ile Thr Cys Ser Ala Ser Ser Ser Val Phe Tyr Met
            20                  25                  30

His Trp Phe Gln Gln Lys Pro Gly Thr Ser Pro Lys Leu Trp Ile Tyr
        35                  40                  45

Ser Thr Ser Asn Leu Ala Ser Gly Val Pro Ala Arg Phe Ser Gly Ser
    50                  55                  60

Gly Ser Gly Thr Ser Tyr Ser Leu Thr Ile Ser Arg Met Glu Ala Glu
65                  70                  75                  80

Asp Ala Ala Thr Tyr Tyr Cys Gln Gln Arg Arg Ser Tyr Pro Tyr Thr
                85                  90                  95

Phe Gly Gly Gly Thr Lys Leu Glu Ile Lys
            100                 105
```

<210> 52
<211> 106
<212> PRT
<213> artificial

<220>
<223> VL-104E10

<400> 52

```
Gln Ile Val Leu Thr Gln Ser Pro Ala Ile Met Ser Ala Ser Pro Gly
1               5                   10                  15

Glu Lys Val Thr Met Thr Cys Ser Ala Ser Ser Ser Val Ile Tyr Met
            20                  25                  30

His Trp Phe Gln Gln Lys Pro Gly Thr Ser Pro Lys Leu Trp Ile Tyr
        35                  40                  45

Ser Thr Ser Asn Leu Ala Ser Gly Val Pro Ala Arg Phe Ser Gly Ser
    50                  55                  60

Gly Ser Gly Thr Ser Tyr Ser Leu Thr Ile Ser Arg Met Glu Ala Glu
65                  70                  75                  80

Asp Ala Ala Thr Tyr Tyr Cys Gln Gln Arg Arg Ser Tyr Pro Tyr Thr
                85                  90                  95

Phe Gly Gly Gly Thr Lys Leu Glu Ile Lys

                    100                 105
```

<210> 53
<211> 329
<212> PRT
<213> artificial

<220>
<223> CH

<400> 53

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205
```

```
Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly
                325
```

<210> 54
<211> 107
<212> PRT
<213> artificial

<220>
<223> CL

<400> 54

```
Arg Thr Val Ala Ala Pro Ser Val Phe Ile Phe Pro Pro Ser Asp Glu
1               5                   10                  15

Gln Leu Lys Ser Gly Thr Ala Ser Val Val Cys Leu Leu Asn Asn Phe
            20                  25                  30

Tyr Pro Arg Glu Ala Lys Val Gln Trp Lys Val Asp Asn Ala Leu Gln
        35                  40                  45

Ser Gly Asn Ser Gln Glu Ser Val Thr Glu Gln Asp Ser Lys Asp Ser
    50                  55                  60

Thr Tyr Ser Leu Ser Ser Thr Leu Thr Leu Ser Lys Ala Asp Tyr Glu
65                  70                  75                  80
```

Lys His Lys Val Tyr Ala Cys Glu Val Thr His Gln Gly Leu Ser Ser
85 90 95

Pro Val Thr Lys Ser Phe Asn Arg Gly Glu Cys
100 105

<210> 55
<211> 447
<212> PRT
<213> artificial

<220>
<223> VH-CH-122A2

<400> 55

Gln Val Gln Leu Gln Gln Ser Gly Ala Glu Leu Val Arg Pro Gly Val
1 5 10 15

Ser Val Lys Ile Ser Cys Lys Gly Ser Gly Tyr Thr Phe Thr Asp Tyr
20 25 30

Ser Met His Trp Val Lys Gln Ser His Ala Lys Ser Leu Glu Trp Ile
35 40 45

Gly Val Ile Ser Thr Tyr Tyr Gly Asp Ser Asn Tyr Asn Gln Lys Phe
50 55 60

Lys Gly Lys Ala Thr Met Thr Val Asp Lys Ser Ser Thr Thr Ala Tyr
65 70 75 80

Met Glu Leu Ala Arg Leu Thr Ser Glu Asp Ser Ala Ile Tyr Tyr Cys
85 90 95

Ala Arg Asn Gly Asn Phe Tyr Val Met Asp Tyr Trp Gly Gln Gly Thr
100 105 110

Ser Val Thr Val Ser Ser Ala Ser Thr Lys Gly Pro Ser Val Phe Pro
115 120 125

Leu Ala Pro Ser Ser Lys Ser Thr Ser Gly Gly Thr Ala Ala Leu Gly
130 135 140

Cys Leu Val Lys Asp Tyr Phe Pro Glu Pro Val Thr Val Ser Trp Asn
145 150 155 160

Ser Gly Ala Leu Thr Ser Gly Val His Thr Phe Pro Ala Val Leu Gln
165 170 175

```
Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430
```

Leu His Asn His Tyr Thr Gln Lys Ser Leu Ser Leu Ser Pro Gly
435 440 445

<210> 56
<211> 450
<212> PRT
<213> artificial

<220>
<223> VH-CH-102E9

<400> 56

Gln Ile His Leu Val Gln Ser Gly Pro Asp Leu Lys Lys Pro Gly Glu
1 5 10 15

Thr Val Lys Ile Ser Cys Lys Ala Ser Gly Tyr Thr Phe Thr Asp Tyr
20 25 30

Ser Met His Trp Val Lys Gln Ala Pro Gly Lys Gly Leu Lys Trp Met
35 40 45

Gly Trp Ile Asn Thr Glu Thr Gly Glu Pro Thr Tyr Ala Asp Asp Phe
50 55 60

Lys Gly Arg Phe Ala Phe Ser Leu Glu Ser Ser Ala Ser Thr Ala Phe
65 70 75 80

Leu Gln Ile Asn Asn Leu Lys Asn Glu Asp Thr Ser Thr Tyr Phe Cys
85 90 95

Thr Arg Asn Gly Tyr Tyr Val Gly Tyr Tyr Ala Met Asp Tyr Trp Gly
100 105 110

Gln Gly Thr Ser Val Thr Val Ser Ser Ala Ser Thr Lys Gly Pro Ser
115 120 125

Val Phe Pro Leu Ala Pro Ser Ser Lys Ser Thr Ser Gly Gly Thr Ala
130 135 140

Ala Leu Gly Cys Leu Val Lys Asp Tyr Phe Pro Glu Pro Val Thr Val
145 150 155 160

Ser Trp Asn Ser Gly Ala Leu Thr Ser Gly Val His Thr Phe Pro Ala
165 170 175

Val Leu Gln Ser Ser Gly Leu Tyr Ser Leu Ser Ser Val Val Thr Val
180 185 190

```
Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445
```

Pro Gly
450

<210> 57
<211> 447
<212> PRT
<213> artificial

<220>
<223> VH-CH-104C12

<400> 57

```
Gln Val Gln Leu Gln Gln Ser Gly Ala Glu Leu Val Gly Pro Gly Val
1               5                   10                  15

Ser Val Lys Ile Ser Cys Lys Gly Ser Gly Tyr Thr Phe Thr Asp Tyr
            20                  25                  30

Ser Met His Trp Val Lys Gln Ser His Ala Lys Ser Leu Glu Trp Ile
        35                  40                  45

Gly Val Ile Ser Pro Tyr Tyr Gly Asp Thr Asn Tyr Asn Gln Lys Phe
    50                  55                  60

Lys Gly Lys Ala Thr Met Thr Val Asp Lys Ser Ser Ser Thr Ala Tyr
65                  70                  75                  80

Met Glu Leu Ala Ser Leu Thr Ser Glu Asp Ser Ala Ile Tyr Phe Cys
                85                  90                  95

Ala Arg Asn Asp Asp Tyr Tyr Arg Phe Ala Tyr Trp Gly Gln Gly Thr
            100                 105                 110

Leu Val Thr Val Ser Ala Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190
```

```
Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly
        435                 440                 445
```

<210> 58
<211> 450
<212> PRT
<213> artificial

<220>
<223> VH-CH-114D11

<400> 58

```
Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Thr Phe Thr Asp Ser
            20                  25                  30

Ser Met His Trp Val Gln Gln Ala Pro Asn Lys Gly Leu Lys Trp Met
        35                  40                  45

Gly Trp Ile Asn Thr Glu Thr Gly Gly Pro Thr Tyr Ala Asp Asp Phe
    50                  55                  60

Lys Gly Arg Phe Ala Phe Ser Leu Glu Thr Ser Ala Arg Thr Ala Tyr
65                  70                  75                  80

Leu Gln Ile Asn Asn Leu Lys Asn Glu Asp Thr Ala Thr Tyr Phe Cys
                85                  90                  95

Ala Arg Asn Gly Tyr Tyr Val Gly Tyr Tyr Ala Leu Asp Tyr Trp Gly
            100                 105                 110

Gln Gly Thr Ser Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205
```

```
Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly
    450
```

<210> 59
<211> 450
<212> PRT
<213> artificial

<220>
<223> VH-CH-104E10

<400> 59

```
Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Thr Phe Thr Asp Tyr
            20                  25                  30

Ser Met His Trp Val Lys Gln Ala Pro Gly Lys Gly Leu Lys Trp Met
        35                  40                  45

Gly Trp Ile Asn Thr Glu Thr Gly Glu Pro Thr Tyr Ala Asp Asp Phe
    50                  55                  60

Lys Gly Arg Phe Ala Phe Ser Leu Glu Thr Ser Ala Thr Thr Ala Tyr
65                  70                  75                  80

Leu Gln Ile Asn Asn Phe Lys Asn Glu Asp Thr Ala Thr Tyr Phe Cys
                85                  90                  95

Ala Arg Asn Gly Tyr Tyr Val Gly Tyr Tyr Ala Met Asp Tyr Trp Gly
            100                 105                 110

Gln Gly Thr Ser Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205
```

```
Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly
    450
```

<210> 60
<211> 214
<212> PRT
<213> artificial

<220>
<223> VL-CL-122A2

<400> 60

```
Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Ser Asn Tyr
            20                  25                  30

Leu Asn Trp Tyr Gln Gln Lys Pro Asp Gly Thr Val Lys Leu Leu Ile
        35                  40                  45

Tyr Tyr Thr Ser Arg Leu His Ser Gly Val Pro Ser Arg Phe Ser Gly
    50                  55                  60

Ser Gly Ser Gly Thr Asp Tyr Ser Leu Thr Ile Ser Asn Leu Asp Gln
65                  70                  75                  80

Glu Asp Ile Ala Thr Tyr Phe Cys Gln Gln Gly Asn Thr Leu Pro Trp
                85                  90                  95

Thr Phe Gly Gly Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205
```

Phe Asn Arg Gly Glu Cys
210

<210> 61
<211> 213
<212> PRT
<213> artificial

<220>
<223> VL-CL-102E9

<400> 61

```
Gln Ile Val Leu Thr Gln Ser Pro Ala Ile Met Ser Ala Ser Pro Gly
1               5                   10                  15

Glu Lys Val Thr Ile Thr Cys Ser Ala Ser Ser Ser Val Ile Tyr Ile
            20                  25                  30

His Trp Phe Gln Gln Lys Pro Gly Thr Ser Pro Lys Leu Trp Ile Tyr
        35                  40                  45

Ser Thr Ser Tyr Leu Ala Ser Gly Val Pro Ala Arg Phe Ser Gly Ser
    50                  55                  60

Gly Ser Gly Thr Ser Tyr Ser Leu Thr Ile Ser Arg Met Glu Ala Glu
65                  70                  75                  80

Asp Ala Ala Thr Tyr Tyr Cys Gln Gln Arg Arg Ser Tyr Pro Phe Thr
                85                  90                  95

Phe Gly Gly Gly Thr Lys Leu Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe

              195                 200                 205

      Asn Arg Gly Glu Cys
          210
```

<210> 62
<211> 214
<212> PRT
<213> artificial

<220>
<223> VL-CL-104C12

<400> 62

```
Asp Leu Gln Met Thr Gln Thr Pro Ser Ser Leu Ser Ala Ser Leu Gly
1               5                   10                  15

Asp Arg Val Thr Ile Ser Cys Arg Ala Ser Gln Asp Ile Asn Asn Tyr
            20                  25                  30

Leu Ser Trp Tyr Gln Glu Lys Pro Asp Gly Thr Phe Lys Leu Leu Ile
        35                  40                  45

Tyr Tyr Thr Ser Arg Leu His Ser Gly Val Pro Ser Arg Phe Ser Gly
    50                  55                  60

Ser Gly Ser Gly Thr Asp Tyr Ser Leu Thr Val Arg Asn Leu Glu Gln
65                  70                  75                  80

Glu Asp Ile Gly Thr Tyr Phe Cys Gln Gln Gly Lys Thr Leu Pro Trp
                85                  90                  95

Thr Phe Gly Gly Gly Thr Lys Leu Glu Ile Arg Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210
```

<210> 63
<211> 213

<212> PRT
<213> artificial

<220>
<223> VL-CL-114D11

<400> 63

```
Gln Ile Val Leu Thr Gln Ser Pro Ala Ile Met Ser Ala Ser Pro Gly
1               5                   10                  15

Glu Lys Val Thr Ile Thr Cys Ser Ala Ser Ser Ser Val Phe Tyr Met
            20                  25                  30

His Trp Phe Gln Gln Lys Pro Gly Thr Ser Pro Lys Leu Trp Ile Tyr
        35                  40                  45

Ser Thr Ser Asn Leu Ala Ser Gly Val Pro Ala Arg Phe Ser Gly Ser
    50                  55                  60

Gly Ser Gly Thr Ser Tyr Ser Leu Thr Ile Ser Arg Met Glu Ala Glu
65                  70                  75                  80

Asp Ala Ala Thr Tyr Tyr Cys Gln Gln Arg Arg Ser Tyr Pro Tyr Thr
                85                  90                  95

Phe Gly Gly Gly Thr Lys Leu Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175
```

Thr Leu Thr Leu Ser Lys Ala Asp Tyr Glu Lys His Lys Val Tyr Ala
180 185 190

Cys Glu Val Thr His Gln Gly Leu Ser Ser Pro Val Thr Lys Ser Phe
195 200 205

Asn Arg Gly Glu Cys
210

<210> 64
<211> 213
<212> PRT
<213> artificial

<220>
<223> VL-CL-104E10

<400> 64

Gln Ile Val Leu Thr Gln Ser Pro Ala Ile Met Ser Ala Ser Pro Gly
1 5 10 15

Glu Lys Val Thr Met Thr Cys Ser Ala Ser Ser Ser Val Ile Tyr Met
20 25 30

His Trp Phe Gln Gln Lys Pro Gly Thr Ser Pro Lys Leu Trp Ile Tyr
35 40 45

Ser Thr Ser Asn Leu Ala Ser Gly Val Pro Ala Arg Phe Ser Gly Ser
50 55 60

Gly Ser Gly Thr Ser Tyr Ser Leu Thr Ile Ser Arg Met Glu Ala Glu
65 70 75 80

Asp Ala Ala Thr Tyr Tyr Cys Gln Gln Arg Arg Ser Tyr Pro Tyr Thr
85 90 95

Phe Gly Gly Gly Thr Lys Leu Glu Ile Lys Arg Thr Val Ala Ala Pro
100 105 110

Ser Val Phe Ile Phe Pro Pro Ser Asp Glu Gln Leu Lys Ser Gly Thr
115 120 125

Ala Ser Val Val Cys Leu Leu Asn Asn Phe Tyr Pro Arg Glu Ala Lys
130 135 140

Val Gln Trp Lys Val Asp Asn Ala Leu Gln Ser Gly Asn Ser Gln Glu
145 150 155 160

Ser Val Thr Glu Gln Asp Ser Lys Asp Ser Thr Tyr Ser Leu Ser Ser
165 170 175

Thr Leu Thr Leu Ser Lys Ala Asp Tyr Glu Lys His Lys Val Tyr Ala
180 185 190

Cys Glu Val Thr His Gln Gly Leu Ser Ser Pro Val Thr Lys Ser Phe
195 200 205

Asn Arg Gly Glu Cys
210

<210> 65
<211> 18
<212> PRT
<213> artificial

<220>
<223> MB7 signal peptide

<400> 65

Met Arg Trp Ser Trp Ile Phe Leu Leu Leu Leu Ser Ile Thr Ser Ala
1 5 10 15

Asn Ala

<210> 66
<211> 136
<212> PRT
<213> artificial

<220>
<223> MB7-VH-122A2

<400> 66

Met Arg Trp Ser Trp Ile Phe Leu Leu Leu Leu Ser Ile Thr Ser Ala
1 5 10 15

Asn Ala Gln Val Gln Leu Gln Gln Ser Gly Ala Glu Leu Val Arg Pro
20 25 30

Gly Val Ser Val Lys Ile Ser Cys Lys Gly Ser Gly Tyr Thr Phe Thr
35 40 45

Asp Tyr Ser Met His Trp Val Lys Gln Ser His Ala Lys Ser Leu Glu
50 55 60

Trp Ile Gly Val Ile Ser Thr Tyr Tyr Gly Asp Ser Asn Tyr Asn Gln
65 70 75 80

Lys Phe Lys Gly Lys Ala Thr Met Thr Val Asp Lys Ser Ser Thr Thr
85 90 95

Ala Tyr Met Glu Leu Ala Arg Leu Thr Ser Glu Asp Ser Ala Ile Tyr
100 105 110

Tyr Cys Ala Arg Asn Gly Asn Phe Tyr Val Met Asp Tyr Trp Gly Gln
115 120 125

Gly Thr Ser Val Thr Val Ser Ser
130 135

<210> 67
<211> 139
<212> PRT
<213> artificial

<220>
<223> MB7-VH-102E9

<400> 67

Met Arg Trp Ser Trp Ile Phe Leu Leu Leu Leu Ser Ile Thr Ser Ala
1 5 10 15

Asn Ala Gln Ile His Leu Val Gln Ser Gly Pro Asp Leu Lys Lys Pro
20 25 30

Gly Glu Thr Val Lys Ile Ser Cys Lys Ala Ser Gly Tyr Thr Phe Thr
35 40 45

Asp Tyr Ser Met His Trp Val Lys Gln Ala Pro Gly Lys Gly Leu Lys
50 55 60

Trp Met Gly Trp Ile Asn Thr Glu Thr Gly Glu Pro Thr Tyr Ala Asp
65 70 75 80

Asp Phe Lys Gly Arg Phe Ala Phe Ser Leu Glu Ser Ser Ala Ser Thr
85 90 95

Ala Phe Leu Gln Ile Asn Asn Leu Lys Asn Glu Asp Thr Ser Thr Tyr
100 105 110

Phe Cys Thr Arg Asn Gly Tyr Tyr Val Gly Tyr Tyr Ala Met Asp Tyr
115 120 125

Trp Gly Gln Gly Thr Ser Val Thr Val Ser Ser
130 135

<210> 68
<211> 136
<212> PRT
<213> artificial

<220>
<223> MB7-VH-104C12

<400> 68

```
Met Arg Trp Ser Trp Ile Phe Leu Leu Leu Leu Ser Ile Thr Ser Ala
1               5                   10                  15

Asn Ala Gln Val Gln Leu Gln Gln Ser Gly Ala Glu Leu Val Gly Pro
            20                  25                  30

Gly Val Ser Val Lys Ile Ser Cys Lys Gly Ser Gly Tyr Thr Phe Thr
        35                  40                  45

Asp Tyr Ser Met His Trp Val Lys Gln Ser His Ala Lys Ser Leu Glu
    50                  55                  60

Trp Ile Gly Val Ile Ser Pro Tyr Tyr Gly Asp Thr Asn Tyr Asn Gln
65                  70                  75                  80

Lys Phe Lys Gly Lys Ala Thr Met Thr Val Asp Lys Ser Ser Ser Thr
                85                  90                  95

Ala Tyr Met Glu Leu Ala Ser Leu Thr Ser Glu Asp Ser Ala Ile Tyr
            100                 105                 110

Phe Cys Ala Arg Asn Asp Asp Tyr Tyr Arg Phe Ala Tyr Trp Gly Gln
        115                 120                 125

Gly Thr Leu Val Thr Val Ser Ala
    130                 135
```

<210> 69
<211> 139
<212> PRT
<213> artificial

<220>
<223> MB7-VH-114D11

<400> 69

Met Arg Trp Ser Trp Ile Phe Leu Leu Leu Leu Ser Ile Thr Ser Ala
1 5 10 15

Asn Ala Gln Ile Gln Leu Val Gln Ser Gly Pro Glu Leu Lys Lys Pro
20 25 30

Gly Glu Thr Val Lys Ile Ser Cys Lys Ala Ser Gly Tyr Thr Phe Thr
35 40 45

Asp Ser Ser Met His Trp Val Gln Gln Ala Pro Asn Lys Gly Leu Lys
50 55 60

Trp Met Gly Trp Ile Asn Thr Glu Thr Gly Gly Pro Thr Tyr Ala Asp
65 70 75 80

Asp Phe Lys Gly Arg Phe Ala Phe Ser Leu Glu Thr Ser Ala Arg Thr
85 90 95

Ala Tyr Leu Gln Ile Asn Asn Leu Lys Asn Glu Asp Thr Ala Thr Tyr
100 105 110

Phe Cys Ala Arg Asn Gly Tyr Tyr Val Gly Tyr Tyr Ala Leu Asp Tyr
115 120 125

Trp Gly Gln Gly Thr Ser Val Thr Val Ser Ser
130 135

<210> 70
<211> 139
<212> PRT
<213> artificial

<220>
<223> MB7-VH-104E10

<400> 70

```
Met Arg Trp Ser Trp Ile Phe Leu Leu Leu Leu Ser Ile Thr Ser Ala
1               5                   10                  15

Asn Ala Gln Ile Gln Leu Val Gln Ser Gly Pro Glu Leu Lys Lys Pro
            20                  25                  30

Gly Glu Thr Val Lys Ile Ser Cys Lys Ala Ser Gly Tyr Thr Phe Thr
        35                  40                  45

Asp Tyr Ser Met His Trp Val Lys Gln Ala Pro Gly Lys Gly Leu Lys
    50                  55                  60

Trp Met Gly Trp Ile Asn Thr Glu Thr Gly Glu Pro Thr Tyr Ala Asp
65                  70                  75                  80

Asp Phe Lys Gly Arg Phe Ala Phe Ser Leu Glu Thr Ser Ala Thr Thr
                85                  90                  95

Ala Tyr Leu Gln Ile Asn Asn Phe Lys Asn Glu Asp Thr Ala Thr Tyr
            100                 105                 110

Phe Cys Ala Arg Asn Gly Tyr Tyr Val Gly Tyr Tyr Ala Met Asp Tyr
        115                 120                 125

Trp Gly Gln Gly Thr Ser Val Thr Val Ser Ser
    130                 135
```

<210> 71
<211> 125
<212> PRT
<213> artificial

<220>
<223> MB7-VL-122A2

<400> 71

```
Met Arg Trp Ser Trp Ile Phe Leu Leu Leu Leu Ser Ile Thr Ser Ala
1               5                   10                  15

Asn Ala Asp Ile Gln Met Thr Gln Thr Thr Ser Ser Leu Ser Ala Ser
            20                  25                  30

Leu Gly Asp Arg Val Thr Ile Ser Cys Arg Ala Ser Gln Asp Ile Ser
        35                  40                  45

Asn Tyr Leu Asn Trp Tyr Gln Gln Lys Pro Asp Gly Thr Val Lys Leu
    50                  55                  60

Leu Ile Tyr Tyr Thr Ser Arg Leu His Ser Gly Val Pro Ser Arg Phe
65                  70                  75                  80

Ser Gly Ser Gly Ser Gly Thr Asp Tyr Ser Leu Thr Ile Ser Asn Leu
                85                  90                  95

Asp Gln Glu Asp Ile Ala Thr Tyr Phe Cys Gln Gln Gly Asn Thr Leu
            100                 105                 110

Pro Trp Thr Phe Gly Gly Gly Thr Lys Leu Glu Ile Lys
        115                 120                 125
```

<210> 72
<211> 124
<212> PRT
<213> artificial

<220>
<223> MB7-VL-102E9

<400> 72

```
Met Arg Trp Ser Trp Ile Phe Leu Leu Leu Leu Ser Ile Thr Ser Ala
1               5                   10                  15

Asn Ala Gln Ile Val Leu Thr Gln Ser Pro Ala Ile Met Ser Ala Ser
            20                  25                  30

Pro Gly Glu Lys Val Thr Ile Thr Cys Ser Ala Ser Ser Ser Val Ile
        35                  40                  45

Tyr Ile His Trp Phe Gln Gln Lys Pro Gly Thr Ser Pro Lys Leu Trp
    50                  55                  60

Ile Tyr Ser Thr Ser Tyr Leu Ala Ser Gly Val Pro Ala Arg Phe Ser
65                  70                  75                  80

Gly Ser Gly Ser Gly Thr Ser Tyr Ser Leu Thr Ile Ser Arg Met Glu
                85                  90                  95

Ala Glu Asp Ala Ala Thr Tyr Tyr Cys Gln Gln Arg Arg Ser Tyr Pro
            100                 105                 110

Phe Thr Phe Gly Gly Gly Thr Lys Leu Glu Ile Lys
        115                 120
```

<210> 73
<211> 125
<212> PRT
<213> artificial

<220>
<223> MB7-VL-104C12

<400> 73

```
Met Arg Trp Ser Trp Ile Phe Leu Leu Leu Leu Ser Ile Thr Ser Ala
1               5                   10                  15

Asn Ala Asp Leu Gln Met Thr Gln Thr Pro Ser Ser Leu Ser Ala Ser
            20                  25                  30

Leu Gly Asp Arg Val Thr Ile Ser Cys Arg Ala Ser Gln Asp Ile Asn
        35                  40                  45

Asn Tyr Leu Ser Trp Tyr Gln Glu Lys Pro Asp Gly Thr Phe Lys Leu
    50                  55                  60

Leu Ile Tyr Tyr Thr Ser Arg Leu His Ser Gly Val Pro Ser Arg Phe
65                  70                  75                  80
```

Ser Gly Ser Gly Ser Gly Thr Asp Tyr Ser Leu Thr Val Arg Asn Leu
85 90 95

Glu Gln Glu Asp Ile Gly Thr Tyr Phe Cys Gln Gln Gly Lys Thr Leu
100 105 110

Pro Trp Thr Phe Gly Gly Gly Thr Lys Leu Glu Ile Arg
115 120 125

<210> 74
<211> 124
<212> PRT
<213> artificial

<220>
<223> MB7-VL-114D11

<400> 74

Met Arg Trp Ser Trp Ile Phe Leu Leu Leu Leu Ser Ile Thr Ser Ala
1 5 10 15

Asn Ala Gln Ile Val Leu Thr Gln Ser Pro Ala Ile Met Ser Ala Ser
20 25 30

Pro Gly Glu Lys Val Thr Ile Thr Cys Ser Ala Ser Ser Ser Val Phe
35 40 45

Tyr Met His Trp Phe Gln Gln Lys Pro Gly Thr Ser Pro Lys Leu Trp
50 55 60

Ile Tyr Ser Thr Ser Asn Leu Ala Ser Gly Val Pro Ala Arg Phe Ser
65 70 75 80

Gly Ser Gly Ser Gly Thr Ser Tyr Ser Leu Thr Ile Ser Arg Met Glu
85 90 95

Ala Glu Asp Ala Ala Thr Tyr Tyr Cys Gln Gln Arg Arg Ser Tyr Pro
100 105 110

Tyr Thr Phe Gly Gly Gly Thr Lys Leu Glu Ile Lys
115 120

<210> 75
<211> 124
<212> PRT
<213> artificial

<220>
<223> MB7-VL-104E10

<400> 75

```
Met Arg Trp Ser Trp Ile Phe Leu Leu Leu Leu Ser Ile Thr Ser Ala
1               5                   10                  15

Asn Ala Gln Ile Val Leu Thr Gln Ser Pro Ala Ile Met Ser Ala Ser
            20                  25                  30

Pro Gly Glu Lys Val Thr Met Thr Cys Ser Ala Ser Ser Ser Val Ile
        35                  40                  45

Tyr Met His Trp Phe Gln Gln Lys Pro Gly Thr Ser Pro Lys Leu Trp
    50                  55                  60

Ile Tyr Ser Thr Ser Asn Leu Ala Ser Gly Val Pro Ala Arg Phe Ser
65                  70                  75                  80

Gly Ser Gly Ser Gly Thr Ser Tyr Ser Leu Thr Ile Ser Arg Met Glu
                85                  90                  95

Ala Glu Asp Ala Ala Thr Tyr Tyr Cys Gln Gln Arg Arg Ser Tyr Pro
            100                 105                 110

Tyr Thr Phe Gly Gly Gly Thr Lys Leu Glu Ile Lys
        115                 120
```

<210> 76
<211> 465
<212> PRT
<213> artificial

<220>
<223> MB7-VH-CH-122A2

<400> 76

```
Met Arg Trp Ser Trp Ile Phe Leu Leu Leu Leu Ser Ile Thr Ser Ala
1               5                   10                  15

Asn Ala Gln Val Gln Leu Gln Gln Ser Gly Ala Glu Leu Val Arg Pro
            20                  25                  30

Gly Val Ser Val Lys Ile Ser Cys Lys Gly Ser Gly Tyr Thr Phe Thr
        35                  40                  45

Asp Tyr Ser Met His Trp Val Lys Gln Ser His Ala Lys Ser Leu Glu
    50                  55                  60

Trp Ile Gly Val Ile Ser Thr Tyr Tyr Gly Asp Ser Asn Tyr Asn Gln
65                  70                  75                  80
```

Lys Phe Lys Gly Lys Ala Thr Met Thr Val Asp Lys Ser Ser Thr Thr
85 90 95

Ala Tyr Met Glu Leu Ala Arg Leu Thr Ser Glu Asp Ser Ala Ile Tyr
100 105 110

Tyr Cys Ala Arg Asn Gly Asn Phe Tyr Val Met Asp Tyr Trp Gly Gln
115 120 125

Gly Thr Ser Val Thr Val Ser Ser Ala Ser Thr Lys Gly Pro Ser Val
130 135 140

Phe Pro Leu Ala Pro Ser Ser Lys Ser Thr Ser Gly Gly Thr Ala Ala
145 150 155 160

Leu Gly Cys Leu Val Lys Asp Tyr Phe Pro Glu Pro Val Thr Val Ser
165 170 175

Trp Asn Ser Gly Ala Leu Thr Ser Gly Val His Thr Phe Pro Ala Val
180 185 190

Leu Gln Ser Ser Gly Leu Tyr Ser Leu Ser Ser Val Val Thr Val Pro
195 200 205

Ser Ser Ser Leu Gly Thr Gln Thr Tyr Ile Cys Asn Val Asn His Lys
210 215 220

Pro Ser Asn Thr Lys Val Asp Lys Lys Val Glu Pro Lys Ser Cys Asp
225 230 235 240

Lys Thr His Thr Cys Pro Pro Cys Pro Ala Pro Glu Leu Leu Gly Gly
245 250 255

Pro Ser Val Phe Leu Phe Pro Pro Lys Pro Lys Asp Thr Leu Met Ile
260 265 270

Ser Arg Thr Pro Glu Val Thr Cys Val Val Val Asp Val Ser His Glu
275 280 285

Asp Pro Glu Val Lys Phe Asn Trp Tyr Val Asp Gly Val Glu Val His
290 295 300

Asn Ala Lys Thr Lys Pro Arg Glu Glu Gln Tyr Asn Ser Thr Tyr Arg
305 310 315 320

Val Val Ser Val Leu Thr Val Leu His Gln Asp Trp Leu Asn Gly Lys
325 330 335

Glu Tyr Lys Cys Lys Val Ser Asn Lys Ala Leu Pro Ala Pro Ile Glu
340 345 350

Lys Thr Ile Ser Lys Ala Lys Gly Gln Pro Arg Glu Pro Gln Val Tyr
355 360 365

Thr Leu Pro Pro Ser Arg Asp Glu Leu Thr Lys Asn Gln Val Ser Leu
370 375 380

Thr Cys Leu Val Lys Gly Phe Tyr Pro Ser Asp Ile Ala Val Glu Trp
385 390 395 400

Glu Ser Asn Gly Gln Pro Glu Asn Asn Tyr Lys Thr Thr Pro Pro Val
405 410 415

Leu Asp Ser Asp Gly Ser Phe Phe Leu Tyr Ser Lys Leu Thr Val Asp
420 425 430

Lys Ser Arg Trp Gln Gln Gly Asn Val Phe Ser Cys Ser Val Met His
435 440 445

Glu Ala Leu His Asn His Tyr Thr Gln Lys Ser Leu Ser Leu Ser Pro
450 455 460

Gly
465

<210> 77
<211> 468
<212> PRT
<213> artificial

<220>
<223> MB7-VH-CH-102E9

<400> 77

Met Arg Trp Ser Trp Ile Phe Leu Leu Leu Leu Ser Ile Thr Ser Ala
1 5 10 15

Asn Ala Gln Ile His Leu Val Gln Ser Gly Pro Asp Leu Lys Lys Pro
20 25 30

Gly Glu Thr Val Lys Ile Ser Cys Lys Ala Ser Gly Tyr Thr Phe Thr
35 40 45

Asp Tyr Ser Met His Trp Val Lys Gln Ala Pro Gly Lys Gly Leu Lys
50 55 60

```
Trp Met Gly Trp Ile Asn Thr Glu Thr Gly Glu Pro Thr Tyr Ala Asp
65                  70                  75                  80

Asp Phe Lys Gly Arg Phe Ala Phe Ser Leu Glu Ser Ser Ala Ser Thr
                85                  90                  95

Ala Phe Leu Gln Ile Asn Asn Leu Lys Asn Glu Asp Thr Ser Thr Tyr
            100                 105                 110

Phe Cys Thr Arg Asn Gly Tyr Tyr Val Gly Tyr Tyr Ala Met Asp Tyr
        115                 120                 125

Trp Gly Gln Gly Thr Ser Val Thr Val Ser Ser Ala Ser Thr Lys Gly
    130                 135                 140

Pro Ser Val Phe Pro Leu Ala Pro Ser Ser Lys Ser Thr Ser Gly Gly
145                 150                 155                 160

Thr Ala Ala Leu Gly Cys Leu Val Lys Asp Tyr Phe Pro Glu Pro Val
                165                 170                 175

Thr Val Ser Trp Asn Ser Gly Ala Leu Thr Ser Gly Val His Thr Phe
            180                 185                 190

Pro Ala Val Leu Gln Ser Ser Gly Leu Tyr Ser Leu Ser Ser Val Val
        195                 200                 205

Thr Val Pro Ser Ser Ser Leu Gly Thr Gln Thr Tyr Ile Cys Asn Val
    210                 215                 220

Asn His Lys Pro Ser Asn Thr Lys Val Asp Lys Lys Val Glu Pro Lys
225                 230                 235                 240

Ser Cys Asp Lys Thr His Thr Cys Pro Pro Cys Pro Ala Pro Glu Leu
                245                 250                 255

Leu Gly Gly Pro Ser Val Phe Leu Phe Pro Pro Lys Pro Lys Asp Thr
            260                 265                 270

Leu Met Ile Ser Arg Thr Pro Glu Val Thr Cys Val Val Val Asp Val
        275                 280                 285

Ser His Glu Asp Pro Glu Val Lys Phe Asn Trp Tyr Val Asp Gly Val
    290                 295                 300

Glu Val His Asn Ala Lys Thr Lys Pro Arg Glu Glu Gln Tyr Asn Ser
305                 310                 315                 320
```

Thr Tyr Arg Val Val Ser Val Leu Thr Val Leu His Gln Asp Trp Leu
325 330 335

Asn Gly Lys Glu Tyr Lys Cys Lys Val Ser Asn Lys Ala Leu Pro Ala
340 345 350

Pro Ile Glu Lys Thr Ile Ser Lys Ala Lys Gly Gln Pro Arg Glu Pro
355 360 365

Gln Val Tyr Thr Leu Pro Pro Ser Arg Asp Glu Leu Thr Lys Asn Gln
370 375 380

Val Ser Leu Thr Cys Leu Val Lys Gly Phe Tyr Pro Ser Asp Ile Ala
385 390 395 400

Val Glu Trp Glu Ser Asn Gly Gln Pro Glu Asn Asn Tyr Lys Thr Thr
405 410 415

Pro Pro Val Leu Asp Ser Asp Gly Ser Phe Phe Leu Tyr Ser Lys Leu
420 425 430

Thr Val Asp Lys Ser Arg Trp Gln Gln Gly Asn Val Phe Ser Cys Ser
435 440 445

Val Met His Glu Ala Leu His Asn His Tyr Thr Gln Lys Ser Leu Ser
450 455 460

Leu Ser Pro Gly
465

<210> 78
<211> 465
<212> PRT
<213> artificial

<220>
<223> MB7-VH-CH-104C12

<400> 78

Met Arg Trp Ser Trp Ile Phe Leu Leu Leu Leu Ser Ile Thr Ser Ala
1 5 10 15

Asn Ala Gln Val Gln Leu Gln Gln Ser Gly Ala Glu Leu Val Gly Pro
20 25 30

Gly Val Ser Val Lys Ile Ser Cys Lys Gly Ser Gly Tyr Thr Phe Thr
35 40 45

```
Asp Tyr Ser Met His Trp Val Lys Gln Ser His Ala Lys Ser Leu Glu
    50                  55                  60

Trp Ile Gly Val Ile Ser Pro Tyr Tyr Gly Asp Thr Asn Tyr Asn Gln
65                  70                  75                  80

Lys Phe Lys Gly Lys Ala Thr Met Thr Val Asp Lys Ser Ser Ser Thr
                85                  90                  95

Ala Tyr Met Glu Leu Ala Ser Leu Thr Ser Glu Asp Ser Ala Ile Tyr
            100                 105                 110

Phe Cys Ala Arg Asn Asp Asp Tyr Tyr Arg Phe Ala Tyr Trp Gly Gln
        115                 120                 125

Gly Thr Leu Val Thr Val Ser Ala Ala Ser Thr Lys Gly Pro Ser Val
    130                 135                 140

Phe Pro Leu Ala Pro Ser Ser Lys Ser Thr Ser Gly Gly Thr Ala Ala
145                 150                 155                 160

Leu Gly Cys Leu Val Lys Asp Tyr Phe Pro Glu Pro Val Thr Val Ser
                165                 170                 175

Trp Asn Ser Gly Ala Leu Thr Ser Gly Val His Thr Phe Pro Ala Val
            180                 185                 190

Leu Gln Ser Ser Gly Leu Tyr Ser Leu Ser Ser Val Val Thr Val Pro
        195                 200                 205

Ser Ser Ser Leu Gly Thr Gln Thr Tyr Ile Cys Asn Val Asn His Lys
    210                 215                 220

Pro Ser Asn Thr Lys Val Asp Lys Lys Val Glu Pro Lys Ser Cys Asp
225                 230                 235                 240

Lys Thr His Thr Cys Pro Pro Cys Pro Ala Pro Glu Leu Leu Gly Gly
                245                 250                 255

Pro Ser Val Phe Leu Phe Pro Pro Lys Pro Lys Asp Thr Leu Met Ile
            260                 265                 270

Ser Arg Thr Pro Glu Val Thr Cys Val Val Val Asp Val Ser His Glu
        275                 280                 285

Asp Pro Glu Val Lys Phe Asn Trp Tyr Val Asp Gly Val Glu Val His
    290                 295                 300
```

Asn Ala Lys Thr Lys Pro Arg Glu Glu Gln Tyr Asn Ser Thr Tyr Arg
305 310 315 320

Val Val Ser Val Leu Thr Val Leu His Gln Asp Trp Leu Asn Gly Lys
325 330 335

Glu Tyr Lys Cys Lys Val Ser Asn Lys Ala Leu Pro Ala Pro Ile Glu
340 345 350

Lys Thr Ile Ser Lys Ala Lys Gly Gln Pro Arg Glu Pro Gln Val Tyr
355 360 365

Thr Leu Pro Pro Ser Arg Asp Glu Leu Thr Lys Asn Gln Val Ser Leu
370 375 380

Thr Cys Leu Val Lys Gly Phe Tyr Pro Ser Asp Ile Ala Val Glu Trp
385 390 395 400

Glu Ser Asn Gly Gln Pro Glu Asn Asn Tyr Lys Thr Thr Pro Pro Val
405 410 415

Leu Asp Ser Asp Gly Ser Phe Phe Leu Tyr Ser Lys Leu Thr Val Asp
420 425 430

Lys Ser Arg Trp Gln Gln Gly Asn Val Phe Ser Cys Ser Val Met His
435 440 445

Glu Ala Leu His Asn His Tyr Thr Gln Lys Ser Leu Ser Leu Ser Pro
450 455 460

Gly
465

<210> 79
<211> 468
<212> PRT
<213> artificial

<220>
<223> MB7-VH-CH-114D11

<400> 79

Met Arg Trp Ser Trp Ile Phe Leu Leu Leu Leu Ser Ile Thr Ser Ala
1 5 10 15

Asn Ala Gln Ile Gln Leu Val Gln Ser Gly Pro Glu Leu Lys Lys Pro
20 25 30

```
Gly Glu Thr Val Lys Ile Ser Cys Lys Ala Ser Gly Tyr Thr Phe Thr
        35                  40                  45

Asp Ser Ser Met His Trp Val Gln Gln Ala Pro Asn Lys Gly Leu Lys
    50                  55                  60

Trp Met Gly Trp Ile Asn Thr Glu Thr Gly Gly Pro Thr Tyr Ala Asp
65                  70                  75                  80

Asp Phe Lys Gly Arg Phe Ala Phe Ser Leu Glu Thr Ser Ala Arg Thr
                85                  90                  95

Ala Tyr Leu Gln Ile Asn Asn Leu Lys Asn Glu Asp Thr Ala Thr Tyr
            100                 105                 110

Phe Cys Ala Arg Asn Gly Tyr Tyr Val Gly Tyr Tyr Ala Leu Asp Tyr
        115                 120                 125

Trp Gly Gln Gly Thr Ser Val Thr Val Ser Ser Ala Ser Thr Lys Gly
    130                 135                 140

Pro Ser Val Phe Pro Leu Ala Pro Ser Ser Lys Ser Thr Ser Gly Gly
145                 150                 155                 160

Thr Ala Ala Leu Gly Cys Leu Val Lys Asp Tyr Phe Pro Glu Pro Val
                165                 170                 175

Thr Val Ser Trp Asn Ser Gly Ala Leu Thr Ser Gly Val His Thr Phe
            180                 185                 190

Pro Ala Val Leu Gln Ser Ser Gly Leu Tyr Ser Leu Ser Ser Val Val
        195                 200                 205

Thr Val Pro Ser Ser Ser Leu Gly Thr Gln Thr Tyr Ile Cys Asn Val
    210                 215                 220

Asn His Lys Pro Ser Asn Thr Lys Val Asp Lys Lys Val Glu Pro Lys
225                 230                 235                 240

Ser Cys Asp Lys Thr His Thr Cys Pro Pro Cys Pro Ala Pro Glu Leu
                245                 250                 255

Leu Gly Gly Pro Ser Val Phe Leu Phe Pro Pro Lys Pro Lys Asp Thr
            260                 265                 270

Leu Met Ile Ser Arg Thr Pro Glu Val Thr Cys Val Val Val Asp Val
        275                 280                 285
```

```
Ser His Glu Asp Pro Glu Val Lys Phe Asn Trp Tyr Val Asp Gly Val
    290                 295                 300

Glu Val His Asn Ala Lys Thr Lys Pro Arg Glu Glu Gln Tyr Asn Ser
305                 310                 315                 320

Thr Tyr Arg Val Val Ser Val Leu Thr Val Leu His Gln Asp Trp Leu
                325                 330                 335

Asn Gly Lys Glu Tyr Lys Cys Lys Val Ser Asn Lys Ala Leu Pro Ala
            340                 345                 350

Pro Ile Glu Lys Thr Ile Ser Lys Ala Lys Gly Gln Pro Arg Glu Pro
        355                 360                 365

Gln Val Tyr Thr Leu Pro Pro Ser Arg Asp Glu Leu Thr Lys Asn Gln
    370                 375                 380

Val Ser Leu Thr Cys Leu Val Lys Gly Phe Tyr Pro Ser Asp Ile Ala
385                 390                 395                 400

Val Glu Trp Glu Ser Asn Gly Gln Pro Glu Asn Asn Tyr Lys Thr Thr
                405                 410                 415

Pro Pro Val Leu Asp Ser Asp Gly Ser Phe Phe Leu Tyr Ser Lys Leu
            420                 425                 430

Thr Val Asp Lys Ser Arg Trp Gln Gln Gly Asn Val Phe Ser Cys Ser
        435                 440                 445

Val Met His Glu Ala Leu His Asn His Tyr Thr Gln Lys Ser Leu Ser
    450                 455                 460

Leu Ser Pro Gly
465
```

<210> 80
<211> 468
<212> PRT
<213> artificial

<220>
<223> MB7-VH-CH-104E10

<400> 80

```
Met Arg Trp Ser Trp Ile Phe Leu Leu Leu Leu Ser Ile Thr Ser Ala
1               5                   10                  15
```

Asn Ala Gln Ile Gln Leu Val Gln Ser Gly Pro Glu Leu Lys Lys Pro
20 25 30

Gly Glu Thr Val Lys Ile Ser Cys Lys Ala Ser Gly Tyr Thr Phe Thr
35 40 45

Asp Tyr Ser Met His Trp Val Lys Gln Ala Pro Gly Lys Gly Leu Lys
50 55 60

Trp Met Gly Trp Ile Asn Thr Glu Thr Gly Glu Pro Thr Tyr Ala Asp
65 70 75 80

Asp Phe Lys Gly Arg Phe Ala Phe Ser Leu Glu Thr Ser Ala Thr Thr
85 90 95

Ala Tyr Leu Gln Ile Asn Asn Phe Lys Asn Glu Asp Thr Ala Thr Tyr
100 105 110

Phe Cys Ala Arg Asn Gly Tyr Tyr Val Gly Tyr Tyr Ala Met Asp Tyr
115 120 125

Trp Gly Gln Gly Thr Ser Val Thr Val Ser Ser Ala Ser Thr Lys Gly
130 135 140

Pro Ser Val Phe Pro Leu Ala Pro Ser Ser Lys Ser Thr Ser Gly Gly
145 150 155 160

Thr Ala Ala Leu Gly Cys Leu Val Lys Asp Tyr Phe Pro Glu Pro Val
165 170 175

Thr Val Ser Trp Asn Ser Gly Ala Leu Thr Ser Gly Val His Thr Phe
180 185 190

Pro Ala Val Leu Gln Ser Ser Gly Leu Tyr Ser Leu Ser Ser Val Val
195 200 205

Thr Val Pro Ser Ser Ser Leu Gly Thr Gln Thr Tyr Ile Cys Asn Val
210 215 220

Asn His Lys Pro Ser Asn Thr Lys Val Asp Lys Lys Val Glu Pro Lys
225 230 235 240

Ser Cys Asp Lys Thr His Thr Cys Pro Pro Cys Pro Ala Pro Glu Leu
245 250 255

Leu Gly Gly Pro Ser Val Phe Leu Phe Pro Pro Lys Pro Lys Asp Thr
260 265 270

Leu Met Ile Ser Arg Thr Pro Glu Val Thr Cys Val Val Val Asp Val
275 280 285

Ser His Glu Asp Pro Glu Val Lys Phe Asn Trp Tyr Val Asp Gly Val
290 295 300

Glu Val His Asn Ala Lys Thr Lys Pro Arg Glu Glu Gln Tyr Asn Ser
305 310 315 320

Thr Tyr Arg Val Val Ser Val Leu Thr Val Leu His Gln Asp Trp Leu
325 330 335

Asn Gly Lys Glu Tyr Lys Cys Lys Val Ser Asn Lys Ala Leu Pro Ala
340 345 350

Pro Ile Glu Lys Thr Ile Ser Lys Ala Lys Gly Gln Pro Arg Glu Pro
355 360 365

Gln Val Tyr Thr Leu Pro Pro Ser Arg Asp Glu Leu Thr Lys Asn Gln
370 375 380

Val Ser Leu Thr Cys Leu Val Lys Gly Phe Tyr Pro Ser Asp Ile Ala
385 390 395 400

Val Glu Trp Glu Ser Asn Gly Gln Pro Glu Asn Asn Tyr Lys Thr Thr
405 410 415

Pro Pro Val Leu Asp Ser Asp Gly Ser Phe Phe Leu Tyr Ser Lys Leu
420 425 430

Thr Val Asp Lys Ser Arg Trp Gln Gln Gly Asn Val Phe Ser Cys Ser
435 440 445

Val Met His Glu Ala Leu His Asn His Tyr Thr Gln Lys Ser Leu Ser
450 455 460

Leu Ser Pro Gly
465

<210> 81
<211> 232
<212> PRT
<213> artificial

<220>
<223> MB7-VL-CL-122A2

<400> 81

```
Met Arg Trp Ser Trp Ile Phe Leu Leu Leu Leu Ser Ile Thr Ser Ala
1               5                   10                  15

Asn Ala Asp Ile Gln Met Thr Gln Thr Thr Ser Ser Leu Ser Ala Ser
            20                  25                  30

Leu Gly Asp Arg Val Thr Ile Ser Cys Arg Ala Ser Gln Asp Ile Ser
        35                  40                  45

Asn Tyr Leu Asn Trp Tyr Gln Gln Lys Pro Asp Gly Thr Val Lys Leu
    50                  55                  60

Leu Ile Tyr Tyr Thr Ser Arg Leu His Ser Gly Val Pro Ser Arg Phe
65                  70                  75                  80

Ser Gly Ser Gly Ser Gly Thr Asp Tyr Ser Leu Thr Ile Ser Asn Leu
                85                  90                  95

Asp Gln Glu Asp Ile Ala Thr Tyr Phe Cys Gln Gln Gly Asn Thr Leu
            100                 105                 110

Pro Trp Thr Phe Gly Gly Gly Thr Lys Leu Glu Ile Lys Arg Thr Val
        115                 120                 125

Ala Ala Pro Ser Val Phe Ile Phe Pro Pro Ser Asp Glu Gln Leu Lys
    130                 135                 140

Ser Gly Thr Ala Ser Val Val Cys Leu Leu Asn Asn Phe Tyr Pro Arg
145                 150                 155                 160

Glu Ala Lys Val Gln Trp Lys Val Asp Asn Ala Leu Gln Ser Gly Asn
                165                 170                 175

Ser Gln Glu Ser Val Thr Glu Gln Asp Ser Lys Asp Ser Thr Tyr Ser
            180                 185                 190

Leu Ser Ser Thr Leu Thr Leu Ser Lys Ala Asp Tyr Glu Lys His Lys
        195                 200                 205

Val Tyr Ala Cys Glu Val Thr His Gln Gly Leu Ser Ser Pro Val Thr
    210                 215                 220

Lys Ser Phe Asn Arg Gly Glu Cys
225                 230
```

<210> 82
<211> 231

<212> PRT
<213> artificial

<220>
<223> MB7-VL-CL-102E9

<400> 82

```
Met Arg Trp Ser Trp Ile Phe Leu Leu Leu Leu Ser Ile Thr Ser Ala
1               5                   10                  15

Asn Ala Gln Ile Val Leu Thr Gln Ser Pro Ala Ile Met Ser Ala Ser
            20                  25                  30

Pro Gly Glu Lys Val Thr Ile Thr Cys Ser Ala Ser Ser Ser Val Ile
        35                  40                  45

Tyr Ile His Trp Phe Gln Gln Lys Pro Gly Thr Ser Pro Lys Leu Trp
    50                  55                  60

Ile Tyr Ser Thr Ser Tyr Leu Ala Ser Gly Val Pro Ala Arg Phe Ser
65                  70                  75                  80

Gly Ser Gly Ser Gly Thr Ser Tyr Ser Leu Thr Ile Ser Arg Met Glu
                85                  90                  95

Ala Glu Asp Ala Ala Thr Tyr Tyr Cys Gln Gln Arg Arg Ser Tyr Pro
            100                 105                 110

Phe Thr Phe Gly Gly Gly Thr Lys Leu Glu Ile Lys Arg Thr Val Ala
        115                 120                 125

Ala Pro Ser Val Phe Ile Phe Pro Pro Ser Asp Glu Gln Leu Lys Ser
    130                 135                 140

Gly Thr Ala Ser Val Val Cys Leu Leu Asn Asn Phe Tyr Pro Arg Glu
145                 150                 155                 160

Ala Lys Val Gln Trp Lys Val Asp Asn Ala Leu Gln Ser Gly Asn Ser
                165                 170                 175

Gln Glu Ser Val Thr Glu Gln Asp Ser Lys Asp Ser Thr Tyr Ser Leu
            180                 185                 190

Ser Ser Thr Leu Thr Leu Ser Lys Ala Asp Tyr Glu Lys His Lys Val
        195                 200                 205

Tyr Ala Cys Glu Val Thr His Gln Gly Leu Ser Ser Pro Val Thr Lys
    210                 215                 220

                  Ser Phe Asn Arg Gly Glu Cys
                  225                 230
```

<210> 83
<211> 232
<212> PRT

<213> artificial

<220>
<223> MB7-VL-CL-104C12

<400> 83

```
Met Arg Trp Ser Trp Ile Phe Leu Leu Leu Leu Ser Ile Thr Ser Ala
1               5                   10                  15

Asn Ala Asp Leu Gln Met Thr Gln Thr Pro Ser Ser Leu Ser Ala Ser
            20                  25                  30

Leu Gly Asp Arg Val Thr Ile Ser Cys Arg Ala Ser Gln Asp Ile Asn
        35                  40                  45

Asn Tyr Leu Ser Trp Tyr Gln Glu Lys Pro Asp Gly Thr Phe Lys Leu
    50                  55                  60

Leu Ile Tyr Tyr Thr Ser Arg Leu His Ser Gly Val Pro Ser Arg Phe
65                  70                  75                  80

Ser Gly Ser Gly Ser Gly Thr Asp Tyr Ser Leu Thr Val Arg Asn Leu
                85                  90                  95

Glu Gln Glu Asp Ile Gly Thr Tyr Phe Cys Gln Gln Gly Lys Thr Leu
            100                 105                 110

Pro Trp Thr Phe Gly Gly Gly Thr Lys Leu Glu Ile Arg Arg Thr Val
        115                 120                 125

Ala Ala Pro Ser Val Phe Ile Phe Pro Pro Ser Asp Glu Gln Leu Lys
    130                 135                 140

Ser Gly Thr Ala Ser Val Val Cys Leu Leu Asn Asn Phe Tyr Pro Arg
145                 150                 155                 160

Glu Ala Lys Val Gln Trp Lys Val Asp Asn Ala Leu Gln Ser Gly Asn
                165                 170                 175

Ser Gln Glu Ser Val Thr Glu Gln Asp Ser Lys Asp Ser Thr Tyr Ser
            180                 185                 190

Leu Ser Ser Thr Leu Thr Leu Ser Lys Ala Asp Tyr Glu Lys His Lys
```

195 200 205

Val Tyr Ala Cys Glu Val Thr His Gln Gly Leu Ser Ser Pro Val Thr
210 215 220

Lys Ser Phe Asn Arg Gly Glu Cys
225 230

<210> 84
<211> 231
<212> PRT
<213> artificial

<220>
<223> MB7-VL-CL-114D11

<400> 84

```
Met Arg Trp Ser Trp Ile Phe Leu Leu Leu Leu Ser Ile Thr Ser Ala
1               5                   10                  15

Asn Ala Gln Ile Val Leu Thr Gln Ser Pro Ala Ile Met Ser Ala Ser
            20                  25                  30

Pro Gly Glu Lys Val Thr Ile Thr Cys Ser Ala Ser Ser Ser Val Phe
        35                  40                  45

Tyr Met His Trp Phe Gln Gln Lys Pro Gly Thr Ser Pro Lys Leu Trp
    50                  55                  60

Ile Tyr Ser Thr Ser Asn Leu Ala Ser Gly Val Pro Ala Arg Phe Ser
65                  70                  75                  80

Gly Ser Gly Ser Gly Thr Ser Tyr Ser Leu Thr Ile Ser Arg Met Glu
                85                  90                  95

Ala Glu Asp Ala Ala Thr Tyr Tyr Cys Gln Gln Arg Arg Ser Tyr Pro
            100                 105                 110

Tyr Thr Phe Gly Gly Gly Thr Lys Leu Glu Ile Lys Arg Thr Val Ala
        115                 120                 125

Ala Pro Ser Val Phe Ile Phe Pro Pro Ser Asp Glu Gln Leu Lys Ser
    130                 135                 140

Gly Thr Ala Ser Val Val Cys Leu Leu Asn Asn Phe Tyr Pro Arg Glu
145                 150                 155                 160

Ala Lys Val Gln Trp Lys Val Asp Asn Ala Leu Gln Ser Gly Asn Ser
                165                 170                 175

Gln Glu Ser Val Thr Glu Gln Asp Ser Lys Asp Ser Thr Tyr Ser Leu
            180                 185                 190

Ser Ser Thr Leu Thr Leu Ser Lys Ala Asp Tyr Glu Lys His Lys Val
        195                 200                 205

Tyr Ala Cys Glu Val Thr His Gln Gly Leu Ser Ser Pro Val Thr Lys
    210                 215                 220

Ser Phe Asn Arg Gly Glu Cys
225                 230
```

<210> 85
<211> 231

<212> PRT
<213> artificial

<220>
<223> MB7-VL-CL-104E10

<400> 85

```
Met Arg Trp Ser Trp Ile Phe Leu Leu Leu Leu Ser Ile Thr Ser Ala
1               5                   10                  15

Asn Ala Gln Ile Val Leu Thr Gln Ser Pro Ala Ile Met Ser Ala Ser
            20                  25                  30

Pro Gly Glu Lys Val Thr Met Thr Cys Ser Ala Ser Ser Ser Val Ile
        35                  40                  45

Tyr Met His Trp Phe Gln Gln Lys Pro Gly Thr Ser Pro Lys Leu Trp
    50                  55                  60

Ile Tyr Ser Thr Ser Asn Leu Ala Ser Gly Val Pro Ala Arg Phe Ser
65                  70                  75                  80

Gly Ser Gly Ser Gly Thr Ser Tyr Ser Leu Thr Ile Ser Arg Met Glu
                85                  90                  95

Ala Glu Asp Ala Ala Thr Tyr Tyr Cys Gln Gln Arg Arg Ser Tyr Pro
            100                 105                 110

Tyr Thr Phe Gly Gly Gly Thr Lys Leu Glu Ile Lys Arg Thr Val Ala
        115                 120                 125

Ala Pro Ser Val Phe Ile Phe Pro Pro Ser Asp Glu Gln Leu Lys Ser
    130                 135                 140
```

```
Gly Thr Ala Ser Val Val Cys Leu Leu Asn Asn Phe Tyr Pro Arg Glu
145                 150                 155                 160

Ala Lys Val Gln Trp Lys Val Asp Asn Ala Leu Gln Ser Gly Asn Ser
                165                 170                 175

Gln Glu Ser Val Thr Glu Gln Asp Ser Lys Asp Ser Thr Tyr Ser Leu
            180                 185                 190

Ser Ser Thr Leu Thr Leu Ser Lys Ala Asp Tyr Glu Lys His Lys Val
        195                 200                 205

Tyr Ala Cys Glu Val Thr His Gln Gly Leu Ser Ser Pro Val Thr Lys
    210                 215                 220

Ser Phe Asn Arg Gly Glu Cys
225                 230
```

<210> 86
<211> 354
<212> DNA
<213> artificial

<220>
<223> VH-122A2

<400> 86

```
caggtccagc tgcagcagtc tggggctgag ctggtgaggc ctggggtctc agtgaagatt      60

tcctgcaagg gttctggcta cacattcact gattattcta tgcactgggt gaagcagagt     120

catgcaaaga gtctagagtg gattggagtt attagtactt actatggtga ttctaactat     180

aaccagaagt tcaagggcaa ggccacaatg actgtagaca aatcctccac cacagcctat     240

atggaacttg ccagactgac atctgaggat tctgccatct attactgtgc aagaaatggt     300

aatttctatg ttatggacta ctggggtcaa ggaacctcag tcaccgtctc ctca           354
```

<210> 87
<211> 363
<212> DNA
<213> artificial

<220>
<223> VH-102E9

<400> 87

```
cagatccatt tggtgcagtc tggacctgac ctgaagaagc ctggagagac agtcaagatc        60
tcctgcaagg cttctggtta taccttcaca gactattcaa tgcactgggt gaagcaggct       120
ccaggaaagg gtttaaagtg gatgggctgg ataaacactg agactggtga accaacatat       180
gcagatgact tcaagggacg gtttgccttc tctttggaaa gttctgccag cactgccttt       240
ttgcagatca acaacctcaa aaatgaggac acgtctacat atttctgtac tagaaatggt       300
tactacgtgg gttactatgc tatggactac tggggtcaag gaacctcagt caccgtctcc       360
tca                                                                     363
```

<210> 88
<211> 353
<212> DNA
<213> artificial

<220>
<223> VH-104C12

<400> 88

```
caggtccagc tgcagcagtc tggggctgag ctggtggggc ctggggtctc agtgaagatt        60
tcctgcaagg gttctggcta cacattcact gattattcta tgcactgggt aaagcagagt       120
catgcaaaga gtctagagtg gattggagtt attagtcctt actatggtga tactaactac       180
aaccagaagt tcaagggcaa ggccacaatg actgtagaca aatcctccag cacagcctat       240
atggaacttg ccagtctgac atctgaggat tctgccatct atttctgtgc aagaaatgat       300
gattactaca ggtttgctta ctggggccaa gggactctgg tcactgtctc tgc              353
```

<210> 89
<211> 363
<212> DNA
<213> artificial

<220>
<223> VH-114D11

<400> 89

```
cagatccagt tggtgcagtc tggacctgag ctgaagaagc ctggagagac agtcaagatc       60
tcctgcaagg cttctggtta taccttcaca gactcttcaa tgcactgggt gcagcaggct      120
ccaaacaagg gtttaaagtg gatgggctgg ataaacactg agactggtgg gccaacgtat      180
gcagatgatt tcaagggacg gtttgccttc tctttggaaa cctctgccag aactgcctat      240
ttgcagatca acaacctcaa aaatgaggac acggctacat atttctgtgc tagaaatgga      300
tactacgtgg ggtactatgc tctggactac tggggtcaag gaacctcagt caccgtctcc      360
tca                                                                    363
```

<210> 90
<211> 363
<212> DNA
<213> artificial

<220>
<223> VH-104E10

<400> 90

```
cagatccagt tggtgcagtc tggacctgag ctgaagaagc ctggagagac agtcaagatc       60
tcctgcaagg cttctggtta taccttcaca gactattcaa tgcactgggt gaagcaggct      120
ccaggaaagg gtttaaagtg gatgggctgg ataaacactg agactggtga gccaacatat      180
gcagatgact tcaagggacg gtttgccttc tctttggaaa cctctgccac cactgcctat      240
ttgcagatca acaacttcaa aaatgaggac acggctacat atttctgtgc tagaaatggt      300
tactacgtgg gatattatgc tatggactac tggggtcaag gaacctcagt caccgtctcc      360
tca                                                                    363
```

<210> 91
<211> 321
<212> DNA
<213> artificial

<220>
<223> VL-122A2

<400> 91

gatatccaga tgacacagac tacatcctcc ctgtctgcct ctctgggaga cagagtcacc 60
atcagttgca gggcaagtca ggacattagc aattatttaa actggtatca gcagaaacca 120
gatggaactg ttaaactcct gatctactac acatcaagat tacactcagg agtcccatca 180
aggttcagtg gcagtgggtc tggaacagat tattctctca ccattagcaa cctggaccaa 240
gaagatattg ccacttactt ttgccaacag ggtaatacgc ttccttggac gttcggtgga 300
ggcaccaagc tggaaatcaa a 321

<210> 92
<211> 318
<212> DNA
<213> artificial

<220>
<223> VL-102E9

<400> 92

caaattgttc tcacccagtc tccagcaatc atgtctgcat ctccagggga gaaggtcacc 60
ataacctgca gtgccagctc aagtgtaatt tacattcact ggttccagca gaagccaggc 120
acttctccca aactctggat ttatagcaca tcctacctgg cttctggagt ccctgctcgc 180
ttcagtggca gtggatctgg gacctcttac tctctcacaa tcagccgaat ggaggctgaa 240
gatgctgcca cttattactg ccagcagagg agaagttacc cgttcacgtt cggagggggg 300
accaagctgg aaataaaa 318

<210> 93
<211> 320
<212> DNA
<213> artificial

<220>
<223> VL-104C12

<400> 93

gatctccaga tgacacagac tccatcctcc ctgtctgcct ctctgggaga cagagtcacc 60
atcagttgca gggcaagtca ggacattaac aattatttaa gctggtatca ggagaaacca 120
gatggaactt ttaaactcct gatctactac acatcaagat tacactcagg agtcccatca 180
aggttcagtg gcagtgggtc tggaacagat tattctctca ccgttcgcaa cctggaacag 240
gaagatattg gcacttactt ttgccaacag ggtaaaacgc ttccgtggac gttcggtgga 300
ggcaccaagc tggaaatcag 320

<210> 94
<211> 318

<212> DNA
<213> artificial

<220>
<223> VL-114D11

<400> 94

```
caaattgttc tcacccagtc tccagcaatc atgtctgcat ctccagggga gaaggtcacc        60
ataacctgca gtgccagctc aagtgtattt tacatgcact ggttccagca gaagccaggc       120
acttctccca aactctggat ttatagcaca tccaacctgg cttctggagt ccctgctcgc       180
ttcagtggca gtggatctgg gacctcttac tctctcacaa tcagccgaat ggaggctgaa       240
gatgctgcca cttattactg ccagcaaagg agaagttacc cgtacacgtt cggagggggg       300
accaagctgg aaataaaa                                                     318
```

<210> 95
<211> 318
<212> DNA
<213> artificial

<220>
<223> VL-104E10

<400> 95

```
caaattgttc tcacccagtc tccagcaatc atgtctgcat ctccagggga gaaggtcacc        60
atgacctgca gtgccagttc aagtgtaatt tacatgcact ggttccagca gaagccaggc       120
acttctccca aactctggat ttatagcaca tccaacctgg cttctggagt ccctgctcgc       180
ttcagtggca gtggatctgg gacatcttac tctctcacaa tcagccgaat ggaggctgaa       240
gatgctgcca cttattactg ccagcaaagg agaagttacc cgtacacgtt cggagggggg       300
accaagctgg aaataaaa                                                     318
```

<210> 96
<211> 990
<212> DNA
<213> artificial

<220>
<223> CH

<400> 96

gcctccacca agggcccatc cgtgttcccc ctggccccat ccagcaagtc tacctccgga 60

ggcacagccg ccctgggctg tctggtgaag gactacttcc ccgagccagt gaccgtgtcc 120

tggaactccg gagccctgac atccggcgtg cacaccttcc ccgccgtgct gcagtccagc 180

ggcctgtact ctctgtcttc cgtggtgacc gtgccatcca gctccctggg aacccagaca 240

tacatctgca acgtgaacca caagcctagc aacaccaagg tggacaagaa ggtggagcct 300

aagagctgtg acaagacaca cacatgccct ccttgtccag cccctgagct gctgggcggc 360

ccctccgtgt tcctgttccc ccccaagcct aaggataccc tgatgatcag cagaaccccc 420

gaggtgacct gcgtggtggt ggacgtgtcc cacgaggatc ccgaggtgaa gttcaactgg 480

tacgtggacg gcgtggaggt gcacaacgct aagaccaagc ccagagagga gcagtacaac 540

agcacataca gagtggtgtc tgtgctgacc gtgctgcacc aggactggct gaacgggaag 600

gagtacaagt gcaaggtgtc caacaaggcc ctgcctgccc ctatcgagaa gaccatctct 660

aaggctaagg ggcagccccg ggagccacag gtgtacaccc tgccacccag ccgcgacgag 720

ctgaccaaga accaggtgtc cctgacatgc ctggtgaagg gattctaccc cagcgacatc 780

gccgtggagt gggagagcaa cggccagccc gagaacaact acaagacaac ccctcccgtg 840

ctggacagcg atggatcctt cttcctgtac tccaagctga ccgtggacaa gagcaggtgg 900

cagcagggaa acgtgttctc ttgttccgtg atgcacgagg ctctgcacaa ccactacacc 960

cagaagtccc tgagcctgtc tccaggcaag 990

<210> 97
<211> 321
<212> DNA
<213> artificial

<220>
<223> CL

<400> 97

cgaactgtgg ctgcaccaag tgtcttcatc tttcctccga gtgatgagca gctgaagagc 60

gggacagctt ctgtggtgtg tctgctgaat aacttctacc caagagaagc aaaggtccag 120

tggaaggtgg acaacgccct gcagtctggc aactcacagg agtctgtcac tgagcaggat 180

tccaaggaca gcacttacag cctgtccagc accctcactc tgtccaaagc cgactacgaa 240

aagcataagg tgtatgcttg tgaggtgacc caccagggac tgagcagccc tgtgacgaag 300

tccttcaacc ggggcgagtg c 321

<210> 98
<211> 1344
<212> DNA

<213> artificial

<220>
<223> VH-CH-122A2

<400> 98

```
caggtccagc tgcagcagtc tggggctgag ctggtgaggc ctggggtctc agtgaagatt      60
tcctgcaagg gttctggcta cacattcact gattattcta tgcactgggt gaagcagagt     120
catgcaaaga gtctagagtg gattggagtt attagtactt actatggtga ttctaactat     180
aaccagaagt tcaagggcaa ggccacaatg actgtagaca aatcctccac cacagcctat     240
atggaacttg ccagactgac atctgaggat tctgccatct attactgtgc aagaaatggt     300
aatttctatg ttatggacta ctggggtcaa ggaacctcag tcaccgtctc ctcagcctcc     360
accaagggcc catccgtgtt ccccctggcc ccatccagca agtctacctc cggaggcaca     420
gccgccctgg gctgtctggt gaaggactac ttccccgagc cagtgaccgt gtcctggaac     480
tccggagccc tgacatccgg cgtgcacacc ttccccgccg tgctgcagtc cagcggcctg     540
tactctctgt cttccgtggt gaccgtgcca tccagctccc tgggaaccca gacatacatc     600
tgcaacgtga accacaagcc tagcaacacc aaggtggaca agaaggtgga gcctaagagc     660
tgtgacaaga cacacacatg ccctccttgt ccagcccctg agctgctggg cggcccctcc     720
gtgttcctgt tccccccaa gcctaaggat accctgatga tcagcagaac ccccgaggtg      780
acctgcgtgg tggtggacgt gtcccacgag gatcccgagg tgaagttcaa ctggtacgtg     840
gacggcgtgg aggtgcacaa cgctaagacc aagcccagag aggagcagta caacagcaca     900
tacagagtgg tgtctgtgct gaccgtgctg caccaggact ggctgaacgg gaaggagtac     960
aagtgcaagg tgtccaacaa ggccctgcct gcccctatcg agaagaccat ctctaaggct    1020
aaggggcagc ccgggagcc acaggtgtac accctgccac ccagccgcga cgagctgacc     1080
aagaaccagg tgtccctgac atgcctggtg aagggattct accccagcga catcgccgtg    1140
gagtgggaga gcaacggcca gcccgagaac aactacaaga caacccctcc cgtgctggac    1200
agcgatggat ccttcttcct gtactccaag ctgaccgtgg acaagagcag gtggcagcag    1260
ggaaacgtgt tctcttgttc cgtgatgcac gaggctctgc acaaccacta cacccagaag    1320
tccctgagcc tgtctccagg caag                                           1344
```

<210> 99
<211> 1353
<212> DNA
<213> artificial

<220>
<223> VH-CH-102E9

<400> 99

```
cagatccatt tggtgcagtc tggacctgac ctgaagaagc ctggagagac agtcaagatc      60
tcctgcaagg cttctggtta taccttcaca gactattcaa tgcactgggt gaagcaggct     120
ccaggaaagg gtttaaagtg gatgggctgg ataaacactg agactggtga accaacatat     180
gcagatgact tcaagggacg gtttgccttc tctttggaaa gttctgccag cactgccttt     240
ttgcagatca acaacctcaa aaatgaggac acgtctacat atttctgtac tagaaatggt     300
tactacgtgg gttactatgc tatggactac tggggtcaag gaacctcagt caccgtctcc     360
tcagcctcca ccaagggccc atccgtgttc cccctggccc catccagcaa gtctacctcc     420
ggaggcacag ccgccctggg ctgtctggtg aaggactact tccccgagcc agtgaccgtg     480
tcctggaact ccggagccct gacatccggc gtgcacacct tccccgccgt gctgcagtcc     540
agcggcctgt actctctgtc ttccgtggtg accgtgccat ccagctccct gggaacccag     600
acatacatct gcaacgtgaa ccacaagcct agcaacacca aggtggacaa gaaggtggag     660
cctaagagct gtgacaagac acacacatgc cctccttgtc cagcccctga gctgctgggc     720
ggcccctccg tgttcctgtt ccccccaag cctaaggata ccctgatgat cagcagaacc      780
cccgaggtga cctgcgtggt ggtggacgtg tcccacgagg atcccgaggt gaagttcaac     840
tggtacgtgg acggcgtgga ggtgcacaac gctaagacca agcccagaga ggagcagtac     900
aacagcacat acagagtggt gtctgtgctg accgtgctgc accaggactg gctgaacggg     960
aaggagtaca agtgcaaggt gtccaacaag gccctgcctg cccctatcga gaagaccatc    1020
tctaaggcta aggggcagcc ccgggagcca caggtgtaca ccctgccacc cagccgcgac    1080
gagctgacca agaaccaggt gtccctgaca tgcctggtga agggattcta ccccagcgac    1140
atcgccgtgg agtgggagag caacggccag cccgagaaca actacaagac aacccctccc    1200
gtgctggaca gcgatggatc cttcttcctg tactccaagc tgaccgtgga caagagcagg    1260
tggcagcagg gaaacgtgtt ctcttgttcc gtgatgcacg aggctctgca caaccactac    1320
acccagaagt ccctgagcct gtctccaggc aag                                 1353
```

<210> 100
<211> 1343
<212> **DNA**
<213> artificial

<220>
<223> VH-CH-104C12

<400> 100

```
caggtccagc tgcagcagtc tggggctgag ctggtggggc ctggggtctc agtgaagatt      60
tcctgcaagg gttctggcta cacattcact gattattcta tgcactgggt aaagcagagt     120
catgcaaaga gtctagagtg gattggagtt attagtcctt actatggtga tactaactac     180
aaccagaagt tcaagggcaa ggccacaatg actgtagaca aatcctccag cacagcctat     240
atggaacttg ccagtctgac atctgaggat tctgccatct atttctgtgc aagaaatgat     300
gattactaca ggtttgctta ctggggccaa gggactctgg tcactgtctc tgcgcctcca     360
ccaagggccc atccgtgttc cccctggccc catccagcaa gtctacctcc ggaggcacag     420
ccgccctggg ctgtctggtg aaggactact tccccgagcc agtgaccgtg tcctggaact     480
ccggagccct gacatccggc gtgcacacct tccccgccgt gctgcagtcc agcggcctgt     540
actctctgtc ttccgtggtg accgtgccat ccagctccct gggaacccag acatacatct     600
gcaacgtgaa ccacaagcct agcaacacca aggtggacaa gaaggtggag cctaagagct     660
gtgacaagac acacacatgc cctccttgtc cagcccctga gctgctgggc ggcccctccg     720
tgttcctgtt ccccccaag cctaaggata ccctgatgat cagcagaacc cccgaggtga      780
cctgcgtggt ggtggacgtg tcccacgagg atcccgaggt gaagttcaac tggtacgtgg     840
acggcgtgga ggtgcacaac gctaagacca agcccagaga ggagcagtac aacagcacat     900
acagagtggt gtctgtgctg accgtgctgc accaggactg gctgaacggg aaggagtaca     960
agtgcaaggt gtccaacaag gccctgcctg cccctatcga gaagaccatc tctaaggcta    1020
aggggcagcc ccgggagcca caggtgtaca ccctgccacc cagccgcgac gagctgacca    1080
agaaccaggt gtccctgaca tgcctggtga agggattcta ccccagcgac atcgccgtgg    1140
agtgggagag caacggccag cccgagaaca actacaagac aacccctccc gtgctggaca    1200
gcgatggatc cttcttcctg tactccaagc tgaccgtgga caagagcagg tggcagcagg    1260
gaaacgtgtt ctcttgttcc gtgatgcacg aggctctgca caaccactac acccagaagt    1320
ccctgagcct gtctccaggc aag                                            1343
```

<210> 101
<211> 1353
<212> DNA
<213> artificial

<220>
<223> VH-CH-114D11

<400> 101

cagatccagt tggtgcagtc tggacctgag ctgaagaagc ctggagagac agtcaagatc 60

tcctgcaagg cttctggtta taccttcaca gactcttcaa tgcactgggt gcagcaggct 120

ccaaacaagg gtttaaagtg gatgggctgg ataaacactg agactggtgg gccaacgtat 180

gcagatgatt tcaagggacg gtttgccttc tctttggaaa cctctgccag aactgcctat 240

ttgcagatca acaacctcaa aaatgaggac acggctacat atttctgtgc tagaaatgga 300

tactacgtgg ggtactatgc tctggactac tggggtcaag gaacctcagt caccgtctcc 360

tcagcctcca ccaagggccc atccgtgttc cccctggccc catccagcaa gtctacctcc 420

ggaggcacag ccgccctggg ctgtctggtg aaggactact tccccgagcc agtgaccgtg 480

tcctggaact ccggagccct gacatccggc gtgcacacct tccccgccgt gctgcagtcc 540

agcggcctgt actctctgtc ttccgtggtg accgtgccat ccagctccct gggaacccag 600

acatacatct gcaacgtgaa ccacaagcct agcaacacca aggtggacaa gaaggtggag 660

cctaagagct gtgacaagac acacacatgc cctccttgtc cagcccctga gctgctgggc 720

ggcccctccg tgttcctgtt cccccccaag cctaaggata ccctgatgat cagcagaacc 780

cccgaggtga cctgcgtggt ggtggacgtg tcccacgagg atcccgaggt gaagttcaac 840

tggtacgtgg acggcgtgga ggtgcacaac gctaagacca agcccagaga ggagcagtac 900

aacagcacat acagagtggt gtctgtgctg accgtgctgc accaggactg gctgaacggg 960

aaggagtaca agtgcaaggt gtccaacaag gccctgcctg cccctatcga gaagaccatc 1020

tctaaggcta aggggcagcc ccgggagcca caggtgtaca ccctgccacc cagccgcgac 1080

gagctgacca agaaccaggt gtccctgaca tgcctggtga agggattcta ccccagcgac 1140

atcgccgtgg agtgggagag caacggccag cccgagaaca actacaagac aacccctccc 1200

gtgctggaca gcgatggatc cttcttcctg tactccaagc tgaccgtgga caagagcagg 1260

tggcagcagg gaaacgtgtt ctcttgttcc gtgatgcacg aggctctgca caaccactac 1320

acccagaagt ccctgagcct gtctccaggc aag 1353

<210> 102
<211> 1353
<212> DNA
<213> artificial

<220>
<223> VH-CH-104E10

<400> 102

cagatccagt tggtgcagtc tggacctgag ctgaagaagc ctggagagac agtcaagatc 60
tcctgcaagg cttctggtta taccttcaca gactattcaa tgcactgggt gaagcaggct 120
ccaggaaagg gtttaaagtg gatgggctgg ataaacactg agactggtga gccaacatat 180
gcagatgact tcaagggacg gtttgccttc tctttggaaa cctctgccac cactgcctat 240
ttgcagatca acaacttcaa aaatgaggac acggctacat atttctgtgc tagaaatggt 300
tactacgtgg gatattatgc tatggactac tggggtcaag gaacctcagt caccgtctcc 360
tcagcctcca ccaagggccc atccgtgttc cccctggccc catccagcaa gtctacctcc 420
ggaggcacag ccgccctggg ctgtctggtg aaggactact tccccgagcc agtgaccgtg 480
tcctggaact ccggagccct gacatccggc gtgcacacct tccccgccgt gctgcagtcc 540

agcggcctgt actctctgtc ttccgtggtg accgtgccat ccagctccct gggaacccag 600
acatacatct gcaacgtgaa ccacaagcct agcaacacca aggtggacaa gaaggtggag 660
cctaagagct gtgacaagac acacacatgc cctccttgtc cagcccctga gctgctgggc 720
ggcccctccg tgttcctgtt ccccccaag cctaaggata ccctgatgat cagcagaacc 780
cccgaggtga cctgcgtggt ggtggacgtg tcccacgagg atcccgaggt gaagttcaac 840
tggtacgtgg acggcgtgga ggtgcacaac gctaagacca agcccagaga ggagcagtac 900
aacagcacat acagagtggt gtctgtgctg accgtgctgc accaggactg gctgaacggg 960
aaggagtaca agtgcaaggt gtccaacaag gccctgcctg cccctatcga gaagaccatc 1020
tctaaggcta aggggcagcc ccgggagcca caggtgtaca ccctgccacc cagccgcgac 1080
gagctgacca agaaccaggt gtccctgaca tgcctggtga agggattcta ccccagcgac 1140
atcgccgtgg agtgggagag caacggccag cccgagaaca actacaagac aacccctccc 1200
gtgctggaca gcgatggatc cttcttcctg tactccaagc tgaccgtgga caagagcagg 1260
tggcagcagg gaaacgtgtt ctcttgttcc gtgatgcacg aggctctgca caaccactac 1320
acccagaagt ccctgagcct gtctccaggc aag 1353

<210> 103
<211> 642
<212> DNA
<213> artificial

<220>
<223> VL-CL-122A2

<400> 103

gatatccaga tgacacagac tacatcctcc ctgtctgcct ctctgggaga cagagtcacc 60
atcagttgca gggcaagtca ggacattagc aattatttaa actggtatca gcagaaacca 120
gatggaactg ttaaactcct gatctactac acatcaagat tacactcagg agtcccatca 180
aggttcagtg gcagtgggtc tggaacagat tattctctca ccattagcaa cctggaccaa 240
gaagatattg ccacttactt ttgccaacag ggtaatacgc ttccttggac gttcggtgga 300
ggcaccaagc tggaaatcaa acgaactgtg gctgcaccaa gtgtcttcat ctttcctccg 360
agtgatgagc agctgaagag cgggacagct tctgtggtgt gtctgctgaa taacttctac 420
ccaagagaag caaaggtcca gtggaaggtg gacaacgccc tgcagtctgg caactcacag 480
gagtctgtca ctgagcagga ttccaaggac agcacttaca gcctgtccag caccctcact 540
ctgtccaaag ccgactacga aaagcataag gtgtatgctt gtgaggtgac ccaccaggga 600
ctgagcagcc ctgtgacgaa gtccttcaac cggggcgagt gc 642

<210> 104
<211> 639
<212> DNA
<213> artificial

<220>
<223> VL-CL-102E9

<400> 104

caaattgttc tcacccagtc tccagcaatc atgtctgcat ctccagggga gaaggtcacc 60
ataacctgca gtgccagctc aagtgtaatt tacattcact ggttccagca gaagccaggc 120
acttctccca aactctggat ttatagcaca tcctacctgg cttctggagt ccctgctcgc 180
ttcagtggca gtggatctgg gacctcttac tctctcacaa tcagccgaat ggaggctgaa 240
gatgctgcca cttattactg ccagcagagg agaagttacc cgttcacgtt cggagggggg 300
accaagctgg aaataaaacg aactgtggct gcaccaagtg tcttcatctt tcctccgagt 360
gatgagcagc tgaagagcgg gacagcttct gtggtgtgtc tgctgaataa cttctaccca 420
agagaagcaa aggtccagtg gaaggtggac aacgccctgc agtctggcaa ctcacaggag 480
tctgtcactg agcaggattc caaggacagc acttacagcc tgtccagcac cctcactctg 540
tccaaagccg actacgaaaa gcataaggtg tatgcttgtg aggtgaccca ccagggactg 600
agcagccctg tgacgaagtc cttcaaccgg ggcgagtgc 639

<210> 105
<211> 641
<212> DNA
<213> artificial

<220>

<223> VL-CL-104C12

<400> 105

```
gatctccaga tgacacagac tccatcctcc ctgtctgcct ctctgggaga cagagtcacc      60
atcagttgca gggcaagtca ggacattaac aattatttaa gctggtatca ggagaaacca     120
gatggaactt ttaaactcct gatctactac acatcaagat tacactcagg agtcccatca     180
aggttcagtg gcagtgggtc tggaacagat tattctctca ccgttcgcaa cctggaacag     240
gaagatattg gcacttactt ttgccaacag ggtaaaacgc ttccgtggac gttcggtgga     300
ggcaccaagc tggaaatcag cgaactgtgg ctgcaccaag tgtcttcatc tttcctccga     360
gtgatgagca gctgaagagc gggacagctt ctgtggtgtg tctgctgaat aacttctacc     420
caagagaagc aaaggtccag tggaaggtgg acaacgccct gcagtctggc aactcacagg     480
agtctgtcac tgagcaggat tccaaggaca gcacttacag cctgtccagc accctcactc     540
tgtccaaagc cgactacgaa aagcataagg tgtatgcttg tgaggtgacc caccagggac     600
tgagcagccc tgtgacgaag tccttcaacc ggggcgagtg c                         641
```

<210> 106
<211> 639
<212> DNA
<213> artificial

<220>
<223> VL-CL-114D11

<400> 106

```
caaattgttc tcacccagtc tccagcaatc atgtctgcat ctccagggga gaaggtcacc      60
ataacctgca gtgccagctc aagtgtattt tacatgcact ggttccagca gaagccaggc     120
acttctccca aactctggat ttatagcaca tccaacctgg cttctggagt ccctgctcgc     180
ttcagtggca gtggatctgg gacctcttac tctctcacaa tcagccgaat ggaggctgaa     240
gatgctgcca cttattactg ccagcaaagg agaagttacc cgtacacgtt cggagggggg     300
accaagctgg aaataaaacg aactgtggct gcaccaagtg tcttcatctt tcctccgagt     360
gatgagcagc tgaagagcgg gacagcttct gtggtgtgtc tgctgaataa cttctaccca     420
agagaagcaa aggtccagtg gaaggtggac aacgccctgc agtctggcaa ctcacaggag     480
tctgtcactg agcaggattc caaggacagc acttacagcc tgtccagcac cctcactctg     540
tccaaagccg actacgaaaa gcataaggtg tatgcttgtg aggtgaccca ccagggactg     600
agcagccctg tgacgaagtc cttcaaccgg ggcgagtgc                            639
```

<210> 107
<211> 639

<212> DNA
<213> artificial

<220>
<223> VL-CL-104E10

<400> 107

```
caaattgttc tcacccagtc tccagcaatc atgtctgcat ctccagggga gaaggtcacc        60
atgacctgca gtgccagttc aagtgtaatt tacatgcact ggttccagca gaagccaggc       120
acttctccca aactctggat ttatagcaca tccaacctgg cttctggagt ccctgctcgc       180
ttcagtggca gtggatctgg gacatcttac tctctcacaa tcagccgaat ggaggctgaa       240
gatgctgcca cttattactg ccagcaaagg agaagttacc cgtacacgtt cggagggggg       300
accaagctgg aaataaaacg aactgtggct gcaccaagtg tcttcatctt tcctccgagt       360
gatgagcagc tgaagagcgg gacagcttct gtggtgtgtc tgctgaataa cttctaccca       420
agagaagcaa aggtccagtg gaaggtggac aacgccctgc agtctggcaa ctcacaggag       480
tctgtcactg agcaggattc caaggacagc acttacagcc tgtccagcac cctcactctg       540
tccaaagccg actacgaaaa gcataaggtg tatgcttgtg aggtgaccca ccagggactg       600
agcagccctg tgacgaagtc cttcaaccgg ggcgagtgc                              639
```

<210> 108
<211> 54
<212> DNA
<213> artificial

<220>
<223> MB7 signal peptide

<400> 108
atgaggtggt cctggatctt cctgctgctg ctgagcatca ccagcgccaa cgcc 54

<210> 109
<211> 408
<212> DNA
<213> artificial

<220>
<223> MB7-VH-122A2

<400> 109

```
atgaggtggt cctggatctt cctgctgctg ctgagcatca ccagcgccaa cgcccaggtc        60
cagctgcagc agtctggggc tgagctggtg aggcctgggg tctcagtgaa gatttcctgc       120
aagggttctg gctacacatt cactgattat tctatgcact gggtgaagca gagtcatgca       180
aagagtctag agtggattgg agttattagt acttactatg gtgattctaa ctataaccag       240
aagttcaagg gcaaggccac aatgactgta gacaaatcct ccaccacagc ctatatggaa       300
cttgccagac tgacatctga ggattctgcc atctattact gtgcaagaaa tggtaatttc       360
tatgttatgg actactgggg tcaaggaacc tcagtcaccg tctcctca                    408
```

<210> 110
<211> 417
<212> DNA
<213> artificial

<220>
<223> MB7-VH-102E9

<400> 110

```
atgaggtggt cctggatctt cctgctgctg ctgagcatca ccagcgccaa cgcccagatc        60
catttggtgc agtctggacc tgacctgaag aagcctggag agacagtcaa gatctcctgc       120
aaggcttctg gttatacctt cacagactat tcaatgcact gggtgaagca ggctccagga       180
aagggtttaa agtggatggg ctggataaac actgagactg gtgaaccaac atatgcagat       240
gacttcaagg gacggtttgc cttctctttg gaaagttctg ccagcactgc ctttttgcag       300
atcaacaacc tcaaaaatga ggacacgtct acatatttct gtactagaaa tggttactac       360
gtgggttact atgctatgga ctactggggt caaggaacct cagtcaccgt ctcctca          417
```

<210> 111
<211> 407
<212> DNA
<213> artificial

<220>
<223> MB7-VH-104C12

<400> 111

```
atgaggtggt cctggatctt cctgctgctg ctgagcatca ccagcgccaa cgcccaggtc       60
cagctgcagc agtctggggc tgagctggtg gggcctgggg tctcagtgaa gatttcctgc      120
aagggttctg gctacacatt cactgattat tctatgcact gggtaaagca gagtcatgca      180
aagagtctag agtggattgg agttattagt ccttactatg gtgatactaa ctacaaccag      240
aagttcaagg gcaaggccac aatgactgta gacaaatcct ccagcacagc ctatatggaa      300
cttgccagtc tgacatctga ggattctgcc atctatttct gtgcaagaaa tgatgattac      360
tacaggtttg cttactgggg ccaagggact ctggtcactg tctctgc                    407
```

<210> 112
<211> 417
<212> DNA
<213> artificial

<220>
<223> MB7-VH-114D11

<400> 112

```
atgaggtggt cctggatctt cctgctgctg ctgagcatca ccagcgccaa cgcccagatc       60
cagttggtgc agtctggacc tgagctgaag aagcctggag agacagtcaa gatctcctgc      120
aaggcttctg gttatacctt cacagactct tcaatgcact gggtgcagca ggctccaaac      180
aagggtttaa agtggatggg ctggataaac actgagactg gtgggccaac gtatgcagat      240
gatttcaagg gacggtttgc cttctctttg gaaacctctg ccagaactgc ctatttgcag      300
atcaacaacc tcaaaaatga ggacacggct acatatttct gtgctagaaa tggatactac      360
gtggggtact atgctctgga ctactggggt caaggaacct cagtcaccgt ctcctca         417
```

<210> 113
<211> 417
<212> DNA
<213> artificial

<220>
<223> MB7-VH-104E10

<400> 113

```
atgaggtggt cctggatctt cctgctgctg ctgagcatca ccagcgccaa cgcccagatc       60
cagttggtgc agtctggacc tgagctgaag aagcctggag agacagtcaa gatctcctgc      120
aaggcttctg gttatacctt cacagactat tcaatgcact gggtgaagca ggctccagga      180
aagggtttaa agtggatggg ctggataaac actgagactg gtgagccaac atatgcagat      240
```

gacttcaagg gacggtttgc cttctctttg gaaacctctg ccaccactgc ctatttgcag 300

atcaacaact tcaaaaatga ggacacggct acatatttct gtgctagaaa tggttactac 360

gtgggatatt atgctatgga ctactggggt caaggaacct cagtcaccgt ctcctca 417

<210> 114
<211> 375
<212> DNA
<213> artificial

<220>
<223> MB7-VL-122A2

<400> 114

atgaggtggt cctggatctt cctgctgctg ctgagcatca ccagcgccaa cgccgatatc 60

cagatgacac agactacatc ctccctgtct gcctctctgg gagacagagt caccatcagt 120

tgcagggcaa gtcaggacat tagcaattat ttaaactggt atcagcagaa accagatgga 180

actgttaaac tcctgatcta ctacacatca agattacact caggagtccc atcaaggttc 240

agtggcagtg ggtctggaac agattattct ctcaccatta gcaacctgga ccaagaagat 300

attgccactt acttttgcca acagggtaat acgcttcctt ggacgttcgg tggaggcacc 360

aagctggaaa tcaaa 375

<210> 115
<211> 372
<212> DNA
<213> artificial

<220>
<223> MB7-VL-102E9

<400> 115

atgaggtggt cctggatctt cctgctgctg ctgagcatca ccagcgccaa cgcccaaatt 60

gttctcaccc agtctccagc aatcatgtct gcatctccag gggagaaggt caccataacc 120

tgcagtgcca gctcaagtgt aatttacatt cactggttcc agcagaagcc aggcacttct 180

cccaaactct ggatttatag cacatcctac ctggcttctg gagtccctgc tcgcttcagt 240

ggcagtggat ctgggacctc ttactctctc acaatcagcc gaatggaggc tgaagatgct 300

gccacttatt actgccagca gaggagaagt tacccgttca cgttcggagg ggggaccaag 360

ctggaaataa aa 372

<210> 116
<211> 374
<212> DNA
<213> artificial

<220>
<223> MB7-VL-104C12

<400> 116

```
atgaggtggt cctggatctt cctgctgctg ctgagcatca ccagcgccaa cgccgatctc        60
cagatgacac agactccatc ctccctgtct gcctctctgg gagacagagt caccatcagt       120
tgcagggcaa gtcaggacat taacaattat ttaagctggt atcaggagaa accagatgga       180
acttttaaac tcctgatcta ctacacatca agattacact caggagtccc atcaaggttc       240
agtggcagtg ggtctggaac agattattct ctcaccgttc gcaacctgga acaggaagat       300
attggcactt acttttgcca acagggtaaa acgcttccgt ggacgttcgg tggaggcacc       360
aagctggaaa tcag                                                         374
```

<210> 117
<211> 372
<212> DNA
<213> artificial

<220>
<223> MB7-VL-114D11

<400> 117

```
atgaggtggt cctggatctt cctgctgctg ctgagcatca ccagcgccaa cgcccaaatt        60
gttctcaccc agtctccagc aatcatgtct gcatctccag gggagaaggt caccataacc       120
tgcagtgcca gctcaagtgt attttacatg cactggttcc agcagaagcc aggcacttct       180
cccaaactct ggatttatag cacatccaac ctggcttctg gagtccctgc tcgcttcagt       240
ggcagtggat ctgggacctc ttactctctc acaatcagcc gaatggaggc tgaagatgct       300
gccacttatt actgccagca aaggagaagt tacccgtaca cgttcggagg ggggaccaag       360
ctggaaataa aa                                                           372
```

<210> 118
<211> 372
<212> **DNA**
<213> artificial

<220>
<223> MB7-VL-104E10

<400> 118

```
atgaggtggt cctggatctt cctgctgctg ctgagcatca ccagcgccaa cgcccaaatt        60
gttctcaccc agtctccagc aatcatgtct gcatctccag gggagaaggt caccatgacc       120
tgcagtgcca gttcaagtgt aatttacatg cactggttcc agcagaagcc aggcacttct       180
cccaaactct ggatttatag cacatccaac ctggcttctg gagtccctgc tcgcttcagt       240
ggcagtggat ctgggacatc ttactctctc acaatcagcc gaatggaggc tgaagatgct       300
gccacttatt actgccagca aaggagaagt tacccgtaca cgttcggagg ggggaccaag       360
ctggaaataa aa                                                           372
```

<210> 119
<211> 1398
<212> DNA
<213> artificial

<220>
<223> MB7-VH-CH-122A2

<400> 119

atgaggtggt cctggatctt cctgctgctg ctgagcatca ccagcgccaa cgcccaggtc 60
cagctgcagc agtctggggc tgagctggtg aggcctgggg tctcagtgaa gatttcctgc 120
aagggttctg gctacacatt cactgattat tctatgcact gggtgaagca gagtcatgca 180
aagagtctag agtggattgg agttattagt acttactatg gtgattctaa ctataaccag 240
aagttcaagg gcaaggccac aatgactgta gacaaatcct ccaccacagc ctatatggaa 300
cttgccagac tgacatctga ggattctgcc atctattact gtgcaagaaa tggtaatttc 360
tatgttatgg actactgggg tcaaggaacc tcagtcaccg tctcctcagc ctccaccaag 420
ggcccatccg tgttcccct ggccccatcc agcaagtcta cctccggagg cacagccgcc 480
ctgggctgtc tggtgaagga ctacttcccc gagccagtga ccgtgtcctg gaactccgga 540
gccctgacat ccggcgtgca caccttcccc gccgtgctgc agtccagcgg cctgtactct 600
ctgtcttccg tggtgaccgt gccatccagc tccctgggaa cccagacata catctgcaac 660
gtgaaccaca agcctagcaa caccaaggtg gacaagaagg tggagcctaa gagctgtgac 720
aagacacaca catgccctcc ttgtccagcc cctgagctgc tgggcggccc ctccgtgttc 780
ctgttccccc ccaagcctaa ggataccctg atgatcagca gaacccccga ggtgacctgc 840
gtggtggtgg acgtgtccca cgaggatccc gaggtgaagt tcaactggta cgtggacggc 900
gtggaggtgc acaacgctaa gaccaagccc agagaggagc agtacaacag cacatacaga 960
gtggtgtctg tgctgaccgt gctgcaccag gactggctga acgggaagga gtacaagtgc 1020
aaggtgtcca acaaggccct gcctgcccct atcgagaaga ccatctctaa ggctaagggg 1080
cagccccggg agccacaggt gtacaccctg ccacccagcc gcgacgagct gaccaagaac 1140
caggtgtccc tgacatgcct ggtgaaggga ttctacccca gcgacatcgc cgtggagtgg 1200
gagagcaacg gccagcccga gaacaactac aagacaaccc ctcccgtgct ggacagcgat 1260
ggatccttct tcctgtactc caagctgacc gtggacaaga gcaggtggca gcagggaaac 1320
gtgttctctt gttccgtgat gcacgaggct ctgcacaacc actacaccca gaagtccctg 1380
agcctgtctc caggcaag 1398

<210> 120
<211> 1407
<212> DNA
<213> artificial

<220>
<223> MB7-VH-CH-102E9

<400> 120

atgaggtggt cctggatctt cctgctgctg ctgagcatca ccagcgccaa cgcccagatc 60
catttggtgc agtctggacc tgacctgaag aagcctggag agacagtcaa gatctcctgc 120
aaggcttctg gttatacctt cacagactat tcaatgcact gggtgaagca ggctccagga 180
aagggtttaa agtggatggg ctggataaac actgagactg gtgaaccaac atatgcagat 240
gacttcaagg gacggtttgc cttctctttg gaaagttctg ccagcactgc ctttttgcag 300
atcaacaacc tcaaaaatga ggacacgtct acatatttct gtactagaaa tggttactac 360
gtgggttact atgctatgga ctactggggt caaggaacct cagtcaccgt ctcctcagcc 420
tccaccaagg gcccatccgt gttccccctg gccccatcca gcaagtctac ctccggaggc 480
acagccgccc tgggctgtct ggtgaaggac tacttccccg agccagtgac cgtgtcctgg 540
aactccggag ccctgacatc cggcgtgcac accttccccg ccgtgctgca gtccagcggc 600
ctgtactctc tgtcttccgt ggtgaccgtg ccatccagct ccctgggaac ccagacatac 660
atctgcaacg tgaaccacaa gcctagcaac accaaggtgg acaagaaggt ggagcctaag 720
agctgtgaca agacacacac atgccctcct tgtccagccc ctgagctgct gggcggcccc 780
tccgtgttcc tgttcccccc caagcctaag gataccctga tgatcagcag aacccccgag 840
gtgacctgcg tggtggtgga cgtgtcccac gaggatcccg aggtgaagtt caactggtac 900
gtggacggcg tggaggtgca caacgctaag accaagccca gagaggagca gtacaacagc 960
acatacagag tggtgtctgt gctgaccgtg ctgcaccagg actggctgaa cgggaaggag 1020
tacaagtgca aggtgtccaa caaggccctg cctgccccta tcgagaagac catctctaag 1080
gctaaggggc agccccggga gccacaggtg tacaccctgc cacccagccg cgacgagctg 1140
accaagaacc aggtgtccct gacatgcctg gtgaagggat tctaccccag cgacatcgcc 1200
gtggagtggg agagcaacgg ccagcccgag aacaactaca agacaacccc tcccgtgctg 1260
gacagcgatg gatccttctt cctgtactcc aagctgaccg tggacaagag caggtggcag 1320
cagggaaacg tgttctcttg ttccgtgatg cacgaggctc tgcacaacca ctacacccag 1380
aagtccctga gcctgtctcc aggcaag 1407

<210> 121
<211> 1397
<212> DNA
<213> artificial

<220>
<223> MB7-VH-CH-104C12

<400> 121

```
atgaggtggt cctggatctt cctgctgctg ctgagcatca ccagcgccaa cgcccaggtc      60
cagctgcagc agtctggggc tgagctggtg gggcctgggg tctcagtgaa gatttcctgc     120
aagggttctg gctacacatt cactgattat tctatgcact gggtaaagca gagtcatgca     180
aagagtctag agtggattgg agttattagt ccttactatg gtgatactaa ctacaaccag     240
aagttcaagg gcaaggccac aatgactgta gacaaatcct ccagcacagc ctatatggaa     300
cttgccagtc tgacatctga ggattctgcc atctatttct gtgcaagaaa tgatgattac     360
tacaggtttg cttactgggg ccaagggact ctggtcactg tctctgcgcc tccaccaagg     420
gcccatccgt gttcccctg gccccatcca gcaagtctac ctccggaggc acagccgccc     480
tgggctgtct ggtgaaggac tacttccccg agccagtgac cgtgtcctgg aactccggag     540
ccctgacatc cggcgtgcac accttccccg ccgtgctgca gtccagcggc ctgtactctc     600
tgtcttccgt ggtgaccgtg ccatccagct ccctgggaac ccagacatac atctgcaacg     660
tgaaccacaa gcctagcaac accaaggtgg acaagaaggt ggagcctaag agctgtgaca     720
agacacacac atgccctcct tgtccagccc ctgagctgct gggcggcccc tccgtgttcc     780
tgttcccccc caagcctaag gataccctga tgatcagcag aacccccgag gtgacctgcg     840
tggtggtgga cgtgtcccac gaggatcccg aggtgaagtt caactggtac gtggacggcg     900
tggaggtgca caacgctaag accaagccca gagaggagca gtacaacagc acatacagag     960
tggtgtctgt gctgaccgtg ctgcaccagg actggctgaa cgggaaggag tacaagtgca    1020
aggtgtccaa caaggccctg cctgccccta tcgagaagac catctctaag gctaaggggc    1080
agccccggga gccacaggtg tacaccctgc cacccagccg cgacgagctg accaagaacc    1140
aggtgtccct gacatgcctg gtgaagggat tctaccccag cgacatcgcc gtggagtggg    1200
agagcaacgg ccagcccgag aacaactaca agacaacccc tcccgtgctg gacagcgatg    1260
gatccttctt cctgtactcc aagctgaccg tggacaagag caggtggcag cagggaaacg    1320
tgttctcttg ttccgtgatg cacgaggctc tgcacaacca ctacacccag aagtccctga    1380
gcctgtctcc aggcaag                                                   1397
```

<210> 122
<211> 1407
<212> DNA
<213> artificial

<220>
<223> MB7-VH-CH-114D11

<400> 122

```
atgaggtggt cctggatctt cctgctgctg ctgagcatca ccagcgccaa cgcccagatc      60
cagttggtgc agtctggacc tgagctgaag aagcctggag agacagtcaa gatctcctgc     120
aaggcttctg gttatacctt cacagactct tcaatgcact gggtgcagca ggctccaaac     180
aagggtttaa agtggatggg ctggataaac actgagactg gtgggccaac gtatgcagat     240
gatttcaagg gacggtttgc cttctctttg gaaacctctg ccagaactgc ctatttgcag     300
atcaacaacc tcaaaaatga ggacacggct acatatttct gtgctagaaa tggatactac     360
gtggggtact atgctctgga ctactggggt caaggaacct cagtcaccgt ctcctcagcc     420
tccaccaagg gcccatccgt gttccccctg gccccatcca gcaagtctac ctccggaggc     480
acagccgccc tgggctgtct ggtgaaggac tacttccccg agccagtgac cgtgtcctgg     540
aactccggag ccctgacatc cggcgtgcac accttccccg ccgtgctgca gtccagcggc     600
ctgtactctc tgtcttccgt ggtgaccgtg ccatccagct ccctgggaac ccagacatac     660
atctgcaacg tgaaccacaa gcctagcaac accaaggtgg acaagaaggt ggagcctaag     720
agctgtgaca agacacacac atgccctcct tgtccagccc ctgagctgct gggcggcccc     780
tccgtgttcc tgttcccccc caagcctaag gataccctga tgatcagcag aacccccgag     840
gtgacctgcg tggtggtgga cgtgtcccac gaggatcccg aggtgaagtt caactggtac     900
gtggacggcg tggaggtgca caacgctaag accaagccca gagaggagca gtacaacagc     960
acatacagag tggtgtctgt gctgaccgtg ctgcaccagg actggctgaa cgggaaggag    1020
tacaagtgca aggtgtccaa caaggccctg cctgccccta tcgagaagac catctctaag    1080
gctaaggggc agccccggga gccacaggtg tacaccctgc cacccagccg cgacgagctg    1140
accaagaacc aggtgtccct gacatgcctg gtgaagggat tctaccccag cgacatcgcc    1200
gtggagtggg agagcaacgg ccagcccgag aacaactaca agacaacccc tcccgtgctg    1260
gacagcgatg gatccttctt cctgtactcc aagctgaccg tggacaagag caggtggcag    1320
cagggaaacg tgttctcttg ttccgtgatg cacgaggctc tgcacaacca ctacacccag    1380
aagtccctga gcctgtctcc aggcaag                                        1407
```

<210> 123
<211> 1407
<212> DNA
<213> artificial

<220>
<223> MB7-VH-CH-104E10

<400> 123

```
atgaggtggt cctggatctt cctgctgctg ctgagcatca ccagcgccaa cgcccagatc       60
cagttggtgc agtctggacc tgagctgaag aagcctggag agacagtcaa gatctcctgc      120
aaggcttctg gttatacctt cacagactat tcaatgcact gggtgaagca ggctccagga      180
aagggtttaa agtggatggg ctggataaac actgagactg gtgagccaac atatgcagat      240
gacttcaagg gacggtttgc cttctctttg gaaacctctg ccaccactgc ctatttgcag      300
atcaacaact tcaaaaatga ggacacggct acatatttct gtgctagaaa tggttactac      360
gtgggatatt atgctatgga ctactggggt caaggaacct cagtcaccgt ctcctcagcc      420
tccaccaagg gcccatccgt gttcccccctg gccccatcca gcaagtctac ctccggaggc      480
acagccgccc tgggctgtct ggtgaaggac tacttccccg agccagtgac cgtgtcctgg      540
aactccggag ccctgacatc cggcgtgcac accttccccg ccgtgctgca gtccagcggc      600
ctgtactctc tgtcttccgt ggtgaccgtg ccatccagct ccctgggaac ccagacatac      660
atctgcaacg tgaaccacaa gcctagcaac accaaggtgg acaagaaggt ggagcctaag      720
agctgtgaca agacacacac atgccctcct tgtccagccc ctgagctgct gggcggcccc      780
tccgtgttcc tgttcccccc caagcctaag gataccctga tgatcagcag aacccccgag      840
gtgacctgcg tggtggtgga cgtgtcccac gaggatcccg aggtgaagtt caactggtac      900
gtggacggcg tggaggtgca caacgctaag accaagccca gagaggagca gtacaacagc      960
acatacagag tggtgtctgt gctgaccgtg ctgcaccagg actggctgaa cgggaaggag     1020
tacaagtgca aggtgtccaa caaggccctg cctgccccta tcgagaagac catctctaag     1080
gctaaggggc agccccggga gccacaggtg tacaccctgc cacccagccg cgacgagctg     1140
accaagaacc aggtgtccct gacatgcctg gtgaagggat tctaccccag cgacatcgcc     1200
gtggagtggg agagcaacgg ccagcccgag aacaactaca agacaacccc tcccgtgctg     1260
gacagcgatg gatccttctt cctgtactcc aagctgaccg tggacaagag caggtggcag     1320
cagggaaacg tgttctcttg ttccgtgatg cacgaggctc tgcacaacca ctacacccag     1380
aagtccctga gcctgtctcc aggcaag                                         1407
```

<210> 124
<211> 696
<212> DNA
<213> artificial

<220>
<223> MB7-VL-CL-122A2

<400> 124

```
atgaggtggt cctggatctt cctgctgctg ctgagcatca ccagcgccaa cgccgatatc        60
cagatgacac agactacatc ctccctgtct gcctctctgg gagacagagt caccatcagt       120
tgcagggcaa gtcaggacat tagcaattat ttaaactggt atcagcagaa accagatgga       180
actgttaaac tcctgatcta ctacacatca agattacact caggagtccc atcaaggttc       240
agtggcagtg ggtctggaac agattattct ctcaccatta gcaacctgga ccaagaagat       300
attgccactt acttttgcca acagggtaat acgcttcctt ggacgttcgg tggaggcacc       360
aagctggaaa tcaaacgaac tgtggctgca ccaagtgtct tcatctttcc tccgagtgat       420
gagcagctga agagcgggac agcttctgtg gtgtgtctgc tgaataactt ctacccaaga       480
gaagcaaagg tccagtggaa ggtggacaac gccctgcagt ctggcaactc acaggagtct       540
gtcactgagc aggattccaa ggacagcact tacagcctgt ccagcaccct cactctgtcc       600
aaagccgact acgaaaagca taaggtgtat gcttgtgagg tgacccacca gggactgagc       660
agccctgtga cgaagtcctt caaccggggc gagtgc                                 696
```

<210> 125
<211> 693
<212> DNA
<213> artificial

<220>
<223> MB7-VL-CL-102E9

<400> 125

```
atgaggtggt cctggatctt cctgctgctg ctgagcatca ccagcgccaa cgcccaaatt        60
gttctcaccc agtctccagc aatcatgtct gcatctccag gggagaaggt caccataacc       120
tgcagtgcca gctcaagtgt aatttacatt cactggttcc agcagaagcc aggcacttct       180
cccaaactct ggatttatag cacatcctac ctggcttctg gagtccctgc tcgcttcagt       240
ggcagtggat ctgggacctc ttactctctc acaatcagcc gaatggaggc tgaagatgct       300
gccacttatt actgccagca gaggagaagt tacccgttca cgttcggagg ggggaccaag       360
ctggaaataa aacgaactgt ggctgcacca agtgtcttca tctttcctcc gagtgatgag       420
cagctgaaga gcgggacagc ttctgtggtg tgtctgctga ataacttcta cccaagagaa       480
gcaaaggtcc agtggaaggt ggacaacgcc ctgcagtctg gcaactcaca ggagtctgtc       540
actgagcagg attccaagga cagcacttac agcctgtcca gcaccctcac tctgtccaaa       600
gccgactacg aaaagcataa ggtgtatgct tgtgaggtga cccaccaggg actgagcagc       660
cctgtgacga agtccttcaa ccggggcgag tgc                                    693
```

<210> 126

<211> 695
<212> DNA
<213> artificial

<220>
<223> MB7-VL-CL-104C12

<400> 126

```
atgaggtggt cctggatctt cctgctgctg ctgagcatca ccagcgccaa cgccgatctc      60
cagatgacac agactccatc ctccctgtct gcctctctgg gagacagagt caccatcagt     120
tgcagggcaa gtcaggacat taacaattat ttaagctggt atcaggagaa accagatgga     180
acttttaaac tcctgatcta ctacacatca agattacact caggagtccc atcaaggttc     240
agtggcagtg ggtctggaac agattattct ctcaccgttc gcaacctgga acaggaagat     300
attggcactt acttttgcca acagggtaaa acgcttccgt ggacgttcgg tggaggcacc     360
aagctggaaa tcagcgaact gtggctgcac caagtgtctt catctttcct ccgagtgatg     420
agcagctgaa gagcgggaca gcttctgtgg tgtgtctgct gaataacttc tacccaagag     480
aagcaaaggt ccagtggaag gtggacaacg ccctgcagtc tggcaactca caggagtctg     540
tcactgagca ggattccaag gacagcactt acagcctgtc cagcaccctc actctgtcca     600
aagccgacta cgaaaagcat aaggtgtatg cttgtgaggt gacccaccag ggactgagca     660
gccctgtgac gaagtccttc aaccggggcg agtgc                                695
```

<210> 127
<211> 693
<212> DNA
<213> artificial

<220>
<223> MB7-VL-CL-114D11

<400> 127

atgaggtggt cctggatctt cctgctgctg ctgagcatca ccagcgccaa cgcccaaatt 60
gttctcaccc agtctccagc aatcatgtct gcatctccag gggagaaggt caccataacc 120
tgcagtgcca gctcaagtgt attttacatg cactggttcc agcagaagcc aggcacttct 180
cccaaactct ggatttatag cacatccaac ctggcttctg gagtccctgc tcgcttcagt 240
ggcagtggat ctgggacctc ttactctctc acaatcagcc gaatggaggc tgaagatgct 300
gccacttatt actgccagca aaggagaagt tacccgtaca cgttcggagg ggggaccaag 360
ctggaaataa aacgaactgt ggctgcacca agtgtcttca tctttcctcc gagtgatgag 420
cagctgaaga gcgggacagc ttctgtggtg tgtctgctga ataacttcta cccaagagaa 480
gcaaaggtcc agtggaaggt ggacaacgcc ctgcagtctg gcaactcaca ggagtctgtc 540
actgagcagg attccaagga cagcacttac agcctgtcca gcaccctcac tctgtccaaa 600
gccgactacg aaaagcataa ggtgtatgct tgtgaggtga cccaccaggg actgagcagc 660
cctgtgacga agtccttcaa ccggggcgag tgc 693

<210> 128
<211> 693
<212> DNA
<213> artificial

<220>
<223> MB7-VL-CL-104E10

<400> 128

atgaggtggt cctggatctt cctgctgctg ctgagcatca ccagcgccaa cgcccaaatt 60
gttctcaccc agtctccagc aatcatgtct gcatctccag gggagaaggt caccatgacc 120
tgcagtgcca gttcaagtgt aatttacatg cactggttcc agcagaagcc aggcacttct 180
cccaaactct ggatttatag cacatccaac ctggcttctg gagtccctgc tcgcttcagt 240
ggcagtggat ctgggacatc ttactctctc acaatcagcc gaatggaggc tgaagatgct 300
gccacttatt actgccagca aaggagaagt tacccgtaca cgttcggagg ggggaccaag 360
ctggaaataa aacgaactgt ggctgcacca agtgtcttca tctttcctcc gagtgatgag 420
cagctgaaga gcgggacagc ttctgtggtg tgtctgctga ataacttcta cccaagagaa 480
gcaaaggtcc agtggaaggt ggacaacgcc ctgcagtctg gcaactcaca ggagtctgtc 540
actgagcagg attccaagga cagcacttac agcctgtcca gcaccctcac tctgtccaaa 600
gccgactacg aaaagcataa ggtgtatgct tgtgaggtga cccaccaggg actgagcagc 660
cctgtgacga agtccttcaa ccggggcgag tgc 693

<210> 129

<211> 1313
<212> DNA
<213> Homo sapiens

<220>
<221> misc_feature
<223> CD303 humain (AF293615.1)

<400> 129

```
cagtgattct cgtgcctcag cctcctgagt agccgaaatt acagacgtgt gccaccatgc       60
ttggctaatt ttttggattt ttagtagaga tggggtttca ctatgttggc caggctagtc      120
ttgaactcct ggcctgaagc aatccgccca cctcagcctc ccaaagtgct gagattatag      180
gcacgagcca ctacacctgg ccacaaaatt ctttaaagaa gccaatccca tcctccctca      240
agagccaagg ggccacctca ccctcttgtt acagcagatc ctgcctccca cagtcaccct      300
gctcccaagt gcaacctctg tctgaccctg catggtgtgc ggtgccctcc tgcctcaggc      360
cgcgaagaag gatctaaggg cttggcttgt ttgaaagaac cacaccccga aagtaacatc      420
tttggagaaa gtgatacaag agcttctgca cccacctgat agaggaagtc caaagggtgt      480
gcgcacacac aatggtgcct gaagaagagc ctcaagaccg agagaaagga ctctggtggt      540
tccagttgaa ggtctggtcc atggcagtcg tatccatctt gctcctcagt gtctgtttca      600
ctgtgagttc tgtggtgcct cacaatttta tgtatagcaa aactgtcaag aggctgtcca      660
agttacgaga gtatcaacag tatcatccaa gcctgacctg cgtcatggaa ggaaaggaca      720
tagaagattg gagctgctgc ccaacccctt ggacttcatt tcagtctagt tgctacttta      780
tttctactgg gatgcaatct tggactaaga gtcaaaagaa ctgttctgtg atgggggctg      840
atctggtggt gatcaacacc agggaagaac aggatttcat cattcagaat ctgaaaagaa      900
attcttctta ttttctgggg ctgtcagatc caggggggtcg gcgacattgg caatgggttg      960
accagacacc atacaatgaa aatgtcacat tctggcactc aggtgaaccc aataaccttg     1020
atgagcgttg tgcgataata aatttccgtt cttcagaaga atggggctgg aatgacattc     1080
actgtcatgt acctcagaag tcaatttgca agatgaagaa gatctacata taaatgaaat     1140
attctccctg gaaatgtgtt tgggttggca tccaccgttg tagaaagcta aattgatttt     1200
ttaatttatg tgtaagtttt gtacaaggaa tgcccctaaa atgtttcagc aggctgtcac     1260
ctattacact tatgatataa tccaaaaaaa aaaaaaaaaa aaaaaaaaaa aaa            1313
```

<210> 130
<211> 213
<212> PRT
<213> Homo sapiens

<220>
<221> SITE
<222> (1)..(213)
<223> CD303 humain (AAL37036.1)

<400> 130

```
Met Val Pro Glu Glu Glu Pro Gln Asp Arg Glu Lys Gly Leu Trp Trp
1               5                   10                  15

Phe Gln Leu Lys Val Trp Ser Met Ala Val Val Ser Ile Leu Leu Leu
            20                  25                  30

Ser Val Cys Phe Thr Val Ser Ser Val Val Pro His Asn Phe Met Tyr
        35                  40                  45

Ser Lys Thr Val Lys Arg Leu Ser Lys Leu Arg Glu Tyr Gln Gln Tyr
    50                  55                  60

His Pro Ser Leu Thr Cys Val Met Glu Gly Lys Asp Ile Glu Asp Trp
65                  70                  75                  80

Ser Cys Cys Pro Thr Pro Trp Thr Ser Phe Gln Ser Ser Cys Tyr Phe
                85                  90                  95

Ile Ser Thr Gly Met Gln Ser Trp Thr Lys Ser Gln Lys Asn Cys Ser
            100                 105                 110

Val Met Gly Ala Asp Leu Val Val Ile Asn Thr Arg Glu Glu Gln Asp
        115                 120                 125

Phe Ile Ile Gln Asn Leu Lys Arg Asn Ser Ser Tyr Phe Leu Gly Leu
    130                 135                 140

Ser Asp Pro Gly Gly Arg Arg His Trp Gln Trp Val Asp Gln Thr Pro
```

```
145                 150                 155                 160

Tyr Asn Glu Asn Val Thr Phe Trp His Ser Gly Glu Pro Asn Asn Leu
                165                 170                 175

Asp Glu Arg Cys Ala Ile Ile Asn Phe Arg Ser Ser Glu Glu Trp Gly
            180                 185                 190

Trp Asn Asp Ile His Cys His Val Pro Gln Lys Ser Ile Cys Lys Met
        195                 200                 205

Lys Lys Ile Tyr Ile
    210
```

## Description

La présente invention a pour objet une nouvelle utilisation d'un anticorps dirigé contre une protéine membranaire, et notamment pour le traitement de pathologies.

L'utilisation d'anticorps dirigé contre une protéine membranaire a déjà été décrite dans l'art antérieur.

La demande de brevet européen EP 1 783 141 divulgue par exemple une composition permettant d'induire une réponse immunitaire chez un patient, ladite composition comprenant des cellules dendritiques préalablement traitées avec un anticorps anti-CD303 et exprimant un antigène (tumoral, viral, bactérien...). Ceci consiste en un traitement par thérapie cellulaire.

La demande WO 2006/037247 décrit l'utilisation d'anticorps monoclonaux dirigés contre la protéine CD303, dans le cadre du traitement d'une maladie auto-immune particulière, à savoir le psoriasis.

Nestle *et al.,* (Nestle et al. 2005, J. Exp. Med, vol 202, n°1, pp : 135-143) enseigne que des anticorps dirigés contre la protéine CD303 peuvent être injectés par voie intraveineuse, dans un but thérapeutique dans le cadre du traitement du psoriasis.

Blomberg *et al.,* (Blomberg et al., 2003, Arthritis and Rheumatism, vol 48, n°9, pp : 2524-2532) enseigne l'utilisation d'anticorps dirigés contre la protéine CD303, dans le cadre d'une maladie auto-immune : le Lupus Erythémateux. Les auteurs montrent que lesdits anticorps sont capables d'inhiber la production d'interféron $\alpha$ (IFN-a).

La demande WO 01/36487 décrit des anticorps monoclonaux dirigés contre la protéine CD303, et en particulier les clones AC144, AD5-13A11 et AD5-4B8, ainsi que des fragments dérivés. Par ailleurs, cette demande décrit l'utilisation des anticorps monoclonaux dans le cadre du traitement de pathologies telles que les infections virales, les maladies auto-immunes et les tumeurs. Cependant, cette demande ne décrit aucun exemple spécifique de traitement d'une pathologie à l'aide de l'action indirecte d'un anticorps dirigé contre la protéine CD303.

La demande internationale WO 2012/080642 décrit également l'utilisation d'anticorps dirigés contre la protéine CD303 dans le cadre de la prévention ou du traitement des tumeurs hématopoïétiques de phénotype CD4+/CD56+, qui ont pour origine les cellules dendritiques plasmacytoïdes. La présente invention se distingue de cette demande WO 2012/080642 en ce que l'utilisation des anticorps selon l'invention vise à traiter les tumeurs qui n'ont pas pour origine les cellules dendritiques plasmacytoïdes, ces cellules n'ayant pas le marqueur CD56; lesdites cellules sont dans l'environnement de la tumeur et ont des propriétés immunosuppressives qui favorisent le développement de la tumeur.

Les cellules dendritiques plasmacytoïdes sont à l'origine de telles tumeurs hématopoïétiques de phénotype CD4+/CD56+, lorsqu'elles acquièrent un marqueur supplémentaire qui est le CD56+. C'est pourquoi on parle de tumeurs hématopoïétiques CD4+/CD56+. De telles tumeurs résultent donc du développement tumoral des cellules dendritiques plasmacytoïdes.

Les cellules dendritiques plasmacytoïdes ne sont cependant pas à l'origine de toutes les tumeurs.

Il existe donc un réel besoin de trouver un traitement pour les tumeurs qui n'ont pas pour origine les cellules dendritiques plasmacytoïdes, notamment les tumeurs qui n'ont pas pour origine l'acquisition d'un marqueur supplémentaire tel que le CD56+ par les cellules dendritiques plasmacytoïdes.

C'est pourquoi l'un des buts de l'invention est de fournir un traitement permettant de prévenir et/ou traiter les tumeurs qui n'ont pas pour origine les cellules dendritiques plasmacytoïdes, notamment les tumeurs qui n'ont pas pour origine l'acquisition d'un marqueur supplémentaire par les cellules dendritiques plasmacytoïdes.

C'est pourquoi l'un des buts de l'invention est de prévenir et/ou traiter les tumeurs ne résultant pas du développement tumoral des cellules dendritiques plasmacytoïdes.

La présente invention concerne ainsi une nouvelle utilisation d'un anticorps dirigé contre la protéine membranaire CD303 définie selon les revendications 1 à 13.

La présente invention concerne également un produit selon la revendication 14.

La présente description divulgue également une composition comprenant un mélange d'anticorps dirigés contre une protéine membranaire, et son utilisation pour la prévention et/ou le traitement de tumeurs qui n'ont pas pour origine les cellules dendritiques plasmacytoïdes.

La présente invention repose sur la constatation inattendue des Inventeurs selon laquelle les anticorps dirigés contre une protéine membranaire, notamment la protéine CD303, peuvent être utilisés pour la prévention et/ou le traitement de tumeurs n'ayant pas pour origine l'acquisition d'un marqueur par les cellules dendritiques plasmacytoïdes, en d'autres termes pour la prévention et/ou le traitement de tumeurs n'ayant pas pour origine le développement tumoral des cellules dendritiques plasmacytoïdes.

L'invention se distingue ainsi de l'art antérieur en ce qu'elle supprime les cellules dendritiques plasmacytoïdes par l'action cytotoxique de l'anticorps dirigé contre la protéine CD303, pour prévenir et/ou traiter les tumeurs, bien que ces cellules dendritiques plasmacytoïdes ne soient pas à l'origine de la tumeur. En effet, bien qu'elles ne soient pas à l'origine de la tumeur, les cellules dendritiques plasmacytoïdes peuvent cependant favoriser la croissance des cellules tumorales et leur survie, par exemple en développant des propriétés immunosuppressives et/ou tolérogènes. Ceci peut ainsi inhiber ou diminuer l'efficacité des anti-cancéreux classiquement utilisés en monothérapie. Ainsi, dans le cas de

la présente invention, les anticorps dirigés contre la protéine CD303 ont une action indirecte sur le traitement des tumeurs : les anticorps dirigés contre la protéine CD303 permettent de diminuer les cellules dendritiques plasmacytoïdes dans l'environnement tumoral, et ainsi diminuer, avantageusement supprimer les propriétés immunosuppressives et/ou tolérogènes de ces dernières dans l'environnement tumoral. Selon l'invention, les anticorps dirigés contre la protéine CD303 peuvent ainsi faciliter, stimuler ou potentialiser l'action des anti-cancéreux (par exemple un anticorps anti-antigène tumoral) avec lesquels ils peuvent être utilisés de façon simultanée, séparée ou étalée dans le temps. Les anticorps dirigés contre la protéine CD303 selon l'invention peuvent également présenter une synergie avec les anti-cancéreux avec lesquels il peuvent être utilisés.

Dans un premier aspect, l'invention concerne ainsi un anticorps dirigé contre la protéine CD303 pour son utilisation dans la prévention et/ou le traitement d'une tumeur impliquant une activation de cellules dendritiques plasmacytoïdes dans l'environnement de ladite tumeur, lesdites cellules dendritiques plasmacytoïdes ayant des propriétés immunosuppressives et/ou tolérogènes, et n'étant pas à l'origine de la tumeur et n'ayant pas le marqueur CD56+.

Le terme « anticorps » selon l'invention fait référence à une immunoglobuline, protéine constituée de quatre chaînes participant à la réponse immunitaire acquise. Les immunoglobulines sont bien connues de l'homme de métier et sont constituées d'un assemblage de deux dimères constitués chacun d'une chaîne lourde et d'une chaîne légère. Le complexe multimérique est assemblé par la liaison d'une chaîne légère et d'une chaîne lourde par un pont disulfure entre deux cystéines, les deux chaînes lourdes étant elles-mêmes également reliées entre elles par deux ponts disulfures.

Chacune des chaînes lourdes et des chaînes légères est constituée d'une région constante et d'une région variable. L'assemblage des chaînes qui composent un anticorps permet de définir une structure tridimensionnelle caractéristique en Y, où

- la base du Y correspond à la région constante Fc qui est reconnue par le complément et les récepteurs Fc, et
- l'extrémité des bras du Y correspond à l'assemblage respectif des régions variables de la chaîne légère et de la chaîne lourde qui sont reconnues par un antigène spécifique.

Plus précisément, chaque chaîne légère est constituée d'une région variable ($V_L$) et d'une région constante ($C_L$). Chaque chaîne lourde est constituée d'une région variable ($V_H$) et d'une région constante constituée de trois domaines constants $C_{H1}$, $C_{H2}$ et $C_{H3}$. Les domaines $C_{H2}$ et $C_{H3}$ composent le domaine Fc.

Les régions variables de la chaîne légère et de la chaine lourde sont constituées de trois domaines déterminant la reconnaissance de l'antigène (régions CDR pour Complementary Determining Régions) entourées de quatre domaines charpentes (régions FR pour Framework). Le repliement tridimensionnel de la région variable est tel que les trois CDR sont exposés du même côté de la protéine et permettent la formation d'une structure spécifique reconnaissant un antigène déterminé.

Les anticorps décrits dans l'invention sont isolés et purifiés, et sont différents des anticorps naturels. Ces anticorps sont matures, c'est-à-dire qu'ils possèdent une structure tridimensionnelle *ad hoc* leur permettant de reconnaitre l'antigène, et possèdent toutes les modifications post-traductionnelles essentielles à leur reconnaissance antigénique.

Selon l'invention un « anticorps dirigé contre la protéine CD303 » se réfère à un anticorps «anti-CD303 ».

Le terme « protéine CD303 » fait référence à la protéine autrefois nommée la protéine BDCA-2. Cette protéine est exprimée de manière spécifique à la surface des cellules dendritiques plasmacytoïdes, et est une protéine de type II appartenant aux lectines de type C. En d'autres termes, l'antigène CD303 humain (ou protéine CD303) est le membre C de la 4$^{ème}$ famille de domaine lectine de type C et est également appelé CLEC4 (pour « C-type lectin domain family 4, member C ») ; DLEC; HECL; BDCA2; CLECSF7; CLECSF11; ou PRO34150 (voir le site EntrezGene pour le gène CLEC4). Il s'agit d'une glycoprotéine transmembranaire de type II de 213 acides aminés, comprenant un court domaine cytoplasmique sans motif de signalisation évident (acides aminés 1-21), une région transmembranaire (acides aminés 22-41), un domaine cou (acides aminés 42-82), et un domaine extracellulaire de reconnaissance de carbohydrates (CRD pour « carbohydrate recognition domain » ; acides aminés 83-213) (Dzionek et al-2001). La séquence de l'ARNm codant pour cette protéine peut être trouvée sur la base de données Genbank dans sa version du 14 février 2002 sous le n° d'accession AF293615.1 (SEQ ID NO : 129), tandis que la séquence d'acides aminés est accessible sur la base de données Genbank dans sa version du 14 février 2002 sous le n° d'accession AAL37036.1 (SEQ ID NO : 130).

L'expression « cellules dendritiques plasmacytoïdes » fait référence à la sous population de cellules dendritiques aussi appelée DC2. Les cellules dendritiques plasmacytoïdes sont caractérisées par les marqueurs Lin- (CD3-, CD19-, CD20-, CD14-, CD56-), HLA-DR+, CDllc-, CD123+ et CD45RA+. Ces cellules ont aussi été caractérisées phénotypiquement : elles expriment les marqueurs CD4 et CD303 et BDCA-4. Elles sont présentes dans les organes lymphoïdes et sont également en circulation dans le sang. Les cellules dendritiques plasmacytoïdes ont la capacité de sécréter de l'IFN de type I en présence d'une infection virale.

L'expression « activation de cellules dendritiques plasmacytoïdes dans l'environnement de ladite tumeur » fait référence aux différents mécanismes ayant pour effet d'activer la prolifération, la sécrétion de cytokines déterminées, notamment les interférons de classe I, et la modification phénotypique et morphologique des cellules plasmacytoïdes.

Les cellules dendritiques plasmacytoïdes peuvent également favoriser la croissance des cellules tumorales et leur survie, en induisant notamment un environnement immunosuppressif dans l'environnement de la tumeur, par exemple en induisant la différenciation de lymphocytes T régulateurs (Treg). Ainsi, l'expression « activation de cellules dendritiques plasmacytoïdes dans l'environnement de ladite tumeur » fait également référence aux propriétés immunosuppressives et/ou tolérogènes que les cellules dendritiques plasmacytoïdes peuvent avoir vis-à-vis de la tumeur.

L'expression « lesdites cellules dendritiques plasmacytoïdes n'étant pas à l'origine de la tumeur » signifie que les tumeurs selon la présente invention ne sont pas liées au développement tumoral des cellules dendritiques plasmacytoïdes. Plus précisément, les tumeurs selon la présente invention ne sont pas liées à l'acquisition par les cellules dendritiques plasmacytoïdes d'un marqueur supplémentaire tel que le CD56.

Le terme « prévention » doit s'entendre comme la prévention de l'expansion tumorale *in situ,* ou bien comme la prévention du développement de métastases à partir de tumeurs déjà infiltrées dans leur environnement par de cellules dendritiques plasmacytoïdes.

Dans un aspect particulier de l'invention, les cellules dendritiques plasmacytoïdes ont des propriétés immunosuppressives et/ou tolérogènes.

L'expression « propriétés immunosuppressives » se réfère aux propriétés des cellules dendritiques de développer et maintenir l'immunosuppression dans l'environnement de la tumeur.

L'expression « propriétés tolérogènes » signifie que les cellules dendritiques plasmacytoïdes ne vont pas induire de réponse immunitaire.

Dans un aspect particulier de l'invention, les tumeurs impliquant une activation des cellules dendritiques plasmacytoïdes sont des tumeurs solides ou des tumeurs hématopoïétiques.

Le terme « tumeurs solides » fait référence à une masse cellulaire résultant d'une multiplication excessive de cellules. Les tumeurs solides peuvent se développer dans n'importe quel tissu, notamment la peau, les muqueuses, les os ou les organes, ... Les tumeurs solides peuvent être distinguées en deux groupes : les carcinomes et les sarcomes.

Les carcinomes sont issus de cellules épithéliales (présentes dans la peau, les muqueuses ou les glandes) ; il s'agit par exemple des cancers du sein, des poumons, de la prostate, de l'intestin, ...

Les sarcomes sont issus de cellules des tissus conjonctifs ; il s'agit par exemple des cancers de l'os, du cartilage, ....

Le terme « tumeurs hématopoïétiques » fait référence aux tumeurs impliquant des cellules de la lignée sanguine, ou cellules hématopoïétiques, ou aux tumeurs qui affectent les organes hématopoïétiques, à savoir les organes capables d'hématopoïèse qui participent à l'élaboration des cellules sanguines. Les organes hématopoïétiques sont la moelle osseuse et ceux formant le tissu lymphoïde (le thymus, les ganglions et la rate par exemple). Les tumeurs hématopoïétiques peuvent également être appelées hémopathies malignes.

Dans un aspect particulier de l'invention, les tumeurs solides impliquent une infiltration de cellules dendritiques plasmacytoïdes dans l'environnement de ladite tumeur, et appartiennent de préférence au groupe des tumeurs de la tête et du cou, du mélanome, des cancers urogénitaux, du cancer du sein.

L'expression « les tumeurs solides impliquent une infiltration de cellules dendritiques plasmacytoïdes dans l'environnement de ladite tumeur » signifie que les cellules dendritiques plasmacatyoïdes sont recrutées sur le site de la tumeur. Les cellules dendritiques plasmacytoïdes recrutées sur le site de la tumeur possèdent notamment des propriétés immunosuppressives et/ou tolérogènes. Les cellules dendritiques plasmacytoïdes infiltrées dans l'environnement de la tumeur peuvent également induire la différentiation des lymphocytes T régulateurs et/ou induire l'infiltration de ces derniers sur le site de la tumeur.

L'expression « tumeurs de la tête et du cou » fait référence aux cancers de la cavité buccale, du pharynx, du nasopharynx, du larynx, des fosses nasales, des sinus, ou des glandes salivaires.

Le terme « mélanome » fait référence à une tumeur maligne qui se développe à partir de cellules de la peau appelées mélanocytes. Quatre principaux types de mélanome de la peau existent : le mélanome superficiel extensif, le mélanome nodulaire, le mélanome de Dubreuilh et le mélanome acrolentigineux.

Le terme « cancers urogénitaux » fait référence au cancer de l'appareil uro-génital dans les organes masculins ou féminins. Il s'agit par exemple du cancer de la prostate, du cancer du testicule, du cancer du pénis, du cancer de l'endomètre, du cancer de la vulve et du vagin, du cancer de l'utérus, du cancer du col de l'utérus, du cancer de l'ovaire, du cancer du rein, du cancer de la vessie.

Le terme « cancer du sein » fait référence au cancer des glandes mammaires, qu'il soit non invasif (Carcinome Canalaire *In Situ* / Intracanalaire (CCIS)) ou invasif.

Dans un aspect particulier de l'invention, les tumeurs hématopoïétiques appartiennent au groupe du myélome multiple, du lymphome, de la leucémie, notamment la leucémie à cellules T.

Le terme « myélome multiple » fait référence à une maladie de la moelle osseuse caractérisée par la multiplication dans la moelle osseuse d'un plasmocyte anormal.

Le terme « lymphome » fait référence aux tumeurs dans lesquelles les cellules sanguines prolifèrent anormalement dans les organes lymphoïdes secondaires (ganglions, rate, ...).

Le terme « leucémie » fait référence aux tumeurs dans lesquelles les cellules sanguines prolifèrent anormalement dans le sang.

Dans un aspect particulier de l'invention, ledit anticorps pour son utilisation susmentionnée est monoclonal ou polyclonal.

Le terme « monoclonal » se réfère à un anticorps qui ne reconnait qu'un seul déterminant antigénique dans CD303, contrairement aux anticorps polyclonaux qui correspondent à un mélange d'anticorps monoclonaux, et donc peuvent reconnaitre plusieurs déterminants antigéniques dans une même protéine.

Le terme « anticorps monoclonal » ou « composition d'anticorps monoclonal » se réfère à une composition comprenant des molécules d'anticorps possédant une spécificité antigénique identique et unique. Les molécules d'anticorps présentes dans la composition sont susceptibles de varier au niveau de leurs modifications post-traductionnelles, et notamment au niveau de leurs structures de glycosylation ou de leur point isoélectrique, mais ont toutes été codées par les mêmes séquences de chaines lourde et légère et ont donc, avant toute modification post-traductionnelle, la même séquence protéique. Certaines différences de séquences protéiques, liées à des modifications post-traductionnelles (comme par exemple le clivage de la lysine C-terminale de la chaine lourde, la déamidation de résidus asparagine et/ou l'isomérisation de résidus aspartate), peuvent néanmoins exister entre les différentes molécules d'anticorps présentes dans la composition.

Les anticorps monoclonaux de l'invention peuvent être obtenus par des techniques bien connues de l'homme de métier, notamment la technique de fusion cellulaire, la technique de clonage des séquences des chaines lourdes et légères, la technique de phage ou ribosome display, par immunisation de souris ayant le répertoire des immunoglobulines humaines et expression dans une cellule *ad hoc* ou un animal transgénique.

Dans un aspect particulier de l'invention, ledit anticorps pour son utilisation susmentionnée est choisi parmi un anticorps murin, un anticorps chimérique, un anticorps humanisé ou un anticorps humain.

Dans un aspect particulier de l'invention, ledit anticorps pour son utilisation susmentionnée est un anticorps chimérique, et de préférence un anticorps chimérique choisi parmi un anticorps chimérique murin/humain ou un anticorps chimérique humain/macaque.

Dans un aspect particulier de l'invention, l'anticorps, fragment fonctionnel ou dérivé de celui-ci selon l'invention est avantageusement un anticorps chimérique ou humanisé, en particulier un anticorps chimérique dont la région constante des chaines lourdes et légères est d'origine humaine.

Le terme « anticorps murin » fait référence à un anticorps dont les séquences des chaines lourdes et des chaines légères qui le constituent sont des séquences dont on retrouve la correspondance en acide nucléique dans le génome des cellules B murines. Cet anticorps est donc constitué de séquences d'acides aminés murines, quelle que soit l'origine de la cellule qui permet sa production. Par exemple, les séquences d'anticorps de souris exprimées dans des cellules de macaque donneront des anticorps murins.

La définition ci-dessus s'applique *mutatis mutandis* aux anticorps humains.

Le terme « anticorps chimérique » fait référence à un anticorps isolé, dans lequel la séquence de chaque chaîne légère et/ou de chaque chaîne lourde qui le constitue comprend ou consiste en une séquence hybride issue d'au moins deux animaux distincts. Notamment, les anticorps chimériques de l'invention sont des hybrides homme/macaque ou homme/souris, ce qui signifie qu'une région de la séquence des chaînes légères et des chaînes lourdes est issue de la séquence d'une immunoglobuline de macaque ou de souris, et que le reste de la séquence desdites chaînes lourdes et desdites chaînes légères est issue de la séquence d'une, ou éventuellement plusieurs, immunoglobuline humaine. Le terme « anticorps chimérique » se réfère également à un anticorps qui contient une région variable (chaîne légère et chaîne lourde) naturelle dérivée d'un anticorps d'une espèce donnée en association avec les régions constantes de chaîne légère et chaîne lourde d'un anticorps d'une espèce hétérologue à ladite espèce donnée. Avantageusement, si la composition d'anticorps monoclonal pour son utilisation en tant que médicament selon l'invention comprend un anticorps monoclonal chimérique, celui-ci comprend des régions constantes humaines. Partant d'un anticorps non humain, un anticorps chimérique peut être préparé en utilisant les techniques de recombinaison génétique bien connues de l'homme du métier. Par exemple, l'anticorps chimérique pourra être réalisé en clonant pour la chaine lourde et la chaine légère un ADN recombinant comportant un promoteur et une séquence codant pour la région variable de l'anticorps non humain, et une séquence codant pour la région constante d'un anticorps humain. Pour les méthodes de préparation d'anticorps chimériques, on pourra par exemple se référer au document Verhoeyn et al-1988.

Le terme « anticorps humanisé » fait référence à un anticorps issu d'un animal autre que l'homme dans lequel les séquences des chaines lourdes et des chaines légères autres que les CDR ont été remplacées par des séquences correspondantes d'un ou plusieurs anticorps d'origine humaine. L'anticorps est donc majoritairement constitué de séquences humaines, mais sa spécificité pour l'antigène conférée par les CDRs est issue d'une autre espèce. Le terme « anticorps humanisé » fait également référence à un anticorps qui contient des régions CDRs dérivées d'un anticorps d'origine non humaine, les autres parties de la molécule d'anticorps étant dérivées d'un (ou de plusieurs) anticorps humains. En outre, certains des résidus des segments du squelette (dénommés FR) peuvent être modifiés pour conserver l'affinité de liaison (Jones et al-1986 ; Verhoeyen et al- 1988 ; Riechmann et al-1988). Les anticorps humanisés selon

l'invention peuvent être préparés par des techniques connues de l'homme de l'art telles les technologies de « CDR grafting », de « resurfacing », de SuperHumanisation, de « Human string content », de « FR libraries », de « Guided selection », de « FR shuffling » et de « Humaneering », comme résumé dans la revue de Almagro et al-2008.

Dans un aspect particulier de l'invention, ledit anticorps pour son utilisation susmentionnée est un anticorps monoclonal dirigé contre l'ectodomaine de l'antigène CD303 humain (SEQ ID NO : 130), caractérisé en ce que :

a) il entre en compétition pour la liaison à l'antigène CD303 humain avec au moins un anticorps choisi parmi :

i) Un anticorps dont la région variable de chaine lourde comprend la séquence SEQ ID NO : 43 et la région variable de chaine légère comprend la séquence SEQ ID NO : 48 ;
ii) Un anticorps dont la région variable de chaine lourde comprend la séquence SEQ ID NO : 44 et la région variable de chaine légère comprend la séquence SEQ ID NO : 49 ;
iii) Un anticorps dont la région variable de chaine lourde comprend la séquence SEQ ID NO : 45 et la région variable de chaine légère comprend la séquence SEQ ID NO : 50 ;
iv) Un anticorps dont la région variable de chaine lourde comprend la séquence SEQ ID NO : 46 et la région variable de chaine légère comprend la séquence SEQ ID NO : 51 ;
v) Un anticorps dont la région variable de chaine lourde comprend la séquence SEQ ID NO : 47 et la région variable de chaine légère comprend la séquence SEQ ID NO : 52 ; et

b) les régions constantes des chaînes légères et des chaînes lourdes sont des régions constantes provenant d'une espèce non-murine.

Avantageusement, les chaînes lourdes comprennent trois CDR-H (CDR de chaîne lourde selon la nomenclature IMGT) ayant les séquences d'acides aminés suivantes, ou des séquences ayant au moins 80% d'identité avec les séquences suivantes, et les chaînes légères comprennent trois CDR-L (CDR de chaîne légère selon la nomenclature IMGT) ayant les séquences d'acides aminés suivantes, ou des séquences ayant au moins 80% d'identité avec les séquences suivantes :

i) CDR1-H-famille 1 : SEQ ID NO : 1, CDR2-H-famille 1 : SEQ ID NO : 2, CDR3-H-famille 1 : SEQ ID NO : 3, CDR1-L-famille 1 : SEQ ID NO : 4, CDR2-L-famille 1 : SEQ ID NO : 5, CDR3-L-famille 1 : SEQ ID NO : 6; ou
ii) CDR1-H-famille 2 : SEQ ID NO : 7, CDR2-H-famille 2 : SEQ ID NO : 8, CDR3-H-famille 2 : SEQ ID NO : 9, CDR1-L-famille 2 : SEQ ID NO : 10, CDR2-L-famille 2 : SEQ ID NO : 11, CDR3-L-famille 2 : SEQ ID NO : 12.

L'expression « au moins 80% d'identité » signifie une identité de 80%, 81%, 82%, 83%, 84%, 85%, 86%, 87%, 88%, 89%, 90%, 91%, 92%, 93%, 94%, 95%, 96%, 97%, 98%, 99% et 100%.

Le **Tableau 1** ci-dessous résume les séquences d'acides aminés des CDR-IMGT des deux familles d'anticorps pouvant être utilisées selon l'invention :

**Tableau 1**. Séquences d'acides aminés des CDR des deux familles d'anticorps pouvant être utilisées selon l'invention selon la nomenclature IMGT. Dans chaque séquence, X peut représenter n'importe quel acide aminé.

| | Famille 1 | Famille 2 |
|---|---|---|
| CDR1-H | GYTFTDYS (SEQ ID NO : 1) | GYTFTDXS (SEQ ID NO : 7) |
| CDR2-H | ISXYYGDX (SEQ ID NO : 2) | INTETGXP (SEQ ID NO : 8) |
| CDR3-H | ARNXXXYXXXY (SEQ ID NO : 3) | XRNGYYVGYYAXDY (SEQ ID NO : 9) |
| CDR1-L | QDIXNY (SEQ ID NO : 4) | SSVXY (SEQ ID NO : 10) |
| CDR2-L | YTS (SEQ ID NO : 5) | STS (SEQ ID NO : 11) |
| CDR3-L | QQGXTLPWT (SEQ ID NO : 6) | QQRRSYPXT (SEQ ID NO : 12) |

Avantageusement, les chaînes lourdes d'un anticorps selon l'invention comprennent trois CDR-H (CDR de chaîne lourde selon la nomenclature IMGT) ayant les séquences d'acides aminés suivantes, ou des séquences ayant au moins 80% d'identité avec les séquences suivantes, et les chaînes légères comprennent trois CDR-L (CDR de chaîne légère selon la nomenclature IMGT) ayant les séquences d'acides aminés suivantes, ou des séquences ayant au moins 80% d'identité avec les séquences suivantes :

i) CDR1-H-122A2 : SEQ ID NO : 13, CDR2-H-122A2 : SEQ ID NO : 14, CDR3-H-122A2 : SEQ ID NO : 15, CDR1-L-122A2 : SEQ ID NO : 16, CDR2-L-122A2 : SEQ ID NO : 17, CDR3-L-122A2 : SEQ ID NO : 18;

ii) CDR1-H-102E9 : SEQ ID NO : 19, CDR2-H-102E9: SEQ ID NO : 20, CDR3-H-102E9: SEQ ID NO : 21, CDR1-L-102E9: SEQ ID NO : 22, CDR2-L-102E9: SEQ ID NO : 23, CDR3-L-102E9: SEQ ID NO : 24;

iii) CDR1-H-104C12 : SEQ ID NO : 25, CDR2-H-104C12: SEQ ID NO : 26, CDR3-H-104C12: SEQ ID NO : 27, CDR1-L-104C12: SEQ ID NO : 28, CDR2-L-104C12: SEQ ID NO : 29, CDR3-L-104C12: SEQ ID NO : 30;

iv) CDR1-H-114D11: SEQ ID NO : 31, CDR2-H-114D11 : SEQ ID NO : 32, CDR3-H-114D11: SEQ ID NO : 33, CDR1-L-114D11: SEQ ID NO : 34, CDR2-L-114D11: SEQ ID NO : 35, CDR3-L-114D11: SEQ ID NO : 36; ou

v) CDR1-H-104E10: SEQ ID NO : 37, CDR2-H-104E10: SEQ ID NO : 38, CDR3-H-104E10 : SEQ ID NO : 39, CDR1-L-104E10: SEQ ID NO : 40, CDR2-L-104E10: SEQ ID NO : 41, CDR3-L-104E10: SEQ ID NO : 42.

Avantageusement, les chaines lourdes d'un anticorps, selon l'invention comprennent une région variable ayant une séquence choisie parmi SEQ ID NO : 43 à 47 ou une séquence ayant au moins 80% d'identité avec l'une de SEQ ID NO : 43 à 47.

En outre ou alternativement, les chaines légères d'un anticorps, selon l'invention comprennent une région variable ayant une séquence choisie parmi SEQ ID NO : 48 à 52 ou une séquence ayant au moins 80% d'identité avec l'une de SEQ ID NO : 48 à 52.

Dans un mode de réalisation préféré, l'anticorps, selon l'invention possède des chaînes lourdes et légères dont les régions variables ont les séquences d'acides aminés suivantes ou des séquences ayant au moins 80% d'identité avec les séquences suivantes :

i) Anticorps 122A2: chaîne lourde : SEQ ID NO : 43, chaîne légère : SEQ ID NO : 48,
ii) Anticorps 102E9: chaîne lourde : SEQ ID NO : 44, chaîne légère : SEQ ID NO : 49,
iii) Anticorps 104C12: chaîne lourde : SEQ ID NO : 45, chaîne légère : SEQ ID NO : 50,
iv) Anticorps 114D11: chaîne lourde : SEQ ID NO : 46, chaîne légère : SEQ ID NO : 51, ou
v) Anticorps 104E10: chaîne lourde : SEQ ID NO : 47, chaîne légère : SEQ ID NO : 52.

**Le Tableau 2** ci-dessous résume les segments de gène VH, JH et VL et JL murins utilisés par les différents anticorps selon l'invention et le pourcentage d'identité.

**Tableau 2**. Segments VH, JH et VL et JL murins utilisés par les différents anticorps selon l'invention, tels que définis par IMGT.

| Anticorps | VH | JH | VL | JL |
|---|---|---|---|---|
| 122A2 | IGHV1S 137*01 (94.9% ) | IGHJ2*02 (85%) | IGKV10-96*01 (98.9%) | IGKJ1*01 (100%) |
| 102E9 | IGHV9-2-1*01 (93.9%) | IGHJ4*01 (100%) | IGKV4-57*01 (95.7%) | IGKJ1*02 (100%) |
| 104C 12 | IGHV1S 137*01 (91.8%) | IGHJ3*01 (100%) | IGKV10-96*02 (89.5%) | IGKJ1*02 (100%) |
| 114D11 | IGHV9-2-1*01 (94.9%) | IGHJ4*01 (94.1%) | IGKV4-57*01 (97.9%) | IGKJ1*02 (100%) |
| 104E10 | IGHV9-2-1*01 (98%) | IGHJ4*01 (100%) | IGKV4-57*01 (96.8%) | IGKJ1*02 (100%) |

**Le Tableau 3** ci-dessous résume les séquences d'acides aminés des CDRs et des régions variables des chaînes lourdes et légères des anticorps anti-CD303 générés par les inventeurs, selon l'invention :

**Tableau 3.** Séquences d'acides aminés des CDR1, CDR2, et CDR3 des chaines lourdes et légères selon la nomenclature IMGT, et des fragments VH et VL des anticorps selon l'invention.

| | |
|---|---|
| **Anticorps 122A2** | |
| Chaîne lourde | |
| CDR1-H-IMGT-122A2 | GYTFTDYS (SEQ ID NO : 13) |
| CDR2-H-IMGT-122A2 | ISTYYGDS (SEQ ID NO : 14) |
| CDR3-H-IMGT-122A2 | ARNGNFYVMDY (SEQ ID NO : 15) |
| VH-122A2 | QVQLQQSGAELVRPGVSVKISCKGSGYTFTDYSMHWVKQSHAKSLEWIGVISTYYGDSNYNQKFKGKATMTVDKSSTTAYMELARLTSEDSAIYYCARNGNFYVMDYWGQGTSVTVSS (SEQ ID NO : 43) |
| Chaîne légère | |
| CDR1-L-IMGT-122A2 | QDISNY (SEQ ID NO : 16) |
| CDR2-L-IMGT-122A2 | YTS (SEQ ID NO : 17) |
| CDR3-L-IMGT-122A2 | QQGNTLPWT (SEQ ID NO : 18) |
| VL-122A2 | DIQMTQTTSSLSASLGDRVTISCRASQDISNYLNWYQQKPDGTVKLLIYYTSRLHSGVPSRFSGSGSGTDYSLTISNLDQEDIATYFCQQGNTLPWTFGGGTKLEIK (SEQ ID NO : 48) |
| **Anticorps 102E9** | |
| Chaîne lourde | |
| CDR1-H-IMGT-102E9 | GYTFTDYS (SEQ ID NO : 19) |
| CDR2-H-IMGT-102E9 | INTETGEP (SEQ ID NO : 20) |
| CDR3-H-IMGT-102E9 | TRNGYYVGYYAMDY (SEQ ID NO : 21) |
| VH-102E9 | QIHLVQSGPDLKKPGETVKISCKASGYTFTDYSMHWVKQAPGKGLKWMGWINTETGEPTYADDFKGRFAFSLESSASTAFLQINNLKNEDTSTYFCTRNGYYVGYYAMDYWGQGTSVTVSS (SEQ ID NO : 44) |

(suite)

| | |
|---|---|
| Chaîne légère | |
| CDR1-L-IMGT-102E9 | SSVIY (SEQ ID NO : 22) |
| CDR2-L-IMGT-102E9 | STS (SEQ ID NO : 23) |
| CDR3-L-IMGT-102E9 | QQRRSYPFT (SEQ ID NO : 24) |
| VL-102E9 | QIVLTQSPAIMSASPGEKVTITCSASSSVIYIHWFQQKPGTSPKLWIYSTSYLASGVPARFSGSGSGTSYSLTISRMEAEDAATYYCQQRRSYPFTFGGGTKLEIK (SEQ ID NO : 49) |
| **Anticorps 104C12** | |
| Chaîne lourde | |
| CDR1-H-IMGT-104C12 | GYTFTDYS (SEQ ID NO : 25) |
| CDR2-H-IMGT-104C12 | ISPYYGDT (SEQ ID NO : 26) |
| CDR3-H-IMGT-104C12 | ARNDDYYRFAY (SEQ ID NO : 27) |
| VH-104C12 | QVQLQQSGAELVGPGVSVKISCKGSGYTFTDYSMHWVKQSHAKSLEWIGVISPYYGDTNYNQKFKGKATMTVDKSSSTAYMELASLTSEDSAIYFCARNDDYYRFAYWGQGTLVTVSA (SEQ ID NO : 45) |
| Chaîne légère | |
| CDR1-L-IMGT-104C12 | QDINNY (SEQ ID NO : 28) |
| CDR2-L-IMGT-104C12 | YTS (SEQ ID NO : 29) |
| CDR3-L-IMGT-104C12 | QQGKTLPWT (SEQ ID NO : 30) |
| VL-104C12 | DLQMTQTPSSLSASLGDRVTISCRASQDINNYLSWYQEKPDGTFKLLIYYTSRLHSGVPSRFSGSGSGTDYSLTVRNLEQEDIGTYFCQQGKTLPWTFGGGTKLEIR (SEQ ID NO : 50) |

(suite)

| Anticorps 114D11 | |
|---|---|
| Chaîne lourde | |
| CDR1-H-IMGT-114D11 | GYTFTDSS (SEQ ID NO : 31) |
| CDR2-H-IMGT-114D11 | INTETGGP (SEQ ID NO : 32) |
| CDR3-H-IMGT-114D11 | ARNGYYVGYYALDY (SEQ ID NO : 33) |
| VH-114D11 | QIQLVQSGPELKKPGETVKISCKASGYTFTDSSMHWVQQAPNKGLKWMGWINTETGGPTYADDFKGRFAFSLETSARTAYLQINNLKNEDTATYFCARNGYYVGYYALDYWGQGTSVTVSS (SEQ ID NO : 46) |
| Chaîne légère | |
| CDR1-L-IMGT-114D11 | SSVFY (SEQ ID NO : 34) |
| CDR2-L-IMGT-114D11 | STS (SEQ ID NO : 35) |
| CDR3-L-IMGT-114D11 | QQRRSYPYT (SEQ ID NO : 36) |
| VL-114D11 | QIVLTQSPAIMSASPGEKVTITCSASSSVFYMHWFQQKPGTSPKLWIYSTSNLASGVPARFSGSGSGTSYSLTISRMEAEDAATYYCQQRRSYPYTFGGGTKLEIK (SEQ ID NO : 51) |
| **Anticorps 104E10** | |
| Chaîne lourde | |
| CDR1-H-IMGT-104E10 | GYTFTDYS (SEQ ID NO : 37) |
| CDR2-H-IMGT-104E10 | INTETGEP (SEQ ID NO : 38) |
| CDR3-H-IMGT-104E10 | ARNGYYVGYYAMDY (SEQ ID NO : 39) |
| VH-104E10 | QIQLVQSGPELKKPGETVKISCKASGYTFTDYSMHWVKQAPGKGLKWMGWINTETGEPTYADDFKGRFAFSLETSATTAYLQINNFKNEDTATYFCARNGYYVGYYAMDYWGQGTSVTVSS (SEQ ID NO : 47) |

(suite)

| Chaîne légère | |
|---|---|
| CDR1-L-IMGT-104E10 | SSVIY (SEQ ID NO : 40) |
| CDR2-L-IMGT-104E10 | STS (SEQ ID NO : 41) |
| CDR3-L-IMGT-104E10 | QQRRSYPYT (SEQ ID NO : 42) |
| VL-104E10 | QIVLTQSPAIMSASPGEKVTMTCSASSSVIYMHWFQQKPGTSPKLWIYST SNLASGVPARFSGSGSGTSYSLTISRMEAEDAATYYCQQRRSYPYTFGGG TKLEIK (SEQ ID NO : 52) |

Dans un aspect particulier de l'invention, ledit anticorps chimérique, humanisé ou humain, pour son utilisation susmentionnée, est un anticorps dont la région variable de la chaine lourde est représentée par la séquence SEQ ID NO : 43 ou une séquence ayant au moins 80% d'identité avec ladite SEQ ID NO : 43, et dont la région variable de la chaine légère est représentée par la séquence SEQ ID NO : 48 ou une séquence ayant au moins 80% d'identité avec ladite SEQ ID NO : 48 (anticorps 122A2).

Dans un aspect particulier de l'invention, ledit anticorps chimérique, humanisé ou humain, pour son utilisation susmentionnée, est un anticorps dont la région variable de la chaine lourde est représentée par la séquence SEQ ID NO : 44 ou une séquence ayant au moins 80% d'identité avec ladite SEQ ID NO : 44, et dont la région variable de la chaine légère est représentée par la séquence SEQ ID NO : 49 ou une séquence ayant au moins 80% d'identité avec ladite SEQ ID NO : 49 (anticorps 102E9).

Dans un aspect particulier de l'invention, ledit anticorps chimérique, humanisé ou humain, pour son utilisation susmentionnée, est un anticorps dont la région variable de la chaine lourde est représentée par la séquence SEQ ID NO : 45 ou une séquence ayant au moins 80% d'identité avec ladite SEQ ID NO : 45, et dont la région variable de la chaine légère est représentée par la séquence SEQ ID NO : 50 ou une séquence ayant au moins 80% d'identité avec ladite SEQ ID NO : 50 (anticorps 104C12).

Dans un aspect particulier de l'invention, ledit anticorps chimérique, humanisé ou humain, pour son utilisation susmentionnée, est un anticorps dont la région variable de la chaine lourde est représentée par la séquence SEQ ID NO : 46 ou une séquence ayant au moins 80% d'identité avec ladite SEQ ID NO : 46, et dont la région variable de la chaine légère est représentée par la séquence SEQ ID NO : 51 ou une séquence ayant au moins 80% d'identité avec ladite SEQ ID NO : 51 (anticorps 114D11).

Dans un aspect particulier de l'invention, ledit anticorps chimérique, humanisé ou humain, pour son utilisation susmentionnée, est un anticorps dont la région variable de la chaine lourde est représentée par la séquence SEQ ID NO : 47 ou une séquence ayant au moins 80% d'identité avec ladite SEQ ID NO : 47, et dont la région variable de la chaine légère est représentée par la séquence SEQ ID NO : 52 ou une séquence ayant au moins 80% d'identité avec ladite SEQ ID NO : 52 (anticorps 104E10).

Dans un aspect particulier de l'invention, ledit anticorps chimérique, humanisé ou humain, pour son utilisation susmentionnée, est un anticorps dont la chaine lourde est représentée par la séquence SEQ ID NO : 55 ou une séquence ayant au moins 80% d'identité avec ladite SEQ ID NO : 55, et dont la chaine légère est représentée par la séquence SEQ ID NO: 60 ou une séquence ayant au moins 80% d'identité avec ladite SEQ ID NO: 60 (anticorps 122A2).

Dans un aspect particulier de l'invention, ledit anticorps chimérique, humanisé ou humain, pour son utilisation susmentionnée, est un anticorps dont la chaine lourde est représentée par la séquence SEQ ID NO : 56 ou une séquence ayant au moins 80% d'identité avec ladite SEQ ID NO : 56, et dont la chaine légère est représentée par la séquence SEQ ID NO: 61 ou une séquence ayant au moins 80% d'identité avec ladite SEQ ID NO: 61 (anticorps 102E9).

Dans un aspect particulier de l'invention, ledit anticorps chimérique, humanisé ou humain, pour son utilisation susmentionnée, est un anticorps dont la chaine lourde est représentée par la séquence SEQ ID NO :57 ou une séquence ayant au moins 80% d'identité avec ladite SEQ ID NO : 57, et dont la chaine légère est représentée par la séquence SEQ ID NO: 62 ou une séquence ayant au moins 80% d'identité avec ladite SEQ ID NO: 62 (anticorps 104C12).

Dans un aspect particulier de l'invention, ledit anticorps chimérique, humanisé ou humain, pour son utilisation susmentionnée, est un anticorps dont la chaine lourde est représentée par la séquence SEQ ID NO : 58 ou une séquence ayant au moins 80% d'identité avec ladite SEQ ID NO : 58, et dont la chaine légère est représentée par la séquence SEQ ID NO: 63 ou une séquence ayant au moins 80% d'identité avec ladite SEQ ID NO: 63 (anticorps 114D11).

Dans un aspect particulier de l'invention, ledit anticorps chimérique, humanisé ou humain, pour son utilisation susmentionnée, est un anticorps dont la chaine lourde est représentée par la séquence SEQ ID NO : 59 ou une séquence ayant au moins 80% d'identité avec ladite SEQ ID NO : 59, et dont la chaine légère est représentée par la séquence SEQ ID NO: 64 ou une séquence ayant au moins 80% d'identité avec ladite SEQ ID NO: 64 (anticorps 104E10).

Les pourcentages d'identité auxquels il est fait référence dans le cadre de l'exposé de la présente invention sont déterminés sur la base d'un alignement global des séquences à comparer, c'est-à-dire sur un alignement des séquences prises dans leur intégralité sur toute la longueur en utilisant tout algorithme bien connu de l'homme du métier tel que l'algorithme de Needleman et Wunsch-1970. Cette comparaison de séquences peut être effectuée à l'aide de tout logiciel bien connu de l'homme du métier, par exemple le logiciel needle en utilisant le paramètre « Gap open » égal à 10.0, le paramètre « Gap extend » égal à 0.5 et une matrice « Blosum 62 ». Le logiciel needle est par exemple disponible sur le site internet ebi.ac.uk world wide sous la dénomination « Align ».

Lorsque le CDR ou la région variable d'un anticorps pouvant être utilisés selon l'invention possède une séquence d'acides aminés qui n'est pas 100% identique à l'une de celles décrites ci-dessus et dans le listing de séquences (séquences de référence) mais qui possède au moins 80%, de préférence au moins 85%, au moins 90%, au moins 95%, au moins 96%, au moins 97%, au moins 98%, au moins 99% d'identité avec une telle séquence de référence, elle peut présenter des insertions, délétions ou substitutions par rapport à la séquence de référence. Lorsqu'il s'agit de substitutions, la substitution est de préférence réalisée par un acide aminé « équivalent », c'est-à-dire tout acide aminé dont la structure est proche de celle de l'acide aminé d'origine et est donc peu probable de modifier les activités biologiques de l'anticorps. Des exemples de telles substitutions sont présentés dans le **Tableau 4** suivant :

**Tableau 4**. Substitutions par des acides aminés équivalents

| **Acide aminé d'origine** | **Substitution(s)** |
|---|---|
| Ala (A) | Val, Gly, Pro |
| Arg (R) | Lys, His |
| Asn (N) | Gln |
| Asp (D) | Glu |
| Cys (C) | Ser |
| Gln (Q) | Asn |
| Glu (G) | Asp |
| Gly (G) | Ala |
| His (H) | Arg |
| Ile (I) | Leu |
| Leu (L) | Ile, Val, Met |
| Lys (K) | Arg |
| Met (M) | Leu |
| Phe (F) | Tyr |
| Pro (P) | Ala |
| Ser (S) | Thr, Cys |
| Thr (T) | Ser |
| Trp (W) | Tyr |
| Tyr (Y) | Phe, Trp |
| Val (V) | Leu, Ala |

Les anticorps peuvent être de plusieurs isotypes, en fonction de la nature de leur région constante : les régions

constantes $\gamma$, $\alpha$, $\mu$, $\varepsilon$ et $\delta$ correspondent respectivement aux immunoglobulines IgG, IgA, IgM, IgE et IgD. Avantageusement, l'anticorps monoclonal présent dans une composition utilisée en tant que médicament dans le cadre de l'invention est d'isotype IgG. En effet, cet isotype montre une capacité à engendrer une activité ADCC (« Antibody-Dependent Cellular Cytotoxicity », soit Cytotoxicité cellulaire dépendante de l'anticorps) chez le plus grand nombre d'individus (humains). Les régions constantes $\gamma$ comprennent plusieurs sous-types : $\gamma$1, $\gamma$2, $\gamma$3, ces trois types de régions constantes présentant la particularité de fixer le complément humain, et $\gamma$4, créant ainsi les sous-isotypes IgG1, IgG2, IgG3, et IgG4. Avantageusement, l'anticorps monoclonal présent dans une composition utilisée en tant que médicament dans le cadre de l'invention est d'isotype IgG1 ou IgG3, de préférence IgG1.

Le fragment Fc d'un anticorps pouvant être utilisé selon l'invention peut être naturel, tel que défini ci-dessus, ou bien avoir été modifié de diverses façons, à condition de comprendre un domaine de liaison aux récepteurs FcR (récepteurs FcyR pour les IgG) fonctionnel, et de préférence un domaine de liaison au récepteur FcRn fonctionnel. Les modifications peuvent inclure la délétion de certaines parties du fragment Fc, pourvu que celui-ci contienne un domaine de liaison aux récepteurs FcR (récepteurs FcyR pour les IgG) fonctionnel, et de préférence un domaine de liaison au récepteur FcRn fonctionnel. Les modifications peuvent également inclure différentes substitutions d'acides aminés susceptibles d'affecter les propriétés biologiques de l'anticorps, pourvu que celui-ci contienne un domaine de liaison aux récepteurs FcR fonctionnel, et de préférence un domaine de liaison au récepteur FcRn fonctionnel. En particulier, lorsque l'anticorps est un IgG, il peut comprendre des mutations destinées à augmenter la liaison au récepteur FcyRIIIa (CD16a), telles que décrites dans WO00/42072, Shields et al-2001, Lazar et al-2006, WO2004/029207, WO2004/063351, WO2004/074455. Des mutations permettant d'augmenter la liaison au récepteur FcRn et donc la demi-vie *in vivo* peuvent également être présentes, comme décrit par exemple dans Shields et al-2001, Dall'Acqua et al-2002, Hinton et al-2004, Dall'Acqua et al-2006(a), WO00/42072, WO02/060919, WO2010/045193, ou WO2010/106180. D'autres mutations, comme celles permettant de diminuer ou d'augmenter la liaison aux protéines du complément et donc la réponse CDC, peuvent ou non être présentes (voir WO99/51642, WO2004/074455, Idusogie et al-2001, Dall'Acqua et al-2006(b), et Moore et al-2010).

Dans un aspect particulier de l'invention, des mutants préférés pouvant être utilisés sont ceux comprenant des mutations permettant d'augmenter la liaison au récepteur FcRn et donc la demi-vie *in vivo,* et sont donc les mutants comprenant les combinaisons de mutations suivantes dans leur fragment Fc, décrites dans WO2010/106180 :

- N315D/A330V/N361D/A378V/N434Y,
- P230S/N315D/M428L/N434Y,
- E294del/T307P/N434Y,
- T307A/N315D/A330V/E382V/N389T/N434Y,
- V259I/N315D/N434Y, ou
- T256N/A378V/S383N/N434Y,

où la numérotation des acides aminés dans le fragment Fc est celle de l'index EU décrit dans Kabat et al., Sequences of Proteins of Immunological Interest, 5th Ed. Public Health Service, National Institutes of Health, Bethesda, Md. (1991).

Le terme « index EU » ou « index EU de Kabat » se réfère à la numérotation des acides aminés de l'anticorps IgG1 humain.

Dans un autre aspect de l'invention, les fragments Fc des anticorps pouvant être utilisés selon l'invention portent au moins une mutation choisie parmi :
G316D, K326E, N315D, N361H, P396L, T350A, V284L, V323I, P352S, A378V, Y436H, V266M, N421T, G385R, K326T, H435R, K447N, N434K, K334N, V397M, E283G, A378T, F423L, A431V, F423S, N325S, P343S, K290E, S375R, F405V, K322E, K340E, N389S, F243I, T307P, N389T, S442F, K248E, Y349H, N286I, T359A, S383R, K334R, T394P, V259A, T393A, P352L, Q418P, V302A, L398P, F423P, S442P, V363I, S383N, S254F, K320E, G402D, I253F, V284A, A431T, N315H, Y319H, C226Y, F405L, T393I, N434S, R255W, A287T, N286Y, A231V, K274R, V308G, K414R, M428T, E345G, F243L, P247T, Q362R, S440N, Y278H, D312G, V262A, V305A, K246R, V308I, E380G, N276S, K439Q, S267G, F423Y, A231T, K320R, L410R, K320M, V412M, T307N, T366A, P230S, Y349S, A339T, K246E, K274E, A231P, I336T, S298N, L234P, S267N, V263A, E333G, V308A, K439R, K392R, S440G, V397I, I336V, Y373D, K288E, L309P, P227S, V379A, K288R, K320T, V282A, I377T, N421S et C261R,
la numérotation étant celle de l'index EU ou équivalent dans Kabat.

Dans un autre aspect de l'invention, les fragments Fc des anticorps pouvant être utilisés portent au moins une combinaison de 2 mutations, ladite combinaison étant choisie parmi :

(i) une mutation choisie parmi 307N, 326E, 326T, 334N, 334R, 352L, 378V, 378T, 394P, 396L, 397M et 421T et ;
(ii) au moins une mutation choisie parmi 226Y, 227S, 230S, 231V, 234P, 243I, 243L, 246R, 246E, 247T, 248E, 253F, 254F, 255W, 259A, 261R, 262A, 263A, 266M, 267N, 267G, 274E, 274R, 276S, 278H, 282A, 283G, 284L, 286I, 286Y, 287T, 288E, 288R, 290E, 298N, 302A, 305A, 307P, 308A, 308I, 308G, 309P, 312G, 315D, 316D, 319H,

320T, 320R, 320M, 322E, 323I, 325S, 333G, 334N, 334R, 336T, 339T, 340E, 343S, 345G, 349S, 349H, 350A 352S, 359A, 361H, 362R, 363I, 366A, 373D, 375R, 377T, 378V, 378T, 379A, 380G, 383R, 385R, 389S, 389T, 392R, 393A, 393I, 394P, 396L, 3971, 397M, 398P, 405V, 405L, 410R, 412M, 414R, 421T, 421S, 423L, 423Y, 423S, 423P, 428T, 431V, 431T, 434K, 434S, 435R, 436H, 439R, 440G, 440N, 442F, 442P, et 447N,

la numérotation étant celle de l'index EU ou équivalent dans Kabat et avec la condition que la mutation (i) n'a pas lieu sur le même acide aminé que la mutation (ii).

De préférence, les fragments Fc mutés des anticorps pouvant être utilisés selon l'invention une affinité augmentée pour le complément C1q, et comprenent au moins une combinaison de 2 mutations, ladite combinaison comprenant :

i) une mutation choisie parmi 378V, 378T, 396L, 421T, 334R et 326E; et
ii) au moins une mutation choisie parmi 361H, 290E, 316D, 248E, 410R, 421T, 334R, 394P, 307P, 447N, 378V, 284L, 421T, 396L, 286I, 315D et 397M,

la numérotation étant celle de l'index EU ou équivalent dans Kabat et avec la condition que la mutation (i) n'a pas lieu sur le même acide aminé que la mutation (ii).

De préférence, les fragments Fc mutés des anticorps pouvant être utilisés selon l'invention ont une affinité augmentée pour le récepteur FcγRIIIa (CD16a), et comprend au moins une combinaison de 2 mutations, ladite combinaison comprenant:

i) une mutation choisie parmi 378V, 326E, 397M, 334N et 396L ; et
ii) au moins une mutation choisie parmi 316D, 397M, 334N, 248E, 231V, 246R, 336T, 421T, 361H, 366A, 439R, 290E, 394P, 307P, 378V, 378T, 286I, 286Y et 298N,

la numérotation étant celle de l'index EU ou équivalent dans Kabat et avec la condition que la mutation (i) n'a pas lieu sur le même acide aminé que la mutation (ii).

De préférence, les fragments Fc mutés des anticorps pouvant être utilisés selon l'invention ont une affinité augmentée pour le récepteur FcγRIIa (CD32a), et comprend au moins une combinaison de 2 mutations, ladite combinaison comprenant:

i) une mutation choisie parmi 378V, 326E, 397M, 307N, 394P, 326T, 396L et 334N ; et
ii) au moins une mutation choisie parmi: 316D, 334R, 334N, 323I, 231V, 246R, 336T, 378T, 286Y, 286I, 352S, 383R, 359A, 421T, 361H, 315D, 366A, 290E, 307P et 439R,

la numérotation étant celle de l'index EU ou équivalent dans Kabat et avec la condition que la mutation (i) n'a pas lieu sur le même acide aminé que la mutation (ii).

De préférence, les fragments Fc mutés des anticorps pouvant être utilisés selon l'invention comprennent au moins une combinaison de 3 mutations, ladite combinaison comprenant :

(i) une mutation choisie parmi 326E, 326T, 352L, 378V, 378T, 396L, 397M, 421T, 334N, 334R, 307N et 394P ; et
(ii) au moins 2 mutations choisies parmi 226Y, 227S, 230S, 231V, 234P, 2431, 243L, 246R, 246E, 247T, 248E, 253F, 254F, 255W, 259A, 261R, 262A, 263A, 266M, 267N, 267G, 274E, 274R, 276S, 278H, 282A, 283G, 284L, 286I, 286Y, 287T, 288E, 288R, 290E, 298N, 302A, 305A, 307P, 308A, 3081, 308G, 309P, 312G, 315D, 316D, 319H, 320T, 320R, 320M, 322E, 323I, 325S, 333G, 334N, 334R, 336T, 339T, 340E, 343S, 345G, 349S, 349H, 350A 352S, 359A, 361H, 362R, 363I, 366A, 373D, 375R, 377T, 378V, 378T, 379A, 380G, 383R, 385R, 389S, 389T, 392R, 393A, 393I, 394P, 396L, 397I, 397M, 398P, 405V, 405L, 410R, 412M, 414R, 421T, 421S, 423L, 423Y, 423S, 423P, 428T, 431V, 431T, 434K, 434S, 435R, 436H, 439R, 440G, 440N, 442F, 442P et 447N,

la numérotation étant celle de l'index EU ou équivalent dans Kabat et avec la condition que la mutation (i) n'a pas lieu sur le même acide aminé que la mutation (ii).

Un anticorps pour son utilisation selon l'invention, qui est chimérique avec des régions constantes humaines, ou bien humanisé, comprendra avantageusement une région constante de chaine lourde humaine ayant pour séquence d'acides aminés SEQ ID NO : 53. De plus ou de manière alternative, un anticorps pour son utilisation selon l'invention, qui est chimérique avec des régions constantes humaines, ou bien humanisé, comprendra avantageusement une région constante de chaine légère humaine ayant pour séquence d'acides aminés SEQ ID NO : 54. Les séquences préférées de région constante de chaine lourde ou légère humaine, SEQ ID NO: 53 et SEQ ID NO: 54, d'isotype IgG1, sont présentées dans le **Tableau 5** ci-dessous.

**Tableau 5**. Séquences préférées de région constante de chaine lourde ou légère humaine SEQ ID NO : 53 et SEQ ID NO : 54.

| | |
|---|---|
| Région constante de chaine lourde humaine préférée (IgG1) | ASTKGPSVFPLAPSSKSTSGGTAALGCLVKDYFPEPVTVSWNSG ALTSGVHTFPAVLQSSGLYSLSSVVTVPSSSLGTQTYICNVNHK PSNTKVDKKVEPKSCDKTHTCPPCPAPELLGGPSVFLFPPKPKD TLMISRTPEVTCVVVDVSHEDPEVKFNWYVDGVEVHNAKTKP REEQYNSTYRVVSVLTVLHQDWLNGKEYKCKVSNKALPAPIE KTISKAKGQPREPQVYTLPPSRDELTKNQVSLTCLVKGFYPSDI AVEWESNGQPENNYKTTPPVLDSDGSFFLYSKLTVDKSRWQQ GNVFSCSVMHEALHNHYTQKSLSLSPG (SEQ ID NO :53) |
| Région constante de chaine légère humaine préférée (IgG1) | RTVAAPSVFIFPPSDEQLKSGTASVVCLLNNFYPREAKVQWKV DNALQSGNSQESVTEQDSKDSTYSLSSTLTLSKADYEKHKVYA CEVTHQGLSSPVTKSFNRGEC (SEQ ID NO :54) |

Ainsi, les chaines lourdes et légères des anticorps pour leur utilisation selon l'invention comprennent avantageusement les séquences décrites dans le Tableau 6 ci-dessous.

**Tableau 6**. Séquences d'acides aminés des chaines lourdes et légères des anticorps selon l'invention.

| Anticorps | Chaine lourde | Chaine légère |
|---|---|---|
| 122A2 | Fusion SEQ ID NO : 43-SEQ ID NO:53 (SEQ ID NO:55) | Fusion SEQ ID NO : 48-SEQ ID NO:54 (SEQ ID NO:60) |
| 102E9 | Fusion SEQ ID NO : 44-SEQ ID NO:53 (SEQ ID NO:56) | Fusion SEQ ID NO : 49-SEQ ID NO:54 (SEQ ID NO:61) |
| 104C12 | Fusion SEQ ID NO : 45-SEQ ID NO:53 (SEQ ID NO:57) | Fusion SEQ ID NO : 50-SEQ ID NO:54 (SEQ ID NO:62) |
| 114D11 | Fusion SEQ ID NO : 46-SEQ ID NO:53 (SEQ ID NO:58) | Fusion SEQ ID NO : 51-SEQ ID NO:54 (SEQ ID NO:63) |
| 104E 10 | Fusion SEQ ID NO : 47-SEQ ID NO:53 (SEQ ID NO:59) | Fusion SEQ ID NO : 52-SEQ ID NO:54 (SEQ ID NO:64) |

La chaine lourde et/ou la chaine légère de l'anticorps pour son utilisation selon l'invention comprend avantageusement en outre au moins un peptide signal hétérologue de séquence SEQ ID NO : 65 (MRWSWIFLLLLSITSANA, peptide signal MB7). En effet, ce peptide a été montré comme permettant d'améliorer l'expression et la sécrétion de protéines recombinantes dans des lignées cellulaires eucaryotes supérieures (voir WO2011/114063). Ainsi, les chaines lourdes des anticorps pour leur utilisation selon l'invention comprennent avantageusement une séquence d'acides aminés choisie parmi les séquences SEQ ID NO : 66 à 70, constituées de la fusion de N- en C-terminal entre la séquence d'acides aminés du peptide signal MB7 (SEQ ID NO :65) et d'une des séquences d'acides aminés de la région VH des anticorps selon l'invention (SEQ ID NO : 43 à 47). De plus ou de manière alternative, les chaines légères des anticorps pour leur utilisation selon l'invention comprennent avantageusement une séquence d'acides aminés choisie parmi les séquences SEQ ID NO : 71 à 75, constituées de la fusion de N- en C-terminal entre la séquence d'acides aminés du peptide signal MB7 (SEQ ID NO :65) et d'une des séquences d'acides aminés de la région VL des anticorps selon l'invention (SEQ ID NO : 48 à 52). En ajoutant les régions constantes de chaine lourde et de chaine légère préférées, on obtient des séquences d'acides aminés complètes préférées des anticorps pour leur utilisation selon l'invention, tel que décrit dans le **Tableau 7** ci-dessous.

**Tableau 7**. Séquences d'acides aminés des chaines lourdes et légères des anticorps selon l'invention, avec le peptide signal MB7.

| Anticorps | Chaine lourde | Chaine légère |
|---|---|---|
| 122A2 | Fusion SEQ ID NO :65-SEQ ID NO : 43-SEQ ID NO:53 (SEQ ID NO:76) | Fusion SEQ ID NO :65-SEQ ID NO : 48-SEQ ID NO:54 (SEQ ID NO:81) |
| 102E9 | Fusion SEQ ID NO :65-SEQ ID NO : 44-SEQ ID NO:53 (SEQ ID NO:77) | Fusion SEQ ID NO :65-SEQ ID NO : 49-SEQ ID NO:54 (SEQ ID NO:82) |
| 104C12 | Fusion SEQ ID NO :65-SEQ ID NO : 45-SEQ ID NO:53 (SEQ ID NO:78) | Fusion SEQ ID NO :65-SEQ ID NO : 50-SEQ ID NO:54 (SEQ ID NO:83) |
| 114D11 | Fusion SEQ ID NO :65-SEQ ID NO : 46-SEQ ID NO:53 (SEQ ID NO:79) | Fusion SEQ ID NO :65-SEQ ID NO : 51-SEQ ID NO:54 (SEQ ID NO:84) |
| 104E10 | Fusion SEQ ID NO :65-SEQ ID NO : 47-SEQ ID NO:53 (SEQ ID NO:80) | Fusion SEQ ID NO :65-SEQ ID NO : 52-SEQ ID NO:54 (SEQ ID NO:85) |

Dans un aspect particulier de l'invention, ledit anticorps pour son utilisation susmentionnée possède une faible teneur en fucose, inférieure ou égale à 65%.

Dans un aspect particulier de l'invention, ledit anticorps pour son utilisation susmentionnée possède une teneur en N-glycannes de type oligomannose supérieure ou égale à 30%.

Dans un aspect particulier de l'invention, ledit anticorps pour son utilisation susmentionnée possède une teneur en galactose supérieure ou égale à 50%.

Aux fins de la présente invention, l'anticorps, possède avantageusement une faible teneur en fucose, inférieure ou égale à 65%.

Par « teneur en fucose », on entend le pourcentage de formes fucosylées au sein des N-glycannes attachés au résidu Asn297 du fragment Fc de chaque chaine lourde de chaque anticorps.

Par « faible teneur en fucose », on entend une teneur en fucose inférieure ou égale à 65%. En effet, il est aujourd'hui connu que la teneur en fucose d'une composition d'anticorps joue un rôle crucial dans la capacité de cette composition à induire une forte réponse ADCC via le récepteur FcγRIII.

Avantageusement, la teneur en fucose est inférieure ou égale à 65%, de préférence inférieure ou égale à 60%, à 55% ou à 50%, voire inférieure ou égale à 45%, à 40%, à 35%, à 30%, à 25% ou à 20%. Cependant, il n'est pas nécessaire que la teneur en fucose soit nulle, et elle peut par exemple être supérieure ou égale à 5%, à 10%, à 15% ou à 20%. La teneur en fucose peut par exemple être comprise entre 5 et 65%, entre 5 et 60%, entre 5 et 55%, entre 5 et 50%, entre 5 et 45%, entre 5 et 40%, entre 5 et 35%, entre 5 et 30%, entre 5 et 25%, entre 5 et 20%, entre 10 et 65%, entre 10 et 60%, entre 10 et 55%, entre 10 et 50%, entre 10 et 45%, entre 10 et 40%, entre 10 et 35%, entre 10 et 30%, entre 10 et 25%, entre 10 et 20%, entre 15 et 65%, entre 15 et 60%, entre 15 et 55%, entre 15 et 50%, entre 15 et 45%, entre 15 et 40%, entre 15 et 35%, entre 15 et 30%, entre 15 et 25%, entre 15 et 20%, entre 20 et 65%, entre 20 et 60%, entre 20 et 55%, entre 20 et 50%, entre 20 et 45%, entre 20 et 40%, entre 20 et 35%, entre 20 et 30%, entre 20 et 25%.

L'anticorps, selon l'invention peut par ailleurs posséder différents types de glycosylation (N-glycannes de type oligomannose ou complexes biantennés, proportion variable de résidus N-acétylglucosamine (GlcNAc) intercalaires ou de résidus galactose dans le cas des N-glycannes de type complexes biantennés), à condition de posséder un faible teneur en fucose. Ainsi, des N-glycannes de type oligomannose peuvent être obtenus par culture en présence de différents inhibiteurs de glycosylation, tels que les inhibiteurs de $\alpha$1,2-mannosidase I (comme la *Deoxymannojirimycine* ou « DMM ») ou de l'a-glucosidase (comme la castanospermine ou « Cs ») ; ou bien par production de l'anticorps dans la lignée CHO Lec 1. La production dans le lait de chèvres transgéniques conduit également à l'obtention d'anticorps dont le N-glycanne majoritaire est de type oligomannose, avec comme formes minoritaires des formes complexes biantennées fucosylées avec un ou deux galactoses, sans GlcNAc intercalaire et sans sialylation (G1F ou G2F) (voir WO2007/048077). Des N-glycannes de type complexe biantenné peuvent être obtenus dans la plupart des cellules de mammifères, mais également dans des bactéries, levures ou plantes dont la machinerie de glycosylation a été modifiée.

Pour limiter la teneur en fucose, des lignées possédant naturellement une faible activité de l'enzyme FUT8 (1,6-fucosyltransférase) responsable de l'ajout du fucose sur le GlcNAc lié au fragment Fc ; telles que la lignée YB2/0, la lignée de cellule embryonnaire de canard EB66®, les lignées d'hépatome de rat rat H4-II-E (DSM ACC3129), H4-II-Es (DSM ACC3130), ou les lignées NM-H9D8-E6 (DSM ACC 2807), et NM H9D8-E6Q12 (DSM ACC 2856) peuvent être utilisées. Des lignées mutantes pour d'autres gènes et dont la sous-expression ou la surexpression conduit à une faible teneur en fucose peuvent également être utilisées, comme la lignée CHO Lec13, un mutant de la lignée CHO ayant une synthèse diminuée du GDP-fucose. Il est également possible de sélectionner une lignée d'intérêt et de diminuer ou abolir (notamment par utilisation d'ARN interférents ou par mutation ou délétion du gène exprimant la protéine d'intérêt) l'expression d'une protéine impliquée dans la voie de fucosylation des N-glycannes (notamment FUT8, voir Yamane-Ohnuki et al-2004 ; mais également GMD, un gène impliqué dans le transport du GDP-fucose, voir Kanda et al-2007). Une autre alternative consiste à sélectionner une lignée d'intérêt et à surexprimer une protéine interférant d'une façon ou d'une autre avec la fucosylation des N-glycannes, comme la protéine GnTIII (β(1,4)-N-acétylglucosaminetransférase III). En particulier, des anticorps possédant des N-glycannes faiblement fucosylés ont été notamment obtenus par :

- Production dans YB2/0 (voir EP1176195A1, WO01/77181, Shinkawa et al-2003) CHO Lecl3 (voir Shields et al-2002), EB66® (Olivier et al-2010), les lignées d'hépatome de rat rat H4-II-E (DSM ACC3129), H4-II-Es (DSM ACC3130) (voir WO2012/041768), et les lignées humaines NM-H9D8 (DSM ACC2806), NM-H9D8-E6 (DSM ACC 2807), et NM H9D8-E6Q12 (DSM ACC 2856) (voir WO2008/028686).
- Production dans une lignée CHO sauvage en présence de petits ARNs interférents dirigés contre FUT8 (Mori et al-2004, Suzuki et al-2007, Cardarelli et al-2009, Cardarelli et al-2010, Herbst et al-2010), ou GMD (gène codant pour le transporteur du GDP-fucose dans le Golgi, voir Imai-Nishiya et al-2007)
- Production dans une lignée CHO dont les deux allèles du gène FUT8 codant pour la 1,6-fucosyltransférase ont été délétés (Yamane-Ohnuki et al-2004), ou dont les deux allèles du gène GMD codant pour le transporteur du GDP-fucose dans le Golgi ont été délétés (Kanda et al-2007),
- Production dans une lignée CHO dans laquelle le gène codant pour l'enzyme GnTIII (β(1,4)-N-acétylglucosaminetransférase III) a été surexprimé par transgénèse (Umana et al-1999). Outre une faible fucosylation, les N-glycannes obtenus sont caractérisés par une forte teneur en GlcNAc intercalaire.
- Production dans des plantes (N. benthamiana) transgéniques, avec une forte diminution des teneurs en résidus β1,2-xylose et α1,3-fucose grâce à l'utilisation de petits ARNs interférents (Forthal et al-2010).

Les N-glycannes de type oligomannose ont une demi-vie réduite *in vivo* par rapport aux N-glycannes de type complexe biantenné. Par conséquent, avantageusement, les anticorps selon l'invention possèdent sur leurs sites de N-glycosylation du fragment Fc des structures glycanniques de type complexe biantenné, avec une faible teneur en fucose, comme défini ci-dessus.

En particulier, l'anticorps monoclonal selon l'invention peut posséder une teneur en formes G0+G1+G0F+G1F supérieure à 60% et une faible teneur en fucose, telle que définie ci-dessus. Elle peut également posséder teneur en formes G0+G1+G0F+G1F supérieure à 65% et une faible teneur en fucose, telle que définie ci-dessus. Elle peut également posséder teneur en formes G0+G1+G0F+G1F supérieure à 70% et une faible teneur en fucose, telle que définie ci-dessus. Elle peut également posséder teneur en formes G0+G1+G0F+G1F supérieure à 75% et une faible teneur en fucose, telle que définie ci-dessus. Elle peut également posséder teneur en formes G0+G1+G0F+G1F supérieure à 80% et une faible teneur en fucose, telle que définie ci-dessus. Elle peut également posséder teneur en formes G0+G1+G0F+G1F supérieure à 60%, à 65%, à 70%, à 75% ou à 80% et une teneur en formes G0F+G1F inférieure à 50%. Les formes G0, G1, G0F et G1F sont telles que définies ci-dessous :

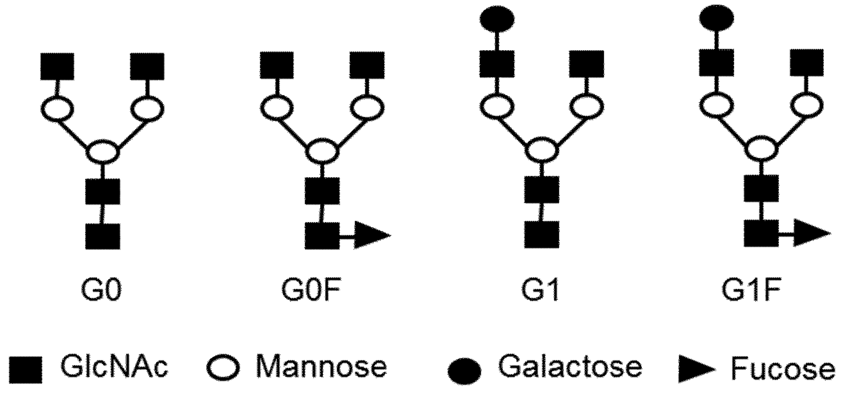

De telles compositions d'anticorps peuvent notamment être obtenues par production dans YB2/0, dans CHO Lec13, dans des lignées CHO sauvages cultivées en présence de petits ARNs interférents dirigés contre FUT8 ou GMD, dans des lignées CHO dont les deux allèles du gène FUT8 codant pour la 1,6-fucosyltransférase ou les deux allèles du gène GMD codant pour le transporteur du GDP-fucose dans le Golgi ont été délétés.
Cependant, dans un autre mode de réalisation, l'anticorps, selon l'invention possède une forte teneur en N-glycannes de type oligomannose.
Par « N-glycannes de type oligomannose », on entend des N-glycannes dont le cœur pentasaccharidique, constitué de deux résidus N-acétylglucosamine (GlcNAc) (l'un d'eux étant lié au résidu Asn297 du fragment Fc de l'anticorps) et trois résidus mannose, est complété par un à six mannoses additionnels fixés aux résidus mannose terminaux du cœur pentasaccharidique. Les N-glycannes de type oligomannose ne sont pas fucosylés.
Par « teneur en N-glycannes de type oligomannose », on entend le pourcentage de formes oligomannose au sein des N-glycannes attachés au résidu Asn297 du fragment Fc de chaque chaine lourde de chaque anticorps. Par « forte teneur en N-glycannes de type oligomannose », on entend une teneur en N-glycannes de type oligomannose supérieure ou égale à 30%, avantageusement supérieure ou égale à 35%, supérieure ou égale à 40%, supérieure ou égale à 45%, supérieure ou égale à 50%, supérieure ou égale à 55%, supérieure ou égale à 60%, supérieure ou égale à 65%, supérieure ou égale à 70%, supérieure ou égale à 75%, supérieure ou égale à 80%, supérieure ou égale à 85%, supérieure ou égale à 90%, voire supérieure ou égale à 95%.
En sus ou alternativement à une faible teneur en fucose, l'anticorps, selon l'invention possède une forte teneur en galactose.
Par « teneur en galactose » ou « taux de galactosylation » de l'anticorps, on entend un pourcentage calculé à partir d'un chromatogramme d'analyse des N-glycannes libérés de l'anticorps, selon la formule suivante :

$$\text{teneur en galactose} = \frac{\sum_{i=1}^{n} (\textit{nombre de Gal}) * (\textit{\% surface relative})}{\sum_{i=1}^{n} (\textit{nombre de A}) * (\textit{\% surface relative})} * 100$$

dans laquelle :

- « n » représente le nombre de pics N-glycannes analysés sur un chromatogramme, par exemple d'un spectre de chromatographie en phase liquide à haute performance en phase normale (NP HPLC),
- « nombre de Gal » représente le nombre de galactoses sur l'antenne du glycanne correspondant au pic,
- « nombre de A » représente le nombre d'antennes N-acétyl-glucosamine de la forme glycannique correspondant au pic, et
- « % surface relative » correspond au pourcentage de l'aire sous le pic correspondant.

Par « forte teneur en galactose », on entend une teneur en galactose supérieure ou égale à 30%, avantageusement supérieure ou égale à 50%, avantageusement supérieure ou égale à 55%, supérieure ou égale à 60%, supérieure ou égale à 65%, supérieure ou égale à 70%, supérieure ou égale à 75%, supérieure ou égale à 80%, supérieure ou égale à 85%, supérieure ou égale à 90%, supérieure ou égale à 95%, voire égale à 100%.
Il est également décrit un fragment d'anticorps dirigé contre la protéine CD303 tel que défini précédemment pour son utilisation.
Le terme « fragment » fait référence aux fragments Fab, F(ab)'2, Fd, scFv, dimère de ScFv, diabody, triabody ou tetrabody.
Le terme « Fab » fait référence à un fragment d'anticorps de masse moléculaire d'environ 50.000 dalton et possédant une activité de liaison à l'antigène. Il comprend environ la moitié du côté N-terminal de la chaîne lourde et la chaîne légère entière liées par un pont disulfure. Le Fab peut être obtenu notamment par le traitement des IgG par une protéase, la papaïne.
Le terme « F(ab')2 » fait référence à un fragment d'environ 100.000 dalton et possédant une activité de liaison à l'antigène. Il correspond à l'association par un pont disulfure de deux fragments Fab décrits ci-dessus. Il peut être obtenu par le traitement des IgG par une protéase, la pepsine.
Le terme « Fd » correspond à la partie de la chaîne lourde qui est inclus dans le fragment Fab. Le fragment Fd est ainsi formé des domaines VH et CH1.
Le terme « scFv » (single chain Fv) est un polypeptide VH:VL synthétisé en utilisant les gènes codant pour les

domaines VL et VH et une séquence codant pour un peptide destiné à lier ces domaines. Un scFv inclut les CDR maintenus dans une conformation appropriée, par exemple en utilisant des techniques de recombinaison génétique.

L'expression « dimères de ScFv » fait référence à deux molécules de scFv reliées entre elles par une liaison peptidique.

Les ScFv peuvent également servir de modules de base pour le développement de structures multimériques (dimérique : « diabody », trimérique : « triabody », tétramerique : « tetrabody »).

Le terme « diabody » fait référence à un dimère de scFv. Ce dimère de fragment a la propriété de maintenir la double valence que l'anticorps parent possède. Le diabody est bivalent, mono ou bispécifique selon qu'il fixe deux antigènes identiques ou différents.

Le terme « triabody » fait référence à l'association trivalente de scFv. Un triabody peut ainsi fixer trois antigènes identiques ou différents.

Le terme « tétrabody » fait référence à l'association tétravalente de scFv. Un tétrabody peut fixer quatre antigènes identiques ou différents.

Dans un aspect particulier de l'invention, ledit anticorps pour son utilisation susmentionnée est couplé à une molécule bioactive choisie parmi :

- les radio-isotopes, notamment choisis parmi At211, I131, I125, Y90, Re186, Re188, Sm153, Bi212, P32,
- les métaux non-radioactifs,
- les toxines, notamment choisies parmi la ricine, l'abrine, la toxine diphtérique,
- les acides nucléiques, notamment choisis parmi les ARNs anti-sens,
- les enzymes, notamment choisies parmi les RNases, la biotine, l'avidine ou la streptavidine,
- les agents cytotoxiques, notamment choisis parmi :
    - les antifolates, et plus particulièrement le méthotrexate, le pémétrexed, le raltitrexed ;
    - les anti-purines, et plus particulièrement la cladribine, la fludarabine, l'azathioprine, l'azathioprine, la mercaptopurine, le 5-fluorouracile, la capécitabine, la cytarabine, la gemcitabine,
    - les inhibiteurs de topoisomérases I et II,
    - les agents alkylants et agents apparentés, et plus particulièrement choisis parmi chlorméthine, cyclophosphamide, ifosfamide, carmustine, fotémustine, mitomycine C, cisplatine, carboplatine, oxaliplatine,
    - les agents intercalants,
    - les anthracyclines, et plus particulièrement choisis parmi daunorubicine, doxorubicine, chlorydrate, epirubicine, idarubicine, bléomycine,
    - les taxanes,
    - les inhibiteurs spécifiques de tyrosine-kinase, imatinib, erlotinib.

Il est également décrit un acide nucléique (encore appelé séquence nucléique ou nucléotidique) codant pour la chaine lourde et/ou la chaine légère d'un anticorps tel que décrit ci-dessus.

Cela inclut toutes les séquences nucléiques différentes, du fait de la dégénérescence du code génétique, codant pour une séquence d'acides aminés particulière. En particulier, la séquence d'un acide nucléique peut avoir été optimisée pour favoriser son expression dans une cellule hôte, un animal non-humain transgénique ou une plante transgénique d'intérêt. En effet, il existe en général plusieurs combinaisons de trois nucléotides codant le même acide-aminé (sauf pour la méthionine et le tryptophane), appelés codons synonymes. Cependant, certaines de ces combinaisons sont en général utilisées préférentiellement par une cellule ou un organisme donné (on parle alors de biais d'usage du code génétique). Cette préférence dépend notamment de l'organisme producteur ou dont est issue la cellule. Par conséquent, lorsqu'une protéine dérivée d'un ou plusieurs organismes est produite dans un organisme hétérologue ou dans une cellule d'un tel organisme hétérologue, il peut être utile de modifier la séquence nucléique codant pour la protéine de façon à utiliser principalement les codons préférés de l'organisme hétérologue. Des données sont disponibles dans la littérature concernant l'usage des codons préféré par différentes espèces et l'homme du métier sait comment optimiser l'expression d'une protéine donnée dans un organisme ou une cellule d'un organisme hétérologue.

Un acide nucléique tel que susmentionné comprend avantageusement au moins une des séquences SEQ ID NO : 86 à 95, telles que décrites dans le **Tableau 8** ci-dessous, qui codent pour les séquences d'acides aminés des régions VH et VL des anticorps selon l'invention et ont été optimisées pour une expression dans des cellules de l'espèce *Rattus norvegicus.*

**Tableau 8**. Séquence nucléotidiques préférées, optimisées pour une expression dans des cellules de l'espèce *Rattus norvegicus* codant pour les régions VH et VL des anticorps, fragments fonctionnels ou dérivés de ceux-ci selon l'invention.

| Anticorps | VH | VL |
|---|---|---|
| 122A2 | CAGGTCCAGCTGCAGCAGTCTGGGGCTGAGCTGGTGAGGCCTGGGGTCTCAGTGAAGATTTCCTGCAAGGGTTCTGGCTACACATTCACTGATTATTCTATGCACTGGGTGAAGCAGAGTCATGCAAAGAGTCTAGAGTGGATTGGAGTTATTAGTACTTACTATGGTGATTCTAACTATAACCAGAAGTTCAAGGGCAAGGCCACAATGACTGTAGACAAATCCTCCACCACAGCCTATATGGAACTTGCCAGACTGACATCTGAGGATTCTGCCATCTATTACTGTGCAAGAAATGGTAATTTCTATGTTATGGACTACTGGGGTCAAGGAACCTCAGTCACCGTCTCCTCA (SEQ ID NO :86) | GATATCCAGATGACACAGACTACATCCTCCCTGTCTGCCTCTCTGGGAGACAGAGTCACCATCAGTTGCAGGGCAAGTCAGGACATTAGCAATTATTTAAACTGGTATCAGCAGAAACCAGATGGAACTGTTAAACTCCTGATCTACTACACATCAAGATTACACTCAGGAGTCCCATCAAGGTTCAGTGGCAGTGGGTCTGGAACAGATTATTCTCTCACCATTAGCAACCTGGACCAAGAAGATATTGCCACTTACTTTTGCCAACAGGGTAATACGCTTCCTTGGACGTTCGGTGGAGGCACCAAGCTGGAAATCAAA (SEQ ID NO :91) |

(suite)

| Anticorps | VH | VL |
| --- | --- | --- |
| 102E9 | CAGATCCATTTGGTGCAGTCTGG ACCTGACCTGAAGAAGCCTGGAG AGACAGTCAAGATCTCCTGCAAG GCTTCTGGTTATACCTTCACAGA CTATTCAATGCACTGGGTGAAGC AGGCTCCAGGAAAGGGTTTAAA GTGGATGGGCTGGATAAACACTG AGACTGGTGAACCAACATATGCA GATGACTTCAAGGGACGGTTTGC CTTCTCTTTGGAAAGTTCTGCCA GCACTGCCTTTTTGCAGATCAAC AACCTCAAAAATGAGGACACGTC TACATATTTCTGTACTAGAAATG GTTACTACGTGGGTTACTATGCT ATGGACTACTGGGGTCAAGGAAC CTCAGTCACCGTCTCCTCA (SEQ ID NO :87) | CAAATTGTTCTCACCCAGTCT CCAGCAATCATGTCTGCATCT CCAGGGGAGAAGGTCACCAT AACCTGCAGTGCCAGCTCAA GTGTAATTTACATTCACTGGT TCCAGCAGAAGCCAGGCACT TCTCCCAAACTCTGGATTTAT AGCACATCCTACCTGGCTTCT GGAGTCCCTGCTCGCTTCAGT GGCAGTGGATCTGGGACCTC TTACTCTCTCACAATCAGCCG AATGGAGGCTGAAGATGCTG CCACTTATTACTGCCAGCAG AGGAGAAGTTACCCGTTCAC GTTCGGAGGGGGGACCAAGC TGGAAATAAAA (SEQ ID NO :92) |

(suite)

| Anticorps | VH | VL |
|---|---|---|
| 104C12 | CAGGTCCAGCTGCAGCAGTCTGGGGCTGAGCTGGTGGGGCCTGGGGTCTCAGTGAAGATTTCCTGCAAGGGTTCTGGCTACACATTCACTGATTATTCTATGCACTGGGTAAAGCAGAGTCATGCAAAGAGTCTAGAGTGGATTGGAGTTATTAGTCCTTACTATGGTGATACTAACTACAACCAGAAGTTCAAGGGCAAGGCCACAATGACTGTAGACAAATCCTCCAGCACAGCCTATATGGAACTTGCCAGTCTGACATCTGAGGATTCTGCCATCTATTTCTGTGCAAGAAATGATGATTACTACAGGTTTGCTTACTGGGGCCAAGGGACTCTGGTCACTGTCTCTGC (SEQ ID NO :88) | GATCTCCAGATGACACAGACTCCATCCTCCCTGTCTGCCTCTCTGGGAGACAGAGTCACCATCAGTTGCAGGGCAAGTCAGGACATTAACAATTATTTAAGCTGGTATCAGGAGAAACCAGATGGAACTTTTAAACTCCTGATCTACTACACATCAAGATTACACTCAGGAGTCCCATCAAGGTTCAGTGGCAGTGGGTCTGGAACAGATTATTCTCTCACCGTTCGCAACCTGGAACAGGAAGATATTGGCACTTACTTTTGCCAACAGGGTAAAACGCTTCCGTGGACGTTCGGTGGAGGCACCAAGCTGGAAATCAG (SEQ ID NO :93) |

(suite)

| Anticorps | VH | VL |
|---|---|---|
| 114D11 | CAGATCCAGTTGGTGCAGTCTGGACCTGAGCTGAAGAAGCCTGGAGAGACAGTCAAGATCTCCTGCAAGGCTTCTGGTTATACCTTCACAGACTCTTCAATGCACTGGGTGCAGCAGGCTCCAAACAAGGGTTTAAAGTGGATGGGCTGGATAAACACTGAGACTGGTGGGCCAACGTATGCAGATGATTTCAAGGGACGGTTTGCCTTCTCTTTGGAAACCTCTGCCAGAACTGCCTATTTGCAGATCAACAACCTCAAAAATGAGGACACGGCTACATATTTCTGTGCTAGAAATGGATACTACGTGGGGTACTATGCTCTGGACTACTGGGGTCAAGGAACCTCAGTCACCGTCTCCTCA (SEQ ID NO :89) | CAAATTGTTCTCACCCAGTCTCCAGCAATCATGTCTGCATCTCCAGGGGAGAAGGTCACCATAACCTGCAGTGCCAGCTCAAGTGTATTTTACATGCACTGGTTCCAGCAGAAGCCAGGCACTTCTCCCAAACTCTGGATTTATAGCACATCCAACCTGGCTTCTGGAGTCCCTGCTCGCTTCAGTGGCAGTGGATCTGGGACCTCTTACTCTCTCACAATCAGCCGAATGGAGGCTGAAGATGCTGCCACTTATTACTGCCAGCAAAGGAGAAGTTACCCGTACACGTTCGGAGGGGGGACCAAGCTGGAAATAAAA (SEQ ID NO :94) |

(suite)

| Anticorps | VH | VL |
|---|---|---|
| 104E10 | CAGATCCAGTTGGTGCAGTCTGG ACCTGAGCTGAAGAAGCCTGGA GAGACAGTCAAGATCTCCTGCAA GGCTTCTGGTTATACCTTCACAG ACTATTCAATGCACTGGGTGAAG CAGGCTCCAGGAAAGGGTTTAAA GTGGATGGGCTGGATAAACACTG AGACTGGTGAGCCAACATATGCA GATGACTTCAAGGGACGGTTTGC CTTCTCTTTGGAAACCTCTGCCAC CACTGCCTATTTGCAGATCAACA ACTTCAAAAATGAGGACACGGCT ACATATTTCTGTGCTAGAAATGG TTACTACGTGGGATATTATGCTA TGGACTACTGGGGTCAAGGAACC TCAGTCACCGTCTCCTCA (SEQ ID NO :90) | CAAATTGTTCTCACCCAGTCT CCAGCAATCATGTCTGCATCT CCAGGGGAGAAGGTCACCAT GACCTGCAGTGCCAGTTCAA GTGTAATTTACATGCACTGGT TCCAGCAGAAGCCAGGCACT TCTCCCAAACTCTGGATTTAT AGCACATCCAACCTGGCTTCT GGAGTCCCTGCTCGCTTCAGT GGCAGTGGATCTGGGACATC TTACTCTCTCACAATCAGCCG AATGGAGGCTGAAGATGCTG CCACTTATTACTGCCAGCAA AGGAGAAGTTACCCGTACAC GTTCGGAGGGGGGACCAAGC TGGAAATAAAA (SEQ ID NO :95) |

Les séquences nucléiques codant pour les régions constantes de chaine lourde ou légère préférées ont également été optimisées pour une expression dans des cellules de l'espèce *Rattus norvegicus* et sont de préférence celles décrites dans le Tableau 9 ci-dessous.

**Tableau 9**. Séquences nucléotidiques préférées codant pour les régions constantes de chaine lourde ou légère humaine préférées.

| | |
|---|---|
| Région constante de chaine lourde humaine préférée | GCCTCCACCAAGGGCCCATCCGTGTTCCCCCTGGCCCCATCCAGCAAGTCTACCTCCGGAGGCACAGCCGCCCTGGGCTGTCTGGTGAAGGACTACTTCCCCGAGCCAGTGACCGTGTCCTGGAACTCCGGAGCCCTGACATCCGGCGTGCACACCTTCCCCGCCGTGCTGCAGTCCAGCGGCCTGTACTCTCTGTCTTCCGTGGTGACCGTGCCATCCAGCTCCCTGGGAACCCAGACATACATCTGCAACGTGAACCACAAGCCTAGCAACACCAAGGTGGACAAGAAGGTGGAGCCTAAGAGCTGTGACAAGACACACACATGCCCTCCTTGTCCAGCCCCTGAGCTGCTGGGCGGCCCCTCCGTGTTCCTGTTCCCCCCCAAGCCTAAGGATACCCTGATGATCAGCAGAACCCCCGAGGTGACCTGCGTGGTGGTGGACGTGTCCCACGAGGATCCCGAGGTGAAGTTCAACTGGTACGTGGACGGCGTGGAGGTGCACAACGCTAAGACCAAGCCCAGAGAGGAGCAGTACAACAGCACATACAGAGTGGTGTCTGTGCTGACCGTGCTGCACCAGGACTGGCTGAACGGGAAGGAGTACAAGTGCAAGGTGTCCAACAAGGCCCTGCCTGCCCCTATCGAGAAGACCATCTCTAAGGCTAAGGGGCAGCCCCGGGAGCCACAGGTGTACACCCTGCCACCCAGCCGCGACGAGCTGACCAAGAACCAGGTGTCCCTGACATGCCTGGTGAAGGGATTCTACCCCAGCGACATCGCCGTGGAGTGGGAGAGCAACGGCCAGCCCGAGAACAACTACAAGACAACCCCTCCCGTGCTGGACAGCGATGGATCCTTCTTCCTGTACTCCAAGCTGACCGTGGACAAGAGCAGGTGGCAGCAGGGAAACGTGTTCTCTTGTTCCGTGATGCACGAGGCTCTGCACAACCACTACACCCAGAAGTCCCTGAGCCTGTCTCCAGGCAAG (SEQ ID NO :96) |

(suite)

| | |
|---|---|
| Région constante de chaine légère humaine préférée | CGAACTGTGGCTGCACCAAGTGTCTTCATCTTTCCTCCGAGTGATGAGCAGCTGAAGAGCGGGACAGCTTCTGTGGTGTGTCTGCTGAATAACTTCTACCCAAGAGAAGCAAAGGTCCAGTGGAAGGTGGACAACGCCCTGCAGTCTGGCAACTCACAGGAGTCTGTCACTGAGCAGGATTCCAAGGACAGCACTTACAGCCTGTCCAGCACCCTCACTCTGTCCAAAGCCGACTACGAAAAGCATAAGGTGTATGCTTGTGAGGTGACCCACCAGGGACTGAGCAGCCCTGTGACGAAGTCCTTCAACCGGGGCGAGTGC (SEQ ID NO :97) |

Ainsi, un acide nucléique codant pour la chaine lourde et/ou la chaine légère d'un anticorps tel que décrit dans la présente demande comprend de préférence au moins une des séquences nucléiques décrites dans le **Tableau 10** ci-dessous, constituées de la fusion de 5' en 3' :

- De l'une des séquences SEQ ID NO :86 à 90 codant pour la région VH des anticorps préférés selon l'invention et de la séquence SEQ DI NO :96 codant pour la région constante préférée de chaine lourde humaine ; ou
- De l'une des séquences SEQ ID NO :91 à 95 codant pour la région VL des anticorps préférés selon l'invention et de la séquence SEQ DI NO :97 codant pour la région constante préférée de chaine légère humaine.

**Tableau 10.** Séquences d'acides aminés des chaines lourdes et légères des anticorps selon l'invention.

| Anticorps | Chaine lourde | Chaine légère |
|---|---|---|
| 122A2 | Fusion SEQ ID NO : 86-SEQ ID NO9684 (SEQ ID NO:98) | Fusion SEQ ID NO : 91-SEQ ID NO:97 (SEQ ID NO:103) |
| 102E9 | Fusion SEQ ID NO : 87-SEQ ID NO:96 (SEQ ID NO:99) | Fusion SEQ ID NO : 92-SEQ ID NO:97 (SEQ ID NO:104) |
| 104C12 | Fusion SEQ ID NO : 88-SEQ ID NO:96 (SEQ ID NO:100) | Fusion SEQ ID NO : 93-SEQ ID NO:97 (SEQ ID NO:105) |
| 114D11 | Fusion SEQ ID NO : 89-SEQ ID NO:96 (SEQ ID NO:101) | Fusion SEQ ID NO : 94-SEQ ID NO:97 (SEQ ID NO:106) |
| 104E10 | Fusion SEQ ID NO : 90-SEQ ID NO:96 (SEQ ID NO:102) | Fusion SEQ ID NO : 95-SEQ ID NO:97 (SEQ ID NO:107) |

Un acide nucléique codant pour la chaine lourde et/ou la chaine légère de l'anticorps tel que décrit dans la présente demande, comprend avantageusement une séquence nucléique codant pour le peptide signal hétérologue MB7 (MRWSWIFLLLLSITSANA, SEQ ID NO:65), et notamment la séquence nucléique SEQ ID NO:108 (ATGAGGTGGTCCTGGATCTTCCTGCTGCTGCTGAGCATCACCAGCGCCAACGCC). En effet, ce peptide a été montré comme permettant d'améliorer l'expression et la sécrétion de protéines recombinantes dans des lignées cellulaires eucaryotes supérieures (voir WO2011/114063). Ainsi, un acide nucléique codant pour la chaine lourde des anticorps décrits dans la présente demande, comprend avantageusement une séquence nucléique choisie parmi les séquences SEQ ID NO : 109 à 113, constituées de la fusion de 5' en 3' entre la séquence nucléique codant pour le peptide signal MB7 (SEQ ID NO :108) et une des séquences nucléiques codant pour la région VH des anticorps selon l'invention (SEQ ID NO : 86 à 90). De plus ou de manière alternative, un acide nucléique codant pour la chaine légère des anticorps décrits dans la présente demande, comprend avantageusement une séquence nucléique choisie parmi les séquences

SEQ ID NO : 114 à 118, constituées de la fusion de 5' en 3' entre la séquence nucléique codant pour le peptide signal MB7 (SEQ ID NO :108) et une des séquences d'acides aminés de la région VL des anticorps selon l'invention (SEQ ID NO : 91 à 95). En ajoutant les régions constantes de chaine lourde et de chaine légère préférées, on obtient des séquences d'acides aminés complètes préférées des anticorps selon l'invention, tel que décrit dans le **Tableau 11** ci-dessous.

**Tableau 11**. Séquences nucléiques codant pour les chaines lourdes et légères des anticorps selon l'invention, avec le peptide signal MB7.

| Anticorps | Chaine lourde | Chaine légère |
|---|---|---|
| 122A2 | Fusion SEQ ID NO : 108-SEQ ID NO : 86-SEQ ID NO:96 (SEQ ID NO:119) | Fusion SEQ ID NO :108-SEQ ID NO : 91-SEQ ID NO:97 (SEQ ID NO:124) |
| 102E9 | Fusion SEQ ID NO : 108-SEQ ID NO : 87-SEQ ID NO:96 (SEQ ID NO:120) | Fusion SEQ ID NO :108-SEQ ID NO : 92-SEQ ID NO:97 (SEQ ID NO:125) |
| 104C12 | Fusion SEQ ID NO : 108-SEQ ID NO : 88-SEQ ID NO:96 (SEQ ID NO:121) | Fusion SEQ ID NO :108-SEQ ID NO : 93-SEQ ID NO:97 (SEQ ID NO:126) |
| 114D11 | Fusion SEQ ID NO : 108-SEQ ID NO : 89-SEQ ID NO:96 (SEQ ID NO:122) | Fusion SEQ ID NO :108-SEQ ID NO : 94-SEQ ID NO:97 (SEQ ID NO:127) |
| 104E10 | Fusion SEQ ID NO : 108-SEQ ID NO : 90-SEQ ID NO:96 (SEQ ID NO:123) | Fusion SEQ ID NO :108-SEQ ID NO : 95-SEQ ID NO:97 (SEQ ID NO:128) |

Dans un aspect particulier de l'invention, ledit anticorps pour son utilisation susmentionnée est utilisé en combinaison avec au moins un agent anti-cancéreux.

Dans un aspect particulier de l'invention, ledit agent anti-cancéreux est un agent anti-cancéreux chimique et/ou un agent anti-cancéreux utilisé en immunothérapie.

Dans un aspect particulier de l'invention, ledit agent anti-cancéreux chimique est choisi parmi les agents anti-métaboliques, les agents alkylants, les agents intercalants, ou les molécules ayant une action sur le fuseau mitotique.

Dans un aspect particulier de l'invention, lesdits agents métaboliques sont choisis parmi :

- les antifoliques, notamment méthotrexate, raltitrexed, et pemetrexed,
- les antipuriques, notamment mercaptopurine, thioguanine, pentostatine, cladribine et fludarabine,
- les antipyrimidiques, notamment 5-fluoro-uracile, tégafur uracile, capécitabine et cytarabine, et
- les anti-métaboliques, notamment l'hydroxycarbamide, l'hydroxyurée et la gemcitabine.

Dans un aspect particulier de l'invention, lesdits agents alkylants sont choisis parmi :

- les moutardes à l'azote, notamment chlorambucil, melphelan, chlorméthine, métachloroéthamine, estramustine, ifosfamide et cyclophosphamide,
- les nitroso-urées, notamment fotémustine, lomustine, carmustine, streptozocine,
- les organoplatines, notamment carboplatine, cisplatine et oxaliplatine,
- les ethylène imines, notamment thiotépa et altrétamine,
- les triazènes, notamment procarbazine, témozolomide et dacarbazine,
- les agents alkylants, notamment busulfan, mitomycine C et pipobroman.

Dans un aspect particulier de l'invention, lesdits agents intercalants sont choisis parmi :

- les dérivés de la camptothécine, notamment irinotécan et topotécan

- les antrhracyclines, notamment epirubicine, daunorubicine, doxorubicine, pirarubicine et idarubicine,
- les agents intercalants, notamment mitoxantrone, amsacrine, elliptinium, actinomycine d, dactinomycine, etoposide et bléomycine.

Dans un aspect particulier de l'invention, les molécules ayant une action sur le fuseau mitotique sont choisies parmi :

- les vinca-alcaloïdes ou poisons du fuseau, notamment vinorelbine, vindésine, vincristine et vinblastine,
- les taxoïdes ou stabilisants du fuseau, notamment paclitaxel et docétaxel,
- les inhibiteurs de tyrosine kinase, notamment dasatinib, erlotinib, imatinib, sorafénib et sunitinib.

Les molécules susmentionnées sont données à titre indicatif mais ne sauraient limiter la portée de l'invention. Les composés susmentionnés correspondent à la dénomination commune internationale (DCI) et l'homme de métier peut très facilement retrouver les marques commerciales correspondantes chez les différents fournisseurs.

Dans un aspect particulier de l'invention, ledit agent anti-cancéreux utilisé en immunothérapie est un anticorps spécifique de la tumeur ciblée, un anticorps anti-CD123, ou un agoniste des TLR.

Dans un autre aspect particulier, l'anticorps dirigé contre la protéine CD303 selon l'invention est utilisé en combinaison avec un anticorps anti-CD123 et/ou un agoniste des TLR.

Dans un autre aspect particulier, l'anticorps dirigé contre la protéine CD303 selon l'invention est utilisé en combinaison avec un anticorps anti-CD123 et un agoniste des TLR.

L'expression « anticorps spécifique de la tumeur ciblée » fait référence à un anticorps dirigé contre un marqueur spécifique de la tumeur à prévenir ou à traiter.

Un « anticorps anti-CD123 » est un anticorps dirigé contre CD123 (ou IL-3R$\alpha$). Un tel anticorps est disponible commercialement, par exemple chez Miltenyi Biotec, sous la référence AC145.

L'expression « agoniste des TLR » fait référence aux agonistes des Toll-Like Receptors, notamment les agonistes de TLR7 et TLR9. Un agoniste de TLR7 est par exemple Imiquimob et un agoniste de TLR9 est par exemple CpG oligodeoxynucleotides (CpG ODN).

Dans un aspect particulier de l'invention, ladite utilisation dudit anticorps et ladite utilisation dudit agent anti-cancéreux sont simultanées, séparées ou étalées dans le temps.

Dans un aspect particulier de l'invention, ladite utilisation dudit anticorps est simultanée à ladite utilisation dudit agent anti-cancéreux.

Dans un aspect particulier de l'invention, ladite utilisation dudit anticorps est successive à ladite utilisation dudit agent anti-cancéreux.

Dans un aspect particulier de l'invention, ladite utilisation dudit anticorps est préalable à ladite utilisation dudit agent anti-cancéreux.

Dans un aspect particulier de l'invention, l'utilisation dudit anticorps dirigé contre la protéine CD303 selon l'invention est simultanée à l'utilisation dudit anticorps anti-CD123 et dudit agoniste des TLR.

Dans un aspect particulier de l'invention, l'utilisation dudit anticorps dirigé contre la protéine CD303 selon l'invention est successive aux utilisations dudit anticorps anti-CD123 et dudit agoniste des TLR. Dans un tel cas, l'utilisation dudit anticorps anti-CD123 peut être successive, simultanée ou préalable à l'utilisation dudit agoniste des TLR.

Dans un aspect particulier de l'invention, l'utilisation dudit anticorps dirigé contre la protéine CD303 selon l'invention est préalable aux utilisations dudit anticorps anti-CD123 et dudit agoniste des TLR. Dans un tel cas, l'utilisation dudit anticorps anti-CD123 peut être successive, simultanée ou préalable à l'utilisation dudit agoniste des TLR.

Dans un aspect particulier de l'invention, ladite utilisation ladite utilisation dudit anticorps est couplée à une radiothérapie.

Dans un aspect particulier de l'invention, dudit anticorps et dudit agent anti-cancéreux est couplée à une radiothérapie.

Dans un aspect particulier de l'invention, ladite prévention ou ledit traitement est effectué chez un patient présentant une déplétion en cellules dendritiques plasmacytoïdes.

L'expression « patient présentant une déplétion en cellules dendritiques plasmacytoïdes » fait référence à un patient dans lequel le nombre de cellules dendritiques plasmacytoïdes a été diminué par un traitement préalable. Cette expression fait également référence à un patient dans lequel le nombre cellules dendritiques plasmacytoïdes va être diminué par un traitement ultérieur. Cela fait donc également référence à un patient ayant besoin ou nécessitant une déplétion en cellules dendritiques plasmacytoïdes, c'est-à-dire un patient dans lequel les anticorps dirigés contre la protéine CD303 vont agir indirectement sur la tumeur pour supprimer les cellules dendritiques plasmacytoïdes de l'environnement tumoral. Chez de tels patients, les anticorps dirigés contre la protéine CD303 vont ainsi faciliter, stimuler ou potentialiser l'action des anti-cancéreux, voire présenter une synergie avec lesdits anti-cancéreux.

Dans un autre aspect, il est également décrit une cellule hôte, un animal non-humain transgénique ou une

plante transgénique comprenant au moins un acide nucléique codant pour l'anticorps dirigé contre la protéine CD303 ou un vecteur d'expression de celui-ci.

Dans un aspect particulier de l'invention, l'anticorps dirigé contre la protéine CD303 est produit par transgénèse, notamment dans un animal non-humain transgénique ou une plante transgénique.

Dans un aspect particulier de l'invention, ledit agent anti-cancéreux est produit par transgénèse, notamment dans un animal non-humain transgénique ou une plante transgénique.

Dans un aspect particulier de l'invention, l'anticorps dirigé contre la protéine CD303 et ledit agent anti-cancéreux sont produits par transgénèse, notamment dans un animal non-humain transgénique ou une plante transgénique.

La cellule hôte peut être d'origine procaryote ou eucaryote, et peut notamment être choisie parmi les cellules bactériennes, les cellules d'insecte, de plantes, de levure ou de mammifères. L'anticorps, fragment fonctionnel ou dérivé selon l'invention peut alors être produit en cultivant la cellule hôte dans des conditions appropriées. Une cellule hôte selon l'invention peut notamment être obtenue par transformation d'une lignée cellulaire par le(s) vecteur(s) d'expression des chaines lourde et légère d'un anticorps, fragment fonctionnel ou dérivé de celui-ci selon l'invention, et séparation des différents clones cellulaires obtenus. La lignée cellulaire transformée est de préférence d'origine eucaryote, et peut notamment être choisie parmi les cellules d'insecte, de plantes, de levure ou de mammifères. Des lignées cellulaires appropriées pour la production d'anticorps incluent notamment les lignées choisies parmi : SP2/0 ; YB2/0 ; IR983F ; le myélome humain Namalwa ; PERC6 ; les lignées CHO, notamment CHO-K-1, CHO-Lec10, CHO-Lec1, CHO-Lec13, CHO Pro-5, CHO dhfr-, ou lignée CHO délétée pour les deux allèles codant pour le gène FUT8 et/ou le gène GMD ; Wil-2; Jurkat; Vero; Molt -4; COS-7; 293-HEK; BHK; K6H6; NSO; SP2/0-Ag 14, P3X63Ag8.653, lignée de cellule de canard embryonnaire EB66® (Valneva) ; les lignées d'hépatome de rat H4-II-E (DSM ACC3129), et H4-II-Es (DSM ACC3130) (voir WO2012/041768), NM-H9D8 (DSM ACC2806), NM-H9D8-E6 (DSM ACC 2807), et NM H9D8-E6Q12 (DSM ACC 2856) (voir WO2008/028686).

L' animal non-humain transgénique tel que susmentionné peut être obtenu par injection directe du ou des gène(s) d'intérêt (ici, les gènes réarrangés codant pour les chaines lourde et légère de l'anticorps) dans un œuf fertilisé (Gordon et al-1980). Un animal non-humain transgénique peut également être obtenu par introduction du ou des gène(s) d'intérêt (ici, les gènes réarrangés codant pour les chaines lourde et légère de l'anticorps) dans une cellule souche embryonnaire et préparation de l'animal par une méthode d'agrégation de chimère ou une méthode d'injection de chimère (voir Manipulating the Mouse Embryo, A Laboratory Manual, Second edition, Cold Spring Harbor Laboratory Press (1994); Gene Targeting, A Practical Approach, IRL Press at Oxford University Press (1993)). Un animal non-humain transgénique peut également être obtenu par une technique de clonage dans laquelle un noyau, dans lequel le ou les gène(s) d'intérêt (ici, les gènes réarrangés codant pour les chaines lourde et légère de l'anticorps) a été introduit, est transplanté dans un œuf énucléé (Ryan et al-1997; Cibelli et al-1998, WO00/26357). Un animal non humain transgénique produisant un anticorps d'intérêt peut être préparé par les méthodes ci-dessus. L'anticorps peut alors être accumulé dans l'animal transgénique et récolté, notamment à partir du lait ou des œufs de l'animal. Pour la production d'anticorps dans le lait d'animaux non humains transgéniques, des procédés de préparation sont notamment décrits dans WO90/04036, WO95/17085, WO01/26455, WO2004/050847, WO2005/033281, WO2007/048077. Des procédés de purification de protéines d'intérêt à partir du lait sont également connus (voir WO01/26455, WO2007/106078). Les animaux non humains transgéniques d'intérêt incluent notamment la souris, le lapin, le rat, la chèvre, les bovins (notamment la vache), et les volailles (notamment le poulet).

La plante transgénique telle que susmentionnée peut être choisie parmi toute plante permettant la production d'anticorps. De nombreux anticorps ont déjà été produits dans des plantes transgéniques et les technologies nécessaires à l'obtention d'une plante transgénique exprimant un anticorps d'intérêt et à la récupération de l'anticorps sont bien connues de l'homme du métier (voir Stoger et al-2002, Fisher et al-2003, Ma et al-2003, Schillberg et al-2005). Il est également possible d'influencer la glycosylation obtenue dans les plantes pour obtenir une glycosylation proche de celle des anticorps humains naturels (sans xylose), mais avec en outre une faible fucosylation, par exemple à l'aide de petits ARNs interférents (Forthal et al-2010).

Il est également décrit une composition comprenant au moins deux anticorps dirigés contre la protéine CD303, ou fragments desdits anticorps, et au moins un agent anti-cancéreux. Une telle composition consiste en un mélange d'anticorps dirigés contre la protéine CD303 et au moins un agent anti-cancéreux.

Dans un aspect particulier, ladite composition comprend au moins deux agents anti-cancéreux.

Il est également décrit une composition comprenant au moins un anticorps dirigé contre la protéine CD303, ou fragments desdits anticorps, et au moins deux agents anti-cancéreux.

Dans un aspect particulier, l'anticorps dans ladite composition est monoclonal ou polyclonal.

Dans un aspect particulier, l'anticorps dans ladite composition est choisi parmi un anticorps murin, un anticorps chimérique, un anticorps humanisé ou un anticorps humain.

Dans un aspect particulier, l'anticorps dans ladite composition est un anticorps chimérique et de préférence un anticorps chimérique choisi parmi un anticorps chimérique murin/humain ou un anticorps chimérique humain/macaque.

Dans un aspect particulier, l'anticorps dans ladite composition est un anticorps monoclonal dirigé contre l'ectodomaine de l'antigène CD303 humain (SEQ ID NO : 130), ou un fragment fonctionnel ou un dérivé de celui-ci, caractérisé en ce que :

c) il entre en compétition pour la liaison à l'antigène CD303 humain avec au moins un anticorps choisi parmi :

vi) Un anticorps dont la région variable de chaine lourde comprend la séquence SEQ ID NO : 43 et la région variable de chaine légère comprend la séquence SEQ ID NO : 48 ;
vii) Un anticorps dont la région variable de chaine lourde comprend la séquence SEQ ID NO : 44 et la région variable de chaine légère comprend la séquence SEQ ID NO : 49 ;
viii) Un anticorps dont la région variable de chaine lourde comprend la séquence SEQ ID NO : 45 et la région variable de chaine légère comprend la séquence SEQ ID NO : 50 ;
ix) Un anticorps dont la région variable de chaine lourde comprend la séquence SEQ ID NO : 46 et la région variable de chaine légère comprend la séquence SEQ ID NO : 51 ;
x) Un anticorps dont la région variable de chaine lourde comprend la séquence SEQ ID NO : 47 et la région variable de chaine légère comprend la séquence SEQ ID NO : 52 ; et

d) les régions constantes des chaînes légères et des chaînes lourdes sont des régions constantes provenant d'une espèce non-murine.

Dans un aspect particulier, l'anticorps dans ladite composition est un anticorps chimérique, humanisé ou humain, fragment fonctionnel ou dérivé de celui-ci, et est un anticorps dont la région variable de la chaine lourde est représentée par la séquence SEQ ID NO : 43 ou une séquence ayant au moins 80% d'identité avec ladite SEQ ID NO : 43, et dont la région variable de la chaine légère est représentée par la séquence SEQ ID NO : 48 ou une séquence ayant au moins 80% d'identité avec ladite SEQ ID NO : 48 (anticorps 122A2).

Dans un aspect particulier, l'anticorps dans ladite composition est un anticorps chimérique, humanisé ou humain, fragment fonctionnel ou dérivé de celui-ci, et est un anticorps dont la région variable de la chaine lourde est représentée par la séquence SEQ ID NO : 44 ou une séquence ayant au moins 80% d'identité avec ladite SEQ ID NO : 44, et dont la région variable de la chaine légère est représentée par la séquence SEQ ID NO : 49 ou une séquence ayant au moins 80% d'identité avec ladite SEQ ID NO : 49 (anticorps 102E9).

Dans un aspect particulier, l'anticorps dans ladite composition est un anticorps chimérique, humanisé ou humain, fragment fonctionnel ou dérivé de celui-ci, et est un anticorps dont la région variable de la chaine lourde est représentée par la séquence SEQ ID NO : 45 ou une séquence ayant au moins 80% d'identité avec ladite SEQ ID NO : 45, et dont la région variable de la chaine légère est représentée par la séquence SEQ ID NO : 50 ou une séquence ayant au moins 80% d'identité avec ladite SEQ ID NO : 50 (anticorps 104C12).

Dans un aspect particulier, l'anticorps dans ladite composition est un anticorps chimérique, humanisé ou humain, fragment fonctionnel ou dérivé de celui-ci, et est un anticorps dont la région variable de la chaine lourde est représentée par la séquence SEQ ID NO : 46 ou une séquence ayant au moins 80% d'identité avec ladite SEQ ID NO : 46, et dont la région variable de la chaine légère est représentée par la séquence SEQ ID NO : 51 ou une séquence ayant au moins 80% d'identité avec ladite SEQ ID NO : 51 (anticorps 114D11).

Dans un aspect particulier, l'anticorps dans ladite composition est un anticorps chimérique, humanisé ou humain, fragment fonctionnel ou dérivé de celui-ci, et est un anticorps dont la région variable de la chaine lourde est représentée par la séquence SEQ ID NO : 47 ou une séquence ayant au moins 80% d'identité avec ladite SEQ ID NO : 47, et dont la région variable de la chaine légère est représentée par la séquence SEQ ID NO : 52 ou une séquence ayant au moins 80% d'identité avec ladite SEQ ID NO : 52 (anticorps 104E10).

Dans un aspect particulier, l'anticorps dans ladite composition est un anticorps chimérique, humanisé ou humain, fragment fonctionnel ou dérivé de celui-ci, et est un anticorps dont la chaine lourde est représentée par la séquence SEQ ID NO : 55 ou une séquence ayant au moins 80% d'identité avec ladite SEQ ID NO : 55, et dont la chaine légère est représentée par la séquence SEQ ID NO : 60 ou une séquence ayant au moins 80% d'identité avec ladite SEQ ID NO : 60 (anticorps 122A2).

Dans un aspect particulier, l'anticorps dans ladite composition est un anticorps chimérique, humanisé ou humain, fragment fonctionnel ou dérivé de celui-ci, et est un anticorps dont la chaine lourde est représentée par la séquence SEQ ID NO : 56 ou une séquence ayant au moins 80% d'identité avec ladite SEQ ID NO : 56, et dont la chaine légère est représentée par la séquence SEQ ID NO : 61 ou une séquence ayant au moins 80% d'identité avec ladite SEQ ID NO : 61 (anticorps 102E9).

Dans un aspect particulier, l'anticorps dans ladite composition est un anticorps chimérique, humanisé ou humain, fragment fonctionnel ou dérivé de celui-ci, et est un anticorps dont la chaine lourde est représentée par la séquence SEQ ID NO : 57 ou une séquence ayant au moins 80% d'identité avec ladite SEQ ID NO : 57, et dont la chaine légère

est représentée par la séquence SEQ ID NO : 62 ou une séquence ayant au moins 80% d'identité avec ladite SEQ ID NO : 62 (anticorps 104C12).

Dans un aspect particulier, l'anticorps dans ladite composition est un anticorps chimérique, humanisé ou humain, fragment fonctionnel ou dérivé de celui-ci, et est un anticorps dont la chaine lourde est représentée par la séquence SEQ ID NO : 58 ou une séquence ayant au moins 80% d'identité avec ladite SEQ ID NO : 58, et dont la chaine légère est représentée par la séquence SEQ ID NO : 63 ou une séquence ayant au moins 80% d'identité avec ladite SEQ ID NO : 63 (anticorps 114D11).

Dans un aspect particulier, l'anticorps dans ladite composition est un anticorps chimérique, humanisé ou humain, fragment fonctionnel ou dérivé de celui-ci, et est un anticorps dont la chaine lourde est représentée par la séquence SEQ ID NO : 59 ou une séquence ayant au moins 80% d'identité avec ladite SEQ ID NO : 59, et dont la chaine légère est représentée par la séquence SEQ ID NO : 64 ou une séquence ayant au moins 80% d'identité avec ladite SEQ ID NO : 64 (anticorps 104E10).

Dans un aspect particulier, l'anticorps dans ladite composition possède une faible teneur en fucose, inférieure ou égale à 65%.

Dans un aspect particulier, l'anticorps dans ladite composition possède une teneur en N-glycannes de type oligomannose supérieure ou égale à 30%.

Dans un aspect particulier, l'anticorps dans ladite composition possède une teneur en galactose supérieure ou égale à 50%.

Dans un aspect particulier, le fragment dans ladite composition est choisi parmi Fab, F(ab)'2, Fd, scFv, dimère de ScFv, diabody, triabody ou tetrabody.

Dans un aspect particulier, ledit anticorps ou ledit fragment dans ladite composition est couplé à une molécule bioactive choisie parmi :

- les radio-isotopes, notamment choisis parmi At211, I131, I125, Y90, Re186, Re188, Sm153, Bi212, P32,
- les métaux non-radioactifs,
- les toxines, notamment choisies parmi la ricine, l'abrine, la toxine diphtérique,
- les acides nucléiques, notamment choisis parmi les ARNs anti-sens,
- les enzymes, notamment choisies parmi les RNases, la biotine, l'avidine ou la streptavidine,
- les agents cytotoxiques, notamment choisis parmi :
    - les antifolates, et plus particulièrement le méthotrexate, le pémétrexed, le raltitrexed ;
    - les anti-purines, et plus particulièrement la cladribine, la fludarabine, l'azathioprine, l'azathioprine, la mercaptopurine, le 5-fluorouracile, la capécitabine, la cytarabine, la gemcitabine,
    - les inhibiteurs de topoisomérases I et II,
    - les agents alkylants et agents apparentés, et plus particulièrement choisis parmi chlorméthine, cyclophosphamide, ifosfamide, carmustine, fotémustine, mitomycine C, cisplatine, carboplatine, oxaliplatine,
    - les agents intercalants,
    - les anthracyclines, et plus particulièrement daunorubicine, doxorubicine et chlorydrate, epirubicine, idarubicine, bléomycine,
    - les taxanes,
    - les inhibiteurs spécifiques de tyrosine-kinase, et plus particulièrement imatinib, erlotinib.

Dans un aspect particulier, ledit agent anti-cancéreux dans ladite composition est un agent anti-cancéreux chimique et/ou un agent anti-cancéreux utilisé en immunothérapie.

Dans un aspect particulier, ledit agent anti-cancéreux chimique dans ladite composition est choisi parmi les agents anti-métaboliques, les agents alkylants, les agents intercalants, ou les molécules ayant une action sur le fuseau mitotique.

Dans un aspect particulier, lesdits agents métaboliques dans ladite composition sont choisis parmi :

- les antifoliques, notamment méthotrexate, raltitrexed, et pemetrexed,
- les antipuriques, notamment mercaptopurine, thioguanine, pentostatine, cladribine et fludarabine,
- les antipyrimidiques, notamment 5-fluoro-uracile, tégafur uracile, capécitabine et cytarabine, et
- les anti-métaboliques, notamment l'hydroxycarbamide, l'hydroxyurée et la gemcitabine.

Dans un aspect particulier de l'invention, lesdits agents alkylants dans ladite composition sont choisis parmi :

- les moutardes à l'azote, notamment chlorambucil, melphelan, chlorméthine, métachloroéthamine, estramustine, ifosfamide et cyclophosphamide,

- les nitroso-urées, notamment fotémustine, lomustine, carmustine, streptozocine,
- les organoplatines, notamment carboplatine, cisplatine et oxaliplatine,
- les ethylène imines, notamment thiotépa et altrétamine,
- les triazènes, notamment procarbazine, témozolomide et dacarbazine,
- les agents alkylants, notamment busulfan, mitomycine C et pipobroman.

Dans un aspect particulier de l'invention, lesdits agents intercalants dans ladite composition sont choisis parmi :

- les dérivés de la camptothécine, notamment irinotécan et topotécan
- les antrhracyclines, notamment epirubicine, daunorubicine, doxorubicine, pirarubicine et idarubicine,
- les agents intercalants, notamment mitoxantrone, amsacrine, elliptinium, actinomycine d, dactinomycine, etoposide et bléomycine.

Dans un aspect particulier de l'invention, les molécules ayant une action sur le fuseau mitotique dans ladite composition sont choisies parmi :

- les vinca-alcaloïdes ou poisons du fuseau, notamment vinorelbine, vindésine, vincristine et vinblastine,
- les taxoïdes ou stabilisants du fuseau, notamment paclitaxel et docétaxel,
- les inhibiteurs de tyrosine kinase, notamment dasatinib, erlotinib, imatinib, sorafénib et sunitinib.

Dans un aspect particulier, ledit agent anti-cancéreux dans ladite composition qui est utilisé en immunothérapie est un anticorps spécifique de la tumeur ciblée, un anticorps anti-CD123, ou un agoniste des TLR.

Dans un aspect particulier, ledit agent anti-cancéreux dans ladite composition qui est utilisé en immunothérapie est un anticorps anti-CD123 et un agoniste des TLR.

Il est également décrit une composition comprenant au moins deux anticorps dirigés contre la protéine CD303, ou fragments desdits anticorps, et au moins un agent anti-cancéreux, pour son utilisation dans la prévention ou le traitement d'une tumeur impliquant une activation de cellules dendritiques plasmacytoïdes dans l'environnement de ladite tumeur.

Il est également décrit une composition comprenant au moins un anticorps dirigé contre la protéine CD303, ou fragment dudit anticorps, et au moins deux agents anti-cancéreux, pour son utilisation dans la prévention ou le traitement d'une tumeur impliquant une activation de cellules dendritiques plasmacytoïdes dans l'environnement de ladite tumeur.

Dans un aspect particulier, et comme indiqué précédemment, lesdites cellules dendritiques plasmacytoïdes ne sont pas à l'origine de la tumeur.

Dans un aspect particulier, et comme indiqué précédemment, les tumeurs impliquant une activation des cellules dendritiques plasmacytoïdes sont des tumeurs solides, notamment impliquant une infiltration de cellules dendritiques plasmacytoïdes dans leur environnement, ou des tumeurs hématopoïétiques.

Dans un aspect particulier, et comme indiqué précédemment, les tumeurs solides appartiennent au groupe des tumeurs de la tête et du cou, du mélanome, des cancers urogénitaux, du cancer du sein.

Dans un aspect particulier, et comme indiqué précédemment, les tumeurs hématopoïétiques appartiennent au groupe du myélome multiple, du lymphome, de la leucémie, notamment de la leucémie à cellules T.

Dans un aspect particulier, ledit anticorps, dans ladite composition pour son utilisation susmentionnée, est monoclonal ou polyclonal.

Dans un aspect particulier ledit anticorps, dans ladite composition pour son utilisation susmentionnée, est choisi parmi un anticorps murin, un anticorps chimérique, un anticorps humanisé ou un anticorps humain.

Dans un aspect particulier, ledit anticorps, dans ladite composition pour son utilisation susmentionnée, est un anticorps chimérique et de préférence un anticorps chimérique choisi parmi un anticorps chimérique murin/humain ou un anticorps chimérique humain/macaque.

Dans un aspect particulier, l'anticorps dans ladite composition pour son utilisation susmentionnée est un anticorps monoclonal dirigé contre l'ectodomaine de l'antigène CD303 humain (SEQ ID NO : 130), ou un fragment fonctionnel ou un dérivé de celui-ci, caractérisé en ce que :

e) il entre en compétition pour la liaison à l'antigène CD303 humain avec au moins un anticorps choisi parmi :

xi) Un anticorps dont la région variable de chaine lourde comprend la séquence SEQ ID NO : 43 et la région variable de chaine légère comprend la séquence SEQ ID NO : 48 ;
xii) Un anticorps dont la région variable de chaine lourde comprend la séquence SEQ ID NO : 44 et la région variable de chaine légère comprend la séquence SEQ ID NO : 49 ;

xiii) Un anticorps dont la région variable de chaine lourde comprend la séquence SEQ ID NO : 45 et la région variable de chaine légère comprend la séquence SEQ ID NO : 50 ;
xiv) Un anticorps dont la région variable de chaine lourde comprend la séquence SEQ ID NO : 46 et la région variable de chaine légère comprend la séquence SEQ ID NO : 51 ;
xv) Un anticorps dont la région variable de chaine lourde comprend la séquence SEQ ID NO : 47 et la région variable de chaine légère comprend la séquence SEQ ID NO : 52 ; et

f) les régions constantes des chaînes légères et des chaînes lourdes sont des régions constantes provenant d'une espèce non-murine.

Dans un aspect particulier, l'anticorps dans ladite composition pour son utilisation susmentionnée est un anticorps chimérique, humanisé ou humain, fragment fonctionnel ou dérivé de celui-ci, et est un anticorps dont la région variable de la chaine lourde est représentée par la séquence SEQ ID NO : 43 ou une séquence ayant au moins 80% d'identité avec ladite SEQ ID NO : 43, et dont la région variable de la chaine légère est représentée par la séquence SEQ ID NO : 48 ou une séquence ayant au moins 80% d'identité avec ladite SEQ ID NO : 48 (anticorps 122A2).
Dans un aspect particulier, l'anticorps dans ladite composition pour son utilisation susmentionnée est un anticorps chimérique, humanisé ou humain, fragment fonctionnel ou dérivé de celui-ci, et est un anticorps dont la région variable de la chaine lourde est représentée par la séquence SEQ ID NO : 44 ou une séquence ayant au moins 80% d'identité avec ladite SEQ ID NO : 44, et dont la région variable de la chaine légère est représentée par la séquence SEQ ID NO : 49 ou une séquence ayant au moins 80% d'identité avec ladite SEQ ID NO : 49 (anticorps 102E9).
Dans un aspect particulier, l'anticorps dans ladite composition pour son utilisation susmentionnée est un anticorps chimérique, humanisé ou humain, fragment fonctionnel ou dérivé de celui-ci, et est un anticorps dont la région variable de la chaine lourde est représentée par la séquence SEQ ID NO : 45 ou une séquence ayant au moins 80% d'identité avec ladite SEQ ID NO : 45, et dont la région variable de la chaine légère est représentée par la séquence SEQ ID NO : 50 ou une séquence ayant au moins 80% d'identité avec ladite SEQ ID NO : 50 (anticorps 104C12).
Dans un aspect particulier, l'anticorps dans ladite composition pour son utilisation susmentionnée est un anticorps chimérique, humanisé ou humain, fragment fonctionnel ou dérivé de celui-ci, et est un anticorps dont la région variable de la lourde est représentée par la séquence SEQ ID NO : 46 ou une séquence ayant au moins 80% d'identité avec ladite SEQ ID NO : 46, et dont la région variable de la chaine légère est représentée par la séquence SEQ ID NO : 51 ou une séquence ayant au moins 80% d'identité avec ladite SEQ ID NO : 51 (anticorps 114D11).
Dans un aspect particulier, l'anticorps dans ladite composition pour son utilisation susmentionnée est un anticorps chimérique, humanisé ou humain, fragment fonctionnel ou dérivé de celui-ci, et est un anticorps dont la région variable de la chaine lourde est représentée par la séquence SEQ ID NO : 47 ou une séquence ayant au moins 80% d'identité avec ladite SEQ ID NO : 47, et dont la région variable de la chaine légère est représentée par la séquence SEQ ID NO : 52 ou une séquence ayant au moins 80% d'identité avec ladite SEQ ID NO : 52 (anticorps 104E10).
Dans un aspect particulier, l'anticorps dans ladite composition pour son utilisation susmentionnée est un anticorps chimérique, humanisé ou humain, fragment fonctionnel ou dérivé de celui-ci, et est un anticorps dont la chaine lourde est représentée par la séquence SEQ ID NO : 55 ou une séquence ayant au moins 80% d'identité avec ladite SEQ ID NO : 55, et dont la chaine légère est représentée par la séquence SEQ ID NO : 60 ou une séquence ayant au moins 80% d'identité avec ladite SEQ ID NO : 60 (anticorps 122A2).
Dans un aspect particulier, l'anticorps dans ladite composition pour son utilisation susmentionnée est un anticorps chimérique, humanisé ou humain, fragment fonctionnel ou dérivé de celui-ci, et est un anticorps dont la chaine lourde est représentée par la séquence SEQ ID NO : 56 ou une séquence ayant au moins 80% d'identité avec ladite SEQ ID NO : 56, et dont la chaine légère est représentée par la séquence SEQ ID NO : 61 ou une séquence ayant au moins 80% d'identité avec ladite SEQ ID NO : 61 (anticorps 102E9).
Dans un aspect particulier, l'anticorps dans ladite composition pour son utilisation susmentionnée est un anticorps chimérique, humanisé ou humain, fragment fonctionnel ou dérivé de celui-ci, et est un anticorps dont la chaine lourde est représentée par la séquence SEQ ID NO : 57 ou une séquence ayant au moins 80% d'identité avec ladite SEQ ID NO : 57, et dont la chaine légère est représentée par la séquence SEQ ID NO : 62 ou une séquence ayant au moins 80% d'identité avec ladite SEQ ID NO : 62 (anticorps 104C12).
Dans un aspect particulier, l'anticorps dans ladite composition pour son utilisation susmentionnée est un anticorps chimérique, humanisé ou humain, fragment fonctionnel ou dérivé de celui-ci, et est un anticorps dont la lourde est représentée par la séquence SEQ ID NO : 58 ou une séquence ayant au moins 80% d'identité avec ladite SEQ ID NO : 58, et dont la chaine légère est représentée par la séquence SEQ ID NO :63 ou une séquence ayant au moins 80% d'identité avec ladite SEQ ID NO : 63 (anticorps 114D11).
Dans un aspect particulier, l'anticorps dans ladite composition pour son utilisation susmentionnée est un anticorps chimérique, humanisé ou humain, fragment fonctionnel ou dérivé de celui-ci, et est un anticorps dont la chaine lourde est représentée par la séquence SEQ ID NO : 59 ou une séquence ayant au moins 80% d'identité avec ladite

SEQ ID NO : 59, et dont la chaine légère est représentée par la séquence SEQ ID NO : 64 ou une séquence ayant au moins 80% d'identité avec ladite SEQ ID NO : 64 (anticorps 104E10).

Dans un aspect particulier, ledit anticorps, dans ladite composition pour son utilisation susmentionnée, possède une faible teneur en fucose, inférieure ou égale à 65%.

Dans un aspect particulier, ledit anticorps, dans ladite composition pour son utilisation susmentionnée, possède une teneur en N-glycannes de type oligomannose supérieure ou égale à 30%.

Dans un aspect particulier, ledit anticorps, dans ladite composition pour son utilisation susmentionnée, possède une teneur en galactose supérieure ou égale à 50%.

Dans un aspect particulier, ledit anticorps, dans ladite composition pour son utilisation susmentionnée, est un fragment d'anticorps.

Dans un aspect particulier, ledit fragment, dans ladite composition pour son utilisation susmentionnée, est choisi parmi Fab, F(ab)'2, Fd, scFv, dimère de ScFv, diabody, triabody ou tetrabody.

Dans un aspect particulier, ledit anticorps ou ledit fragment, dans ladite composition pour son utilisation susmentionnée, est couplé à une molécule bioactive choisie parmi :

- les radio-isotopes, notamment choisis parmi At211, I131, I125, Y90, Re186, Re188, Sm153, Bi212, P32,
- les métaux non-radioactifs,
- les toxines, notamment choisies parmi la ricine, l'abrine, la toxine diphtérique,
- les acides nucléiques, notamment choisis parmi les ARNs anti-sens,
- les enzymes, notamment choisies parmi les RNases, la biotine, l'avidine ou la streptavidine,
- les agents cytotoxiques, notamment choisis parmi :
    - les antifolates, et plus particulièrement le méthotrexate, le pémétrexed, le raltitrexed ;
    - les anti-purines, et plus particulièrement choisis parmi cladribine, fludarabine, azathioprine, azathioprine, mercaptopurine, 5-fluorouracile capécitabine, cytarabine, gemcitabine,
    - les inhibiteurs de topoisomérases I et II,
    - les agents alkylants et agents apparentés, et plus particulièrement choisis parmi chlorméthine, cyclophosphamide, ifosfamide, carmustine, fotémustine, mitomycine C, cisplatine, carboplatine, oxaliplatine,
    - les agents intercalants,
    - les anthracyclines, et plus particulièrement daunorubicine, doxorubicine et chlorydrate, epirubicine, idarubicine, bléomycine,
    - les taxanes,
    - les inhibiteurs spécifiques de tyrosine-kinase, et plus particulièrement imatinib, erlotinib.

Dans un aspect particulier, ledit agent anti-cancéreux, dans ladite composition pour son utilisation susmentionnée, est un agent anti-cancéreux chimique et/ou un agent anti-cancéreux utilisé en immunothérapie.

Dans un aspect particulier, ledit agent anti-cancéreux chimique, dans ladite composition pour son utilisation susmentionnée, est choisi parmi les agents anti-métaboliques, les agents alkylants, les agents intercalants, ou les molécules ayant une action sur le fuseau mitotique.

Dans un aspect particulier, lesdits agents métaboliques, dans ladite composition pour son utilisation susmentionnée, sont choisis parmi :

- les antifoliques, notamment méthotrexate, raltitrexed, et pemetrexed,
- les antipuriques, notamment mercaptopurine, thioguanine, pentostatine, cladribine et fludarabine,
- les antipyrimidiques, notamment 5-fluoro-uracile, tégafur uracile, capécitabine et cytarabine, et
- les anti-métaboliques, notamment l'hydroxycarbamide, l'hydroxyurée et la gemcitabine.

Dans un aspect particulier de l'invention, lesdits agents alkylants, dans ladite composition pour son utilisation susmentionnée, sont choisis parmi :

- les moutardes à l'azote, notamment chlorambucil, melphelan, chlorméthine, métachloroéthamine, estramustine, ifosfamide et cyclophosphamide,
- les nitroso-urées, notamment fotémustine, lomustine, carmustine, streptozocine,
- les organoplatines, notamment carboplatine, cisplatine et oxaliplatine,
- les ethylène imines, notamment thiotépa et altrétamine,
- les triazènes, notamment procarbazine, témozolomide et dacarbazine,
- les agents alkylants, notamment busulfan, mitomycine C et pipobroman.

Dans un aspect particulier de l'invention, lesdits agents intercalants, dans ladite composition pour son utilisation susmentionnée, sont choisis parmi :

- les dérivés de la camptothécine, notamment irinotécan et topotécan
- les antrhracyclines, notamment epirubicine, daunorubicine, doxorubicine, pirarubicine et idarubicine,
- les agents intercalants, notamment mitoxantrone, amsacrine, elliptinium, actinomycine d, dactinomycine, etoposide et bléomycine.

Dans un aspect particulier, lesdites molécules ayant une action sur le fuseau mitotique, dans ladite composition pour son utilisation susmentionnée, sont choisies parmi :

- les vinca-alcaloïdes ou poisons du fuseau, notamment vinorelbine, vindésine, vincristine et vinblastine,
- les taxoïdes ou stabilisants du fuseau, notamment paclitaxel et docétaxel,
- les inhibiteurs de tyrosine kinase, notamment dasatinib, erlotinib, imatinib, sorafénib et sunitinib.

Dans un aspect particulier, ledit agent anti-cancéreux utilisé en immunothérapie, dans ladite composition pour son utilisation susmentionnée, est un anticorps spécifique de la tumeur ciblée, un anticorps anti-CD123, ou un agoniste des TLR.

Dans un aspect particulier, ledit agent anti-cancéreux utilisé en immunothérapie, dans ladite composition pour son utilisation susmentionnée, est un anticorps anti-CD123 et un agoniste des TLR.

Dans un aspect particulier, l'utilisation dudit anticorps ou dudit fragment dans ladite composition et ladite utilisation dudit agent anti-cancéreux dans ladite composition sont simultanées, séparées ou étalées dans le temps.

Dans un aspect particulier, l'utilisation dudit anticorps ou dudit fragment dans ladite composition et l'utilisation dudit agent anti-cancéreux dans ladite composition sont simultanées.

Dans un aspect particulier, l'utilisation dudit anticorps ou dudit fragment dans ladite composition et l'utilisation dudit agent anti-cancéreux dans ladite composition sont successives.

Dans un aspect particulier, l'utilisation dudit anticorps ou dudit fragment dans ladite composition est préalable à l'utilisation dudit agent anti-cancéreux dans ladite composition.

Dans un aspect particulier, l'utilisation dudit anticorps dirigé contre la protéine CD303 dans ladite composition est simultanée à l'utilisation dudit anticorps anti-CD123 et dudit agoniste des TLR.

Dans un aspect particulier, l'utilisation dudit anticorps dirigé contre la protéine CD303 dans ladite composition est successive aux utilisations dudit anticorps anti-CD123 et dudit agoniste des TLR. Dans un tel cas, l'utilisation dudit anticorps anti-CD123 peut être successive, simultanée ou préalable à l'utilisation dudit agoniste des TLR.

Dans un aspect particulier, l'utilisation dudit anticorps dirigé contre la protéine CD303 dans ladite composition est préalable aux utilisations dudit anticorps anti-CD123 et dudit agoniste des TLR. Dans un tel cas, l'utilisation dudit anticorps anti-CD123 peut être successive, simultanée ou préalable à l'utilisation dudit agoniste des TLR.

Dans un aspect particulier, l'utilisation de ladite composition susmentionnée est couplée à une radiothérapie.

Dans un aspect particulier, l'utilisation de ladite composition susmentionnée pour la prévention ou le traitement d'une tumeur impliquant une activation de cellules dendritiques plasmacytoïdes dans l'environnement de ladite tumeur est effectuée chez un patient présentant une déplétion en cellules dendritiques plasmacytoïdes ou chez un patient ayant besoin ou nécessitant une déplétion en cellules dendritiques plasmacytoïdes.

Il est également décrit un agent anti-cancéreux pour son utilisation dans le cadre de la prévention ou du traitement d'une tumeur chez des patients présentant une déplétion en cellules dendritiques plasmacytoïdes, ou chez un patient ayant besoin ou nécessitant une déplétion en cellules dendritiques plasmacytoïdes.

Dans un aspect particulier, ladite tumeur des patients présentant une déplétion en cellules dendritiques plasmacytoïdes est une tumeur impliquant une activation de cellules dendritiques plasmacytoïdes dans son environnement, dans laquelle lesdites cellules dendritiques plasmacytoïdes ne sont pas à l'origine de la tumeur.

Dans un aspect particulier, et comme indiqué précédemment, les tumeurs impliquant une activation des cellules dendritiques plasmacytoïdes sont des tumeurs solides, notamment impliquant une infiltration de cellules dendritiques plasmacytoïdes dans leur environnement, ou des tumeurs hématopoïétiques.

Dans un aspect particulier, et comme indiqué précédemment, les tumeurs solides appartiennent au groupe des tumeurs de la tête et du cou, du mélanome, des cancers urogénitaux, du cancer du sein.

Dans un aspect particulier, et comme indiqué précédemment, les tumeurs hématopoïétiques appartiennent au groupe du myélome multiple, du lymphome, de la leucémie, notamment la leucémie à cellules T.

Dans un aspect particulier, ledit agent anti-cancéreux, pour son utilisation susmentionnée, est un agent anti-cancéreux chimique et/ou un agent anti-cancéreux utilisé en immunothérapie.

Dans un aspect particulier, ledit agent anti-cancéreux chimique, pour son utilisation susmentionnée, est choisi parmi les agents anti-métaboliques, les agents alkylants, les agents intercalants, ou les molécules ayant une action sur

le fuseau mitotique.

Dans un aspect particulier, ledit agent anti-cancéreux qui est un agent métabolique, pour son utilisation susmentionnée, est choisi parmi :

- les antifoliques, notamment méthotrexate, raltitrexed, et pemetrexed,
- les antipuriques, notamment mercaptopurine, thioguanine, pentostatine, cladribine et fludarabine,
- les antipyrimidiques, notamment 5-fluoro-uracile, tégafur uracile, capécitabine et cytarabine, et
- les anti-métaboliques, notamment l'hydroxycarbamide, l'hydroxyurée et la gemcitabine.

Dans un aspect particulier, ledit agent anti-cancéreux qui est un agent alkylant, pour son utilisation susmentionnée, est choisi parmi :

- les moutardes à l'azote, notamment chlorambucil, melphelan, chlorméthine, métachloroéthamine, estramustine, ifosfamide et cyclophosphamide,
- les nitroso-urées, notamment fotémustine, lomustine, carmustine, streptozocine,
- les organoplatines, notamment carboplatine, cisplatine et oxaliplatine,
- les ethylène imines, notamment thiotépa et altrétamine,
- les triazènes, notamment procarbazine, témozolomide et dacarbazine,
- les agents alkylants, notamment busulfan, mitomycine C et pipobroman.

Dans un aspect particulier, ledit agent anti-cancéreux qui est un agent intercalant, pour son utilisation susmentionnée, est choisi parmi :

- les dérivés de la camptothécine, notamment irinotécan et topotécan
- les antrhracyclines, notamment epirubicine, daunorubicine, doxorubicine, pirarubicine et idarubicine,
- les agents intercalants, notamment mitoxantrone, amsacrine, elliptinium, actinomycine d, dactinomycine, etoposide et bléomycine.

Dans un aspect particulier, ledit agent anti-cancéreux qui est molécule ayant une action sur le fuseau mitotique, pour son utilisation susmentionnée, est choisie parmi :

- les vinca-alcaloïdes ou poisons du fuseau, notamment vinorelbine, vindésine, vincristine et vinblastine,
- les taxoïdes ou stabilisants du fuseau, notamment paclitaxel et docétaxel,
- les inhibiteurs de tyrosine kinase, notamment dasatinib, erlotinib, imatinib, sorafénib et sunitinib.

Dans un aspect particulier, ledit agent anti-cancéreux qui est utilisé en immunothérapie, pour son utilisation susmentionnée, est choisi parmi un anticorps spécifique de la tumeur ciblée, un anticorps anti-CD123, ou un agoniste des TLR.

Dans un aspect particulier, ledit agent anti-cancéreux qui est utilisé en immunothérapie, pour son utilisation susmentionnée, est un anticorps anti-CD123 et un agoniste des TLR.

Dans un aspect particulier, ledit agent anti-cancéreux pour son utilisation susmentionnée est utilisé en combinaison avec un anticorps, monoclonal ou polyclonal, dirigé contre la protéine CD303, ou fragment de celui-ci, notamment un anticorps possédant une faible teneur en fucose, inférieure ou égale à 65%, et/ou une teneur en N-glycannes de type oligomannose supérieure ou égale à 30% et/ou une teneur en galactose supérieure ou égale à 50%.

Dans un aspect particulier, l'anticorps dirigé contre la protéine CD303, utilisé en combinaison avec ledit agent anti-cancéreux pour son utilisation susmentionnée, est choisi parmi un anticorps murin, un anticorps chimérique, un anticorps humanisé ou un anticorps humain, de préférence un anticorps chimérique choisi parmi un anticorps chimérique murin/humain ou un anticorps chimérique humain/macaque.

Dans un aspect particulier, l'anticorps dirigé contre la protéine CD303, utilisé en combinaison avec ledit agent anti-cancéreux pour son utilisation susmentionnée est un anticorps monoclonal dirigé contre l'ectodomaine de l'antigène CD303 humain (SEQ ID NO : 130), ou un fragment fonctionnel ou un dérivé de celui-ci, caractérisé en ce que :

g) il entre en compétition pour la liaison à l'antigène CD303 humain avec au moins un anticorps choisi parmi :

xvi) Un anticorps dont la région variable de chaine lourde comprend la séquence SEQ ID NO : 43 et la région variable de chaine légère comprend la séquence SEQ ID NO : 48 ;

xvii) Un anticorps dont la région variable de chaine lourde comprend la séquence SEQ ID NO : 44 et la région variable de chaine légère comprend la séquence SEQ ID NO : 49 ;

xviii) Un anticorps dont la région variable de chaine lourde comprend la séquence SEQ ID NO : 45 et la région variable de chaine légère comprend la séquence SEQ ID NO : 50 ;
xix) Un anticorps dont la région variable de chaine lourde comprend la séquence SEQ ID NO : 46 et la région variable de chaine légère comprend la séquence SEQ ID NO : 51 ;
xx) Un anticorps dont la région variable de chaine lourde comprend la séquence SEQ ID NO : 47 et la région variable de chaine légère comprend la séquence SEQ ID NO : 52 ; et

h) les régions constantes des chaînes légères et des chaînes lourdes sont des régions constantes provenant d'une espèce non-murine.

Dans un aspect particulier, l'anticorps dirigé contre la protéine CD303, utilisé en combinaison avec ledit agent anti-cancéreux pour son utilisation susmentionnée est un anticorps chimérique, humanisé ou humain, fragment fonctionnel ou dérivé de celui-ci, et est un anticorps dont la région variable de la chaine lourde est représentée par la séquence SEQ ID NO : 43 ou une séquence ayant au moins 80% d'identité avec ladite SEQ ID NO : 43, et dont la région variable de la chaine légère est représentée par la séquence SEQ ID NO : 48 ou une séquence ayant au moins 80% d'identité avec ladite SEQ ID NO : 48 (anticorps 122A2).

Dans un aspect particulier, l'anticorps dirigé contre la protéine CD303, utilisé en combinaison avec ledit agent anti-cancéreux pour son utilisation susmentionnée est un anticorps chimérique, humanisé ou humain, fragment fonctionnel ou dérivé de celui-ci, et est un anticorps dont la région variable de la chaine lourde est représentée par la séquence SEQ ID NO : 44 ou une séquence ayant au moins 80% d'identité avec ladite SEQ ID NO : 44, et dont la région variable de la chaine légère est représentée par la séquence SEQ ID NO : 49 ou une séquence ayant au moins 80% d'identité avec ladite SEQ ID NO : 49 (anticorps 102E9).

Dans un aspect particulier, l'anticorps dirigé contre la protéine CD303, utilisé en combinaison avec ledit agent anti-cancéreux pour son utilisation susmentionnée est un anticorps chimérique, humanisé ou humain, fragment fonctionnel ou dérivé de celui-ci, et est un anticorps dont la région variable de la chaine lourde est représentée par la séquence SEQ ID NO : 45 ou une séquence ayant au moins 80% d'identité avec ladite SEQ ID NO : 45, et dont la région variable de la chaine légère est représentée par la séquence SEQ ID NO : 50 ou une séquence ayant au moins 80% d'identité avec ladite SEQ ID NO : 50 (anticorps 104C12).

Dans un aspect particulier, l'anticorps dirigé contre la protéine CD303, utilisé en combinaison avec ledit agent anti-cancéreux pour son utilisation susmentionnée est un anticorps chimérique, humanisé ou humain, fragment fonctionnel ou dérivé de celui-ci, et est un anticorps dont la région variable de la chaine lourde est représentée par la séquence SEQ ID NO : 46 ou une séquence ayant au moins 80% d'identité avec ladite SEQ ID NO : 46, et dont la région variable de la chaine légère est représentée par la séquence SEQ ID NO : 51 ou une séquence ayant au moins 80% d'identité avec ladite SEQ ID NO : 51 (anticorps 114D11).

Dans un aspect particulier, l'anticorps dirigé contre la protéine CD303, utilisé en combinaison avec ledit agent anti-cancéreux pour son utilisation susmentionnée est un anticorps chimérique, humanisé ou humain, fragment fonctionnel ou dérivé de celui-ci, et est un anticorps dont la région variable de la chaine lourde est représentée par la séquence SEQ ID NO : 47 ou une séquence ayant au moins 80% d'identité avec ladite SEQ ID NO : 47, et dont la région variable de la chaine légère est représentée par la séquence SEQ ID NO : 52 ou une séquence ayant au moins 80% d'identité avec ladite SEQ ID NO : 52 (anticorps 104E10).

Dans un aspect particulier, l'anticorps dirigé contre la protéine CD303, utilisé en combinaison avec ledit agent anti-cancéreux pour son utilisation susmentionnée est un anticorps chimérique, humanisé ou humain, fragment fonctionnel ou dérivé de celui-ci, et est un anticorps dont la chaine lourde est représentée par la séquence SEQ ID NO : 55 ou une séquence ayant au moins 80% d'identité avec ladite SEQ ID NO : 55, et dont la chaine légère est représentée par la séquence SEQ ID NO : 60 ou une séquence ayant au moins 80% d'identité avec ladite SEQ ID NO : 60 (anticorps 122A2).

Dans un aspect particulier, l'anticorps dirigé contre la protéine CD303, utilisé en combinaison avec ledit agent anti-cancéreux pour son utilisation susmentionnée est un anticorps chimérique, humanisé ou humain, fragment fonctionnel ou dérivé de celui-ci, et est un anticorps dont la chaine lourde est représentée par la séquence SEQ ID NO : 56 ou une séquence ayant au moins 80% d'identité avec ladite SEQ ID NO : 56, et dont la chaine légère est représentée par la séquence SEQ ID NO : 61 ou une séquence ayant au moins 80% d'identité avec ladite SEQ ID NO :61 (anticorps 102E9).

Dans un aspect particulier, l'anticorps dirigé contre la protéine CD303, utilisé en combinaison avec ledit agent anti-cancéreux pour son utilisation susmentionnée est un anticorps chimérique, humanisé ou humain, fragment fonctionnel ou dérivé de celui-ci, et est un anticorps la chaine lourde est représentée par la séquence SEQ ID NO :57 ou une séquence ayant au moins 80% d'identité avec ladite SEQ ID NO : 57, et dont la chaine légère est représentée par la séquence SEQ ID NO : 62 ou une séquence ayant au moins 80% d'identité avec ladite SEQ ID NO : 62 (anticorps 104C12).

Dans un aspect particulier, l'anticorps dirigé contre la protéine CD303, utilisé en combinaison avec ledit agent anti-cancéreux pour son utilisation susmentionnée est un anticorps chimérique, humanisé ou humain, fragment fonctionnel ou dérivé de celui-ci, et est un anticorps dont la chaine lourde est représentée par la séquence SEQ ID NO : 58 ou une séquence ayant au moins 80% d'identité avec ladite SEQ ID NO : 58, et dont la chaine légère est représentée par la séquence SEQ ID NO : 63 ou une séquence ayant au moins 80% d'identité avec ladite SEQ ID NO : 63 (anticorps 114D11).

Dans un aspect particulier, l'anticorps dirigé contre la protéine CD303, utilisé en combinaison avec ledit agent anti-cancéreux pour son utilisation susmentionnée est un anticorps chimérique, humanisé ou humain, fragment fonctionnel ou dérivé de celui-ci, et est un anticorps dont la chaine lourde est représentée par la séquence SEQ ID NO : 59 ou une séquence ayant au moins 80% d'identité avec ladite SEQ ID NO : 59 et dont la chaine légère est représentée par la séquence SEQ ID NO : 64 ou une séquence ayant au moins 80% d'identité avec ladite SEQ ID NO : 64 (anticorps 104E10).

Dans un aspect particulier, l'anticorps dirigé contre la protéine CD303, ou fragment de celui-ci, utilisé en combinaison avec ledit agent anti-cancéreux pour son utilisation susmentionnée, est couplé à une molécule bioactive choisie parmi :

- les radio-isotopes, notamment choisis parmi At211, I131, I125, Y90, Re186, Re188, Sm153, Bi212, P32,
- les métaux non-radioactifs,
- les toxines, notamment choisies parmi la ricine, l'abrine, la toxine diphtérique,
- les acides nucléiques, notamment choisis parmi les ARNs anti-sens,
- les enzymes, notamment choisies parmi les RNases, la biotine, l'avidine ou la streptavidine,
- les agents cytotoxiques, notamment choisis parmi :
  - les antifolates, et plus particulièrement le méthotrexate, le pémétrexed, le raltitrexed ;
  - les anti-purines, et plus particulièrement la cladribine, la fludarabine, l'azathioprine, l'azathioprine, la mercaptopurine, le 5-fluorouracile, la capécitabine, la cytarabine, la gemcitabine,
  - les inhibiteurs de topoisomérases I et II,
  - les agents alkylants et agents apparentés, et plus particulièrement chlorméthine, cyclophosphamide, ifosfamide, carmustine, fotémustine, mitomycine C, cisplatine, carboplatine, oxaliplatine,
  - les agents intercalants,
  - les anthracyclines, et plus particulièrement choisis parmis daunorubicine, doxorubicine et chlorydrate, epirubicine, idarubicine, bléomycine,
  - les taxanes,
  - les inhibiteurs spécifiques de tyrosine-kinase, et plus particulièrement imatinib, erlotinib.

Dans un aspect particulier, l'utilisation susmentionnée dudit agent anti-cancéreux et dudit anticorps ou dudit fragment sont simultanées, séparées ou étalées dans le temps.

Dans un aspect particulier, l'utilisation susmentionnée dudit anticorps ou dudit fragment est simultanée à l'utilisation dudit anticorps anti-CD123 et dudit agoniste des TLR.

Dans un aspect particulier, l'utilisation susmentionnée dudit anticorps ou dudit fragment est successive aux utilisations dudit anticorps anti-CD123 et dudit agoniste des TLR. Dans un tel cas, l'utilisation dudit anticorps anti-CD123 peut être successive, simultanée ou préalable à l'utilisation dudit agoniste des TLR.

Dans un aspect particulier, l'utilisation susmentionnée dudit anticorps ou dudit fragment est préalable aux utilisations dudit anticorps anti-CD123 et dudit agoniste des TLR. Dans un tel cas, l'utilisation dudit anticorps anti-CD123 peut être successive, simultanée ou préalable à l'utilisation dudit agoniste des TLR.

Dans un aspect particulier, ledit agent anti-cancéreux pour son utilisation susmentionnée est utilisé en combinaison avec une radiothérapie.

L'invention sera mieux illustrée par les exemples et figures suivantes.

## LEGENDES DES FIGURES

**Figure 1**. Cartes des vecteurs d'expression des anticorps chimériques 122A2 (A), 102E9 (B), 104C12 (C), 114D11 (D), et 104E10 (E).

**Figure 2. Liaison à l'antigène des anticorps selon l'invention**. (A) Intensité de fluorescence moyenne (MFI) de cellules Jurkat chaine Fc Gamma-CD303 marquées par les anticorps selon l'invention, à différentes concentrations d'anticorps (représenté en unité logarithmique). (B) Intensité de fluorescence moyenne (MFI) de cellules CAL-1

(lignée cellulaire de patients BPDCN) marquées par les anticorps selon l'invention, à différentes concentrations d'anticorps (représenté en unité logarithmique).

**Figure 3. Liaison au récepteur FcγRIIIa (CD16a)**. La fixation au récepteur CD16 des anticorps selon l'invention a été étudiée dans une expérience de compétition utilisant un anticorps anti-CD16 3G8 murin couplé à la phycoérythrine. La liaison de l'anti-CD16 3G8 au CD16 (valeurs de fluorescence MFI) est mesurée en fonction de la concentration croissante ajoutée des anticorps selon l'invention (μg/mL).

**Figure 4. Activités ADCC induites par les anticorps de l'invention vis-à-vis des cellules Jurkat chaine Fc Gamma-CD303.** Le pourcentage de lyse par ADCC (% lyse, tel que défini à l'Exemple 2) de cellules cibles Jurkat chaine Fc Gamma-CD303 induit par les anticorps chimériques selon l'invention est représenté en fonction de la concentration en anticorps (ng/mL, échelle logarithmique).

**Figure 5. Déplétion de cellules NF-3C8 induite pas l'anticorps 122A2 dans un test en sang total chez cinq donneurs.** L'abscisse représente les 6 tests réalisés : l'essai réalisé avec un anticorps témoin et les cinq essais réalisés avec l'anticorps 122A2, avec le sang total de cinq donneurs sains dans lequel ont été ajoutées des cellules NF-3C8 afin de mimer le sang d'un patient atteint de BPDCN (Blastic Plasmacytoid Dendritic Cell Neoplasm). L'ordonnée représente le pourcentage de déplétion des cellules NF-3C8.

## EXEMPLES

### Exemple 1 : Obtention et structure de 5 anticorps chimériques

5 anticorps monoclonaux chimériques, avec des régions variables murines et des régions constantes humaines de type IgG1 ont été générés et leurs structures caractérisées.

*Matériels et méthodes*

Séquençage des chaines lourdes et légères à partir d'hybridomes murins

Les ARN totaux de chaque hybridome ont été extraits à l'aide du kit Macherey nagel ; Nucleospin RNAII (purification sur colonne).

Les ARNm ont été convertis en ADNc et les chaines lourdes et légères de l'anticorps ont été amplifiées à l'aide du kit Generacer (invitrogen) et clonées dans un vecteur M13. Des bactéries ont ensuite été transformées par le vecteur M13 et des clones positifs pour les séquences M13 ont été séquencés.

Détermination des segments VH, DH, JH des chaines lourdes et des segments VL et JL des chaines légères

La partie variable, les segments V et J utilisés par les chaines lourdes et légères et les séquences des CDR des chaines lourdes et légères ont été déterminés en utilisant l'outil Domaine Gapalign d'IMGT (voir Ehrenmann et al-2010 et Ehrenmann et al-2011) disponible à l'adresse suivante : http://www.imgt.org/3Dstructure-DB/cgi/DomainGapAlign.cgi.

Construction de vecteurs d'expression pour les anticorps chimériques

Les séquences des régions variables VH et VL des 5 anticorps murins ont été optimisées pour un usage préférentiel des codons de l'espèce *Rattus norvegicus.* Une séquence codant pour le peptide signal hétérologue MB7 a en outre été introduite en 5' de la séquence codant pour la région variable VH ou VL de chaque anticorps.

Les séquences des parties constantes humaines ont été extraites du vecteur CHK622-21 d'expression d'un anticorps humain anti-Rhésus D (T125) par digestion ApaI/AscI pour la chaine H (IgG1m1.17) et DraIII/XbaI pour la chaine Kappa.

Enfin, les parties variable et constante d'une même chaine ont été introduites en simultané dans le vecteur générique HKgenEFss par ligation avec la KAPA T4 ligase.

***Résultats***

Les données concernant les segments VH, DH, JH des chaines lourdes et des segments VL et JL des chaines légères des 5 anticorps sont présentées au **Tableau 2** présenté ci-dessus. On note que :

- Les 2 anticorps de la famille 1 (122A2 et 104C12) partagent l'utilisation du même segment VH (IGHV1S137*01), ainsi que l'utilisation de segments VL (IGKV10-96*01/IGKV10-96*02) et JL (IGKJ1*01/IGKJ1*02) de la même famille,

comme illustré dans le **Tableau 12** ci-dessous. Ces deux anticorps ont donc une structure proche.

- Les 3 anticorps de la famille 2 (102E9, 114D11 et 104E10) partagent l'utilisation des mêmes segments VH, JH, VL et JL, comme illustré dans le **Tableau 12** ci-dessous. Ces trois anticorps ont donc une structure proche.

**Tableau 12.** Segments VH, JH, VL et JL utilisés par les différents anticorps. Les segments identiques au sein d'une même famille sont en gras.

| **Famille 1** | | | | |
|---|---|---|---|---|
| Anticorps | VH | JH | VL | JL |
| 122A2 | **IGHV1S137*01** | IGHJ2*02 | **IGKV10-96***01 | **IGKJ1***01 |
| 104C12 | **IGHV1S137*01** | IGHJ3*01 | **IGKV10-96***02 | **IGKJ1***02 |
| **Famille 2** | | | | |
| Anticorps | VH | JH | VL | JL |
| 102E9 | **IGHV9-2-1*01** | **IGHJ4*01** | **IGKV4-57*01** | **IGKJ1*02** |
| 114D11 | **IGHV9-2-1*01** | **IGHJ4*01** | **IGKV4-57*01** | **IGKJ1*02** |
| 104E10 | **IGHV9-2-1*01** | **IGHJ4*01** | **IGKV4-57*01** | **IGKJ1*02** |

Par ailleurs, les données concernant les séquences d'acides aminés des CDRs et des régions variables des 5 anticorps sont présentées au **Tableau 3** présenté ci-dessus. On note que :

- Les 2 anticorps de la famille 1 (122A2 et 104C12) possèdent les mêmes séquences de CDR1-H et de CDR2-L, et ont par ailleurs des séquences de CDR2-H, CDR1-L et CDR3-L extrêmement proches (1 seul ou 2 acides aminés de différence). Même les séquences de CDR3-H présentent une forte homologie (5/11 acides aminés communs), comme illustré dans le **Tableau 13** ci-dessous. Cela confirme que ces deux anticorps ont une structure très proche.
- Les 3 anticorps de la famille 2 (102E9, 114D11 et 104E10) possèdent les mêmes séquences de CDR2-L, et ont par ailleurs des séquences de CDR1-H, CDR2-H, CDR1-L et CDR3-L extrêmement proches (1 seul acide aminé de différence). Même les séquences de CDR3-H présentent une forte homologie (12/14 acides aminés communs), comme illustré dans le **Tableau 13** ci-dessous. Cela confirme que ces trois anticorps ont une structure vraiment très proche.

**Tableau 13**. Homologie de séquence entre les CDRs des anticorps d'une même famille. Les acides aminés identiques au sein d'une même famille sont en gras.

| Famille 1 | | | | | | |
|---|---|---|---|---|---|---|
| Anticorps | CDR1-H | CDR2-H | CDR3-H | CDR1-L | CDR2-L | CDR3-L |
| 122A2 | **GYTFTDYS** | **IS**T**YYGD**S | **ARN**GNF**Y**VMDY | **QDI**S**NY** | YTS | **QQG**N**TLPWT** |
| 104C12 | **GYTFTDYS** | **IS**P**YYGD**T | ARNDDYYRFAY | **QDI**N**NY** | YTS | **QQG**K**TLPWT** |
| **Famille 2** | | | | | | |
| Anticorps | CDR1-H | CDR2-H | CDR3-H | CDR1-L | CDR2-L | CDR3-L |
| 102E9 | **GYTFTD**YS | **INETG**E**P** | T**RNGYYVGYYA**M**DY** | SSVIY | STS | **QQRRSYP**F**T** |
| 114D11 | **GYTFTD**S**S** | **INETG**G**P** | A**RNGYYVGYYA**L**DY** | SSVFY | STS | **QQRRSYP**Y**T** |
| 104E10 | **GYTFTD**YS | **INETG**E**P** | A**RNGYYVGYYA**M**DY** | SSVIY | STS | **QQRRSYP**Y**T** |

Les données concernant les séquences d'acides aminés des régions constantes des 5 anticorps sont présentées au **Tableau 5** présenté ci-dessus.
De plus, les séquences d'acides nucléiques des régions variables VH et VL et des régions constantes de chaque anticorps sont présentées aux **Tableaux 8** et 9 présentés ci-dessus. Enfin, les cartes des vecteurs d'expression des 5 anticorps sont présentées sur les **Figures 1A** à **1E.**

*Conclusions*

5 anticorps monoclonaux chimériques, avec des régions variables murines et des régions constantes humaines de type IgG1, et dirigés contre l'antigène CD303 ont été générés et leurs structures caractérisées. Il s'avère que deux anticorps (122A2 et 104C12) ont des structures proches et forment une sous-famille d'anticorps (famille 1), et que les trois autres anticorps (102E9, 114D11 et 104E10) sont également très proches sur le plan structural et forment une autre sous-famille d'anticorps (famille 2).
Ces anticorps ont ensuite été caractérisés au niveau de leurs propriétés biologiques (voir Exemple 2).

**Exemple 2 : Propriétés biologiques des 5 anticorps**

Les 5 anticorps monoclonaux chimériques, avec des régions variables murines et des régions constantes humaines de type IgG1, et dirigés contre l'antigène CD303 générés à l'Exemple 1 ont été testés pour différentes propriétés biologiques.

***Matériels et méthodes***

Liaison à l'antigène

*Liaison aux cellules CD303+ (Jurkat chaine Fc Gamma-CD303 et CAL-1)*

Des cellules Jurkat chaine Fc Gamma-CD303 (ou des cellules CAL-1 : Maeda T et al., Int J Hematol. 2005 Feb ; 81(2) :148-54) et les anticorps sont préparés dans du diluant (PBS + 1% SVF).
1 x $10^5$ cellules sont incubées à 4°C pendant 30 minutes avec 100 µL d'anticorps (anti-CD303 ou contrôle négatif) à différentes concentrations (0-40 µg/mL, concentration finale).
Après lavage avec le diluant, les anticorps sont visualisés par ajout d'un fragment F(ab')2 d'IgG de chèvre anti-souris couplé à la phycoérythrine (PE) (100 µL à une dilution de 1:100 dans le diluant) pendant 45 minutes à 4°C. Les cellules sont ensuite lavées et analysées par cytométrie en flux (FC500, Beckman Coulter).

Liaison au récepteur FcεRIIIa (CD16a)

Les cellules NK ont été isolées des cellules mononucléaires du sang périphérique (PBMC), puis incubées avec des concentrations variables des anticorps testés (0 à 100 µg/ml) simultanément incubées avec l'anticorps murin couplé à la phycoérythrine (3G8-PE, Beckman Coulter) à 10µl/test.
Après lavage, la fixation de 3G8-PE au CD16 exprimé par les cellules NK a été évaluée par cytométrie de flux. Les valeurs de fluorescence moyenne (MFI) sont exprimées en pourcentage, 100 % étant la valeur obtenue avec l'anticorps 3G8-PE seul, et 0% la valeur obtenue en l'absence de 3G8-PE.
Les valeurs IC50 (concentration d'anticorps anti-CD303 requise pour induire une inhibition de fixation de 3G8 de 50%) sont calculées à l'aide du logiciel PRISM.

ADCC

Des cellules Jurkat chaine Fc Gamma-CD303 (35000 cellules/puits) sont incubées dans une plaque 96 puits à fond plat avec des cellules NK et des concentrations croissantes d'anticorps anti-CD303 pendant 4 heures à 37°C. Après incubation, le surnageant est collecté. La lyse des cellules cibles induite par les anticorps anti-CD303 est mesurée de façon chromogénique en quantifiant l'enzyme intracellulaires lactate déshydrogénase (LDH) relarguée dans le surnageant par les cellules cibles lysées (Roche Diagnostics - Cytotoxicity Detection Kit LDH).
Le pourcentage de lyse est calculé selon la formule suivante :

$$\%\ \text{lyse} = [(ER - SR) / (100 - SR)] - [(NC - SR) / (100 - SR)]$$

Où ER et SR représentent respectivement les relargages expérimental (ER) et spontané (SR) de LDH, et NC représente la cytotoxicité naturelle des cellules NK.
Les résultats (% lyse) sont exprimés en fonction du facteur de dilution de l'anticorps. Pour chaque anticorps, la valeur d'« activité 50% » correspond au facteur de dilution de l'anticorps nécessaire pour induire 50% de la valeur plateau obtenue pour cet anticorps. Cette valeur a été calculée avec le logiciel PRISM.

***Résultats***

Liaison à l'antigène

*Liaison aux cellules Jurkat chaine Fc Gamma-CD303*

Les résultats des tests de liaison des anticorps selon l'invention à leur antigène CD303 sur les cellules Jurkat chaine Fc Gamma-CD303 sont présentés à la **Figure 2A** et dans le **Tableau 14** ci-dessous.

**Tableau 14.** Liaison des anticorps selon l'invention à leur antigène CD303 sur les cellules Jurkat chaine Fc Gamma-CD303. Bmax : Liaison maximale exprimée en intensité moyenne de fluorescence (MFI). EC50 (μg/mL) : concentration d'anticorps en μg/mL permettant d'obtenir 50% de la liaison maximale obtenue pour cet anticorps.

| | 104C12 | 122A2 | 114D11 | 102C9 | 104E10 |
|---|---|---|---|---|---|
| Bmax (MFI) | 27,74 | 26,21 | 24,69 | 24,95 | 23,73 |
| EC50 (μg/mL) | 0,1781 | 0,1284 | 3,980 | 3,870 | 6,064 |

Ces résultats de Kd relatifs et les valeurs de Bmax calculés après modélisation de la courbe dose réponse, permettent de classer les anticorps en 2 groupes :Un premier groupe qui contient les anticorps 104C12 (Bmax : MFI= 27.7; Kd=0.17 μg/ml) and 122A2 (Bmax : MFI= 26.2 Kd=0.13 μg/ml) qui sont comparables et présentent une meilleure affinité relative que les anticorps du deuxième groupe : 114D11(Bmax : MFI= 24.7; Kd=3.9 μg/ml), 104E10 (Bmax : MFI= 23.7; Kd= 6 μg/ml) et 102E9 (Bmax : MFI= 24.9; Kd=3.8 μg/ml).
Ces résultats montrent que l'ensemble des anticorps chimériques générés lient efficacement l'antigène CD303 exprimé à la surface des cellules Jurkat, pour lequel ils sont spécifiques.

*Liaison aux cellules CAL-1*

Les résultats des tests de liaison des anticorps selon l'invention à leur antigène CD303 sur les cellules CAL-1 sont présentés à la **Figure 2B** et dans le **Tableau 15** ci-dessous.

**Tableau 15.** Liaison des anticorps selon l'invention à leur antigène CD303 sur les cellules CAL-1. Bmax : Liaison maximale exprimée en intensité moyenne de fluorescence (MFI). EC50 (μg/mL) : concentration d'anticorps en μg/mL permettant d'obtenir 50% de la liaison maximale obtenue pour cet anticorps.

| | 104C12 | 122A2 | 114D11 | 102C9 | 104E10 |
|---|---|---|---|---|---|
| Bmax (MFI) | 29.02 | 25.22 | 30.14 | 30.47 | 29.2 |
| EC50 (μg/mL) | 0.3447 | 0.2075 | 1.704 | 1.813 | 1.932 |

Ces résultats de Kd relatifs et les valeurs de Bmax calculés après modélisation de la courbe dose réponse, permettent de classer les anticorps en 2 groupes :Un premier groupe qui contient les anticorps 104C12 (Bmax : MFI= 29.02 Kd=0.34 μg/ml) and 122A2 (Bmax : MFI= 25.2 Kd=0.20 μg/ml) qui sont comparables et présentent une meilleure affinité relative que les anticorps du deuxième groupe : 114D11(Bmax : MFI=30.1 ; Kd=1.7 μg/ml), 104E10 (Bmax : MFI=29.2 ; Kd= 1.93 μg/ml) et 102E9 (Bmax : MFI=30.47 ; Kd=1.81 μg/ml).
Ces résultats corrèlent notamment avec les résultats de liaison aux cellules Jurkat-CD303.

Liaison au récepteur FcγRIIIa (CD16a)

Les résultats des tests de liaison au récepteur FcγRIIIa (CD16a) sont présentés sur la **Figure 3**, et montrent que les anticorps chimériques selon l'invention sont tous capables de lier efficacement le CD16a, avec une affinité de liaison optimisée. Ils possèdent une valeur d'IC50 inférieure à celle de l'anticorps produit dans les cellules CHO (Rituxan)

dans une gamme de 18,08 à 45,02 µg/ml, voire inférieure à 18 µg/ml, comme illustré dans le Tableau 16 ci-dessous.

**Tableau 16**. Valeurs d'IC50 (concentration d'anticorps anti-CD303 requise pour induire une inhibition de fixation de 3G8 de 50%) du contrôle Rituxan et des anticorps chimériques de l'invention

| | RITUXAN | 104C12 | 122A2 | 114D11 | 102E9 | 104E10 |
|---|---|---|---|---|---|---|
| IC50 (µg/ml) | > 80 | 26,75 | 40,44 | 45,02 | 36,99 | 18,08 |

ADCC

Les résultats des tests ADCC sont présentés sur la **Figure 4** et dans le **Tableau 17** ci-dessous.

**Tableau 17.** ADCC induite par les anticorps selon l'invention sur des cellules cibles Jurkat chaine Fc Gamma-CD303. Emax : lyse maximale obtenue avec cet anticorps, exprimée en pourcentage de lyse. EC50 (ng/mL) : concentration d'anticorps en ng/mL permettant d'obtenir 50% de la lyse maximale obtenue pour cet anticorps.

| | 104C12 | 122A2 | 114D11 | 102C9 | 104E10 |
|---|---|---|---|---|---|
| Emax (% lyse) | 41,81 | 42,35 | 37,44 | 38,92 | 40,54 |
| EC50 (ng/mL) | 0,2143 | 0,1592 | 3,612 | 3,424 | 8,280 |

Ces résultats montrent que les cinq anticorps chimériques anti-CD303 induisent la lyse de la cellule Jurkat-CD303 (Emax environ 40%). Les valeurs d'EC50 pour 104C12 (EC50 : 0.21 ng/ml), 122A2 (EC50 : 0.16 ng/ml), 114D11 (EC50 : 3.6 ng/ml), 102C9 (EC50 : 3.4 ng/ml) et 104E10 (EC50 : 8.3 ng/ml) suggèrent que les anticorps ayant une forte affinité sont plus efficace en ADCC que les anticorps de plus faible affinité.

**Exemple 3 : Tests en sang total de déplétion de cellules**

La capacité de l'anticorps 122A2 (tel qu'indiqué à l'Exemple 1) à dépléter des cellules NF-3C8, en sang total, a été testée.

*Matériels et méthodes*

Les cellules NF-3C8 sont des cellules issues de patients atteints de BPDCN (Blastic Plasmacytoid Dendritic Cell Neoplasm). Ces cellules ont été produites à partir de la lignée cellulaire de départ CAL-1 telle que décrite dans Maeda T et al. (Int J Hematol., 2005 Fev, 81(2):148-54) exprimant naturellement la chaine gamma du récepteur FcεRI. Après transfection de cette lignée avec CD303, la lignée 3C8 a été sélectionnée pour exprimer de façon stable entre 40 000 et 50 000 antigènes CD303 à leur surface.

Test en sang total

Pour mimer en conditions *ex-vivo* le sang d'un patient atteint de BPDCN, 500 cellules NF-3C8/µL ont été ajoutées à 100 µL de sang total (en présence d'un anticoagulant : lithium héparinate, issu de donneurs sains, puis incubées dans des tubes à essai stériles avec 1 µg/mL de l'anticorps 122A2 (« Ch. 122A2 mAb ») ou de l'anticorps témoin (« témoin ») toute une nuit à 37°C.

Spécificité de l'antigène et reconnaissance

Après une nuit d'incubation, les cellules sont colorées directement dans le tube à essai avec CD3-FITC (un anticorps anti-CD3, tel que référence A07746 chez Beckman Coulter), CD123PE (un anticorps anti-CD123, tel que référence 130-090-899 chez Miltenyi Biotec), CD56-PC5 (un anticorps anti-CD56, tel que référence A07789 chez Beckman Coulter), et incubées 20 minutes à 4°C. Pour supprimer les globules rouges, les tubes sont traités avec Q-PREP Workstation et IMMUNOPREP Reagent System (Beckman Coulter). Les cellules sont ensuite étudiées en cytométrie de flux (FC500, Beckman Coulter).

La déplétion des cellules NF-3C8 a été quantifiée en cytométrie de flux, basée sur le pourcentage de cellules CD123+CD56+ comparé au pourcentage de cellules CD3+, qui sont utilisées comme cellules de référence.
Les résultats sont présentés en pourcentage de déplétion des cellules NF-3C8. L'anticorps témoin correspond à 0% de

déplétion.
Le pourcentage de déplétion a été calculé de la façon suivante :

- 1ère étape : % de NF-3C8 vs CD3 :

$$X = (\text{nombre de NF-3C8 CD56+CD123+}) / (\text{nombre de cellules CD3+}) * 100$$

- 2ème étape : % de déplétion :

$$100 - (X \text{ de « Ch. 122A2 mAb »} / X \text{ de « témoin »}) * 100$$

Résultats

Les résultats des tests en sang total sont présentés sur la **Figure 5** et dans le **Tableau 18** ci-dessous.

**Tableau 18.** Données correspondantes à la Figure 5

| **mAb - 1µg/ml** | **16h d'incubation à 37°C** | | | |
|---|---|---|---|---|
| **1825 15 865 Donneur 1** | **Nombre de cellules NF-3CB (CD56÷CD113+)** | **Nombre de cellules CD3+** | **% = (CDS6+CD123+ /CD3+)*100** | **% de déplétion NF-3C8** |
| **Témoin** | **4708** | **16042** | **29.35** | **0.0** |
| **Ch. 122A2 mAb** | **2084** | **13860** | **15.04** | **48.8** |
| **1825 15 904 Donneur 2** | | | | |
| **Témoin** | **4186** | **13703** | **30.55** | **0.0** |
| **Ch. 122A2 mAb** | **2246** | **13177** | **17.04** | **44,2** |
| **1825 15 904 Donneur 3** | | | | |
| **Témoin** | **3842** | **3739** | **102.75** | **0.0** |
| **Ch. 122A2 mAb** | **2140** | **3560** | **60.11** | **41.5** |
| **1525 15 905 Donneur 4** | | | | |
| **Témoin** | **3261** | **8384** | **38.90** | **0.0** |
| **Ch. 122A2mAb** | **1983** | **9035** | **21.95** | **43.6** |
| **1825 15 906 Donneur 5** | | | | |
| **Témoin** | **2166** | **10915** | **25.34** | **0.0** |
| **Ch. 122A2 mAb** | **2843** | **16459** | **17.27** | **31.8** |

Ces résultats montrent qu'après 16h d'incubation à 37°C l'anticorps « Ch. 122A2 mAb » induit la déplétion des cellules NF-3C8 dans les échantillons sanguins de cinq donneurs sains indépendants les uns des autres. Au contraire, l'anticorps témoin n'induit pas de déplétion. Ces résultats montrent donc que l'anticorps 122A2 est capable d'induire la

déplétion des cellules NF-3C8.

**Exemple 4 : Impact de l'élimination des pDC sur les activités ADCC et phagocytose d'un anticorps dirigé contre un antigène tumoral**

***Principe de l'étude***

Les pDC (cellules dendritiques plasmacytoïdes) sont responsables de la différenciation des T régulateurs entre autre par l'interaction ICOS/ICOSL. Les T régulateurs ainsi différentiés exercent des mécanismes d'immunosuppression sur les fonctions des autres cellules du système immunitaire, notamment les cellules NK, *via* le contact cellule/cellule mais également *via* la sécrétion de cytokines immuno-modulatrices comme IL-10, IL-35 et TGF-β. Par effet cascade, il est ainsi possible d'étudier l'impact de l'élimination des pDC sur l'activité d'un agent anti-cancéreux spécifique d'une tumeur impliquant une activation des pDC *in situ.* Notamment, l'effet protecteur des anticorps anti-CD303 ciblant les pDC peut être démontré par l'étude corrélative de la diminution ou élimination des cytokines IL-10 et TGF-β dans l'environnement tumoral et son impact sur les fonctions effectrices d'un agent anti-cancéreux administré, spécifique de la tumeur (par exemple un anticorps anti-antigène de tumeur solide). En effet, selon ce modèle, les anticorps anti-CD303 déplétant les pDC entrainent une limitation des effets immunosuppresseurs des T régulateurs et ainsi une limitation de leur sécrétion d'IL-10 et TGF-β. Cette limitation de sécrétion d'IL-10 et TGF-β est corrélée à une meilleure ADCC des cellules NK, validant l'effet stimulateur indirect des anticorps anti-CD303 sur l'action anti-cancéreuse des anticorps anti-antigène de tumeur, par exemple de tumeur solide.

***1 - Effet de l'IL-10 et TGF-β sur l'activité ADCC d'un anticorps anti-antigène tumoral***

Afin de tester l'ADCC dans un contexte tumoral impliquant une activation des pDC d'une part ou en éliminant les pDC d'autre part, des cellules présentatrices de l'antigène tumoral (35000 cellules/puits) sont incubées dans une plaque 96 puits à fond plat avec des cellules effectrices NK et des concentrations croissantes d'anticorps spécifique de l'antigène tumoral, en présence ou non d'IL-10 (5 et 50 ng/ml) , de TGF-β (5 et 50 ng/ml) ou d'IL-10 + TGF-β (5 et 50 ng/ml pour chacune des cytokines) pendant 16 heures à 37°C. Après incubation, le surnageant est collecté.
La lyse des cellules cibles induite par les anticorps anti-cancéreux est mesurée de façon chromogénique en quantifiant l'enzyme intracellulaire lactate déshydrogénase (LDH) relarguée dans le surnageant par les cellules cibles lysées (Roche Diagnostics - Cytotoxicity Detection Kit LDH).
Le pourcentage de lyse est calculé selon la formule suivante :

$$\%\ \text{lyse} = [(ER - SR) / (100 - SR)] - [(NC - SR) / (100 - SR)]$$

Où ER et SR représentent respectivement les relargages expérimental (ER) et spontané (SR) de LDH, et NC représente la cytotoxicité naturelle des cellules NK.
Les résultats (% lyse) sont exprimés en fonction du facteur de dilution de l'anticorps. Pour chaque anticorps, la valeur d'« activité 50% » correspond au facteur de dilution de l'anticorps nécessaire pour induire 50% de la valeur plateau obtenue pour cet anticorps. Cette valeur peut être calculée avec le logiciel PRISM.
Conclusion : Par effet cascade, la comparaison du pourcentage de lyse observable en l'absence de cytokines l'IL-10 et/ou TGF-β, avec le pourcentage de lyse observable en présence de ces mêmes cytokines, permet d'évaluer l'impact de la déplétion des pDC présents sur le site tumoral. Il peut ainsi être démontré que les anticorps anti-CD303 permettent de potentialiser indirectement l'effet des anticorps anti-cancéreux.

***2 - Effet de l'IL-10 et TGF-β sur la phagocytose induite par un anticorps anti-antigène tumoral***

Les monocytes sont différenciés en macrophages CD16+ (M2 like) pendant 2 jours en RPMI 1640 + 10 % SVF + M-CSF 50 ng/ml pendant 48h.
Les cellules exprimant l'antigène de tumeur et les macrophages sont marqués avec PKH-67 (fluorescence verte) et PKH-26 (fluorescence rouge), respectivement.
Les cellules exprimant l'antigène tumoral sont opsonisées avec 10 μg/ml de l'anticorps spécifique de cet antigène ou avec un anticorps irrelevant puis incubées avec les macrophages (1.105 de chaque cellules/puits) en absence ou en présence de différentes concentrations d'IL-10 (5 et 50 ng/ml) seule, de TGF-β (5 et 50 ng/ml) seul et d'IL-10 + TGF-β (5 et 50 ng/ml). Après 3h d'incubation à 37°C, les cellules sont placées sur une cellule de comptage (Mallassez) et observées avec un microscope à fluorescence.
Le pourcentage de phagocytose est évalué en comptant le nombre de macrophages (au moins 100 macrophages)

contenant des cellules tumorales.

Conclusion : Par effet cascade, la comparaison du pourcentage de phagocytose observable en l'absence de cytokines l'IL-10 et/ou TGF-β, avec le pourcentage de lyse observable en présence de ces mêmes cytokines, permet d'évaluer l'impact de la déplétion des pDC présents sur le site tumoral. Il peut ainsi être démontré que les anticorps anti-CD303 permettent de potentialiser indirectement l'effet des anticorps anti-cancéreux.

**Exemple 5 : Effet d'un anticorps anti-CD303 sur l'activation des cellules T régulatrices (Treg)**

***1 - Rôle de l'anti-CD303 sur le phénotype et l'expansion des Treg en PBMC***

Les cellules mononucléées (PBMC) sont isolées à partir d'un tube de sang prélevé sur anticoagulant. Les cellules Treg sont identifiées et phénotypiquement caractérisées par cytométrie en flux sur la base de 3 marqueurs : CD4, CD25 et Fox-P3.

Différentes quantités de l'anticorps anti-CD303 (de 1 ng à 10 μg/ml) sont ajoutées aux PBMC en présence d'IL-2 (500 U/ml). Le nombre de Treg et leur phénotype est suivi au cours du temps (1 à 4 jours).

Dans les mêmes conditions, des billes coatées avec anti-CD3 / anti-CD28 pour stimuler la prolifération T sont ajoutées dans un ratio Treg / billes de 4 / 1 pour vérifier l'activation des Treg.

Conclusion : Il peut ainsi être démontré que les anticorps anti-CD303, en l'absence de pDC, n'ont pas d'impact direct sur l'expansion et le phénotype immunosuppresseur des T régulateurs.

***2 - Rôle de l'anti-CD303 sur le phénotype et l'expansion de Treg purifiés en présence de pDC***

Les cellules Treg ($CD4^+$, $CD25^+$) sont purifiées à partir de PBMC grâce à un procédé en 2 étapes : déplétion des cellules CD4 négatives (cellules positives pour les marqueurs CD8, CD14, CD15, CD16, CD19, CD36, CD56, CD123, TCKγ/δ, et CD235a) puis sélection positive des cellules $CD25^+$.

Des pDC purifiées ou des lignées pDC, e.g. obtenues selon le procédé décrit dans Maeda T et al., Int J Hematol. 2005 Feb ; 81(2) :148-54 (telle que la lignée CAL-1 ou NF-3C8) sont ajoutées dans un ratio Treg / pDC de 100, 10 et 1. Différentes quantités de l'anticorps anti-CD303 (de 1 ng à 10 μg/ml) sont ajoutées au mélange Treg / pDC en présence d'IL-2 (500 U/ml). Le nombre de Treg et leur phénotype est suivi au cours du temps (1 à 4 jours).

Dans les mêmes conditions, des billes coatées avec anti-CD3 / anti-CD28 pour stimuler la prolifération T sont ajoutées dans un ratio Treg / billes de 4 /1 pour vérifier l'activation des Treg.

Un témoin négatif en absence de pDC est établi, de façon à vérifier l'impact (attendu neutre) de l'anti-CD303 directement sur les Treg.

Conclusion : L'observation sur plusieurs jours de l'expansion et de la différenciation des Treg purifiés en présence de pDC, après administration d'anticorps anti-CD303 peut permettre de montrer que l'administration d'anti-CD303 est efficace pour diminuer ou supprimer les propriétés immunosuppressives des pDC.

**Exemple 6 : Déplétion des pDCs humaines via un anticorps anti-CD303 *in vivo* dans le traitement d'un cancer solide**

***1 - Génération d'un modèle d'étude reproductif d'une situation pathologique chez l'homme***

Afin d'étudier l'effet d'un anticorps anti-CD303 dans le traitement d'un cancer solide, un modèle de souris humanisée 'humanized tumor mouse model' (HTM) peut être utilisé. Il est caractérisé par le développement d'un système immunitaire mature humain et la croissance de cellules de cancer solide humaines qui ont été au préalable co-transplantées avec les cellules souches hématopoïétiques humaines.

Ce modèle permet avantageusement de réunir plusieurs éléments pertinents pour la reproductibilité des conditions *in vivo* : présence de pDCs humaines qui seules expriment à leur surface la cible CD303, présence d'infiltration de cellules Treg humaines, présence de cellules tumorales humaines exprimant à leur surface un antigène tumoral cible, molécule ciblée par un anticorps anti-tumoral et un hôte murin immunocompétent (cellules effectrices de type NK pour l'activité ADCC).

Brièvement, des souris NOD-scid IE2Kγnull (NSG) peuvent être obtenues, par exemple auprès des Laboratoires Jackson, puis hébergées dans un établissement spécialisé sans pathogène. Les nouveau-nés seront irradiés (1 Gy) pendant leurs 48 premières heures de vie et 3 heures plus tard transplantées par injection intra-hépatique avec $2,5x10^5$ cellules CD34+ humaines isolées à partir de sang du cordon ombilical (CB) en présence de $3x10^6$ cellules tumorales exprimant spécifiquement un antigène de tumeur solide et exprimant la luciférase pour un suivi en bioluminescence.

***2 - Méthode pouvant être utilisée pour tester l'activité de l'anticorps anti-CD303***

Dès que la tumeur est visible par bioluminescence (IVIS), les souris HTM sont traitées avec 20 mg/kg d'anticorps anti-antigène de tumeur par voie péritonéale toutes les semaines et avec l'anticorps anti-CD303 à la dose 30mg/kg tous les 3 jours par voie intraveineuse.

Le suivi de l'efficacité du traitement est réalisé en bioluminescence, la survie des animaux ainsi que la survie en absence de tumeurs sont monitorées. Des prélèvements sanguins sont réalisés au cours de l'étude afin de s'assurer de l'efficacité de l'anticorps anti-CD303 sur la déplétion des pDCs dans ce contexte tumoral.

Les différentes conditions testées sont ainsi avantageusement les suivantes (10 animaux par groupe) :

1. HTM + anti-antigène tumoral
2. HTM + anti-CD303
3. HTM + anti-antigène tumoral + anti-CD303
4. HTM + isotype Contrôle

Conclusion :

Le modèle murin HTM peut être avantageusement utilisé pour évaluer l'effet indirect de l'administration d'un anticorps anti-CD303 sur l'effet de l'agent anti-tumeur solide, l'anticorps anti-antigène tumoral, dans des conditions reproduisant une situation physiologique *in vivo,* en comparant notamment les résultats obtenus en condition 3 ci-dessus, avec d'une part ceux obtenus en condition 1, et d'autre part ceux obtenus en condition 2.

Ce modèle est ainsi utile pour pouvoir évaluer le bénéfice, avantageusement l'effet synergique, d'administrer un anticorps anti-CD303 en combinaison à l'administration d'un anticorps anti-antigène tumoral dans une tumeur solide.

## Revendications

1. Anticorps, notamment monoclonal ou polyclonal, dirigé contre la protéine CD303 pour son utilisation dans la prévention ou le traitement d'une tumeur impliquant une activation de cellules dendritiques plasmacytoïdes dans l'environnement de ladite tumeur, lesdites cellules dendritiques plasmacytoïdes ayant des propriétés immunosuppressives et/ou tolérogènes, et n'étant pas à l'origine de la tumeur et n'ayant pas le marqueur CD56+.

2. Anticorps dirigé contre la protéine CD303 pour son utilisation selon la revendication 1, dans laquelle les tumeurs impliquant une activation des cellules dendritiques plasmacytoïdes sont des tumeurs solides ou des tumeurs hématopoïétiques.

3. Anticorps dirigé contre la protéine CD303 pour son utilisation selon la revendication 2, dans laquelle lesdites tumeurs solides impliquent une infiltration de cellules dendritiques plasmacytoïdes dans l'environnement de ladite tumeur, et appartiennent de préférence au groupe des tumeurs de la tête et du cou, du mélanome, des cancers urogénitaux, du cancer du sein.

4. Anticorps dirigé contre la protéine CD303 pour son utilisation selon la revendication 2, dans laquelle lesdites tumeurs hématopoïétiques appartiennent au groupe du myélome multiple, du lymphome, de la leucémie, notamment de la leucémie à cellules T.

5. Anticorps dirigé contre la protéine CD303 pour son utilisation selon l'une quelconque des revendications 1 à 4, dans laquelle ledit anticorps est choisi parmi un anticorps murin, un anticorps chimérique, un anticorps humanisé ou un anticorps humain, notamment un anticorps chimérique et de préférence un anticorps chimérique choisi parmi un anticorps chimérique murin/humain ou un anticorps chimérique humain/macaque.

6. Anticorps dirigé contre la protéine CD303 pour son utilisation selon l'une quelconque des revendications 1 à 5, dans laquelle l'anticorps possède une faible teneur en fucose, inférieure ou égale à 65%, et/ou une teneur en N-glycannes de type oligomannose supérieure ou égale à 30%, et/ou une teneur en galactose supérieure ou égale à 50%.

7. Anticorps dirigé contre la protéine CD303, pour son utilisation selon l'une quelconque des revendications 1 à 6, dans laquelle ledit anticorps est couplé à une molécule bioactive choisie parmi :

   - les radio-isotopes, notamment choisis parmi At211, I131, I125, Y90, Re186, Re188, Sm153, Bi212, P32,
   - les métaux non-radioactifs,
   - les toxines, notamment choisies parmi la ricine, l'abrine, la toxine diphtérique,
   - les acides nucléiques, notamment choisis parmi les ARNs anti-sens,
   - les enzymes, notamment choisies parmi les RNases, la biotine, l'avidine ou la streptavidine,
   - les agents cytotoxiques, notamment choisis parmi :

- les antifolates, et plus particulièrement le méthotrexate, le pémétrexed, le raltitrexed ;
- les anti-purines, et plus particulièrement la cladribine, la fludarabine, l'azathioprine, l'azathioprine, la mercaptopurine, le 5-fluorouracile, la capécitabine, la cytarabine, la gemcitabine,
- les inhibiteurs de topoisomérases I et II,
- les agents alkylants et agents apparentés, et plus particulièrement chlorméthine, cyclophosphamide, ifosfamide, carmustine, fotémustine, mitomycine C, cisplatine, carboplatine, oxaliplatine,
- les agents intercalants,
- les anthracyclines, et plus particulièrement choisis parmi daunorubicine, doxorubicine et chlorydrate, epirubicine, idarubicine, bléomycine,
- les taxanes,
- les inhibiteurs spécifiques de tyrosine-kinase, imatinib, erlotinib.

**8.** Anticorps dirigé contre la protéine CD303, pour son utilisation selon l'une quelconque des revendications 1 à 7, dans laquelle ledit anticorps est utilisé en combinaison avec au moins un agent anti-cancéreux, notamment un agent anti-cancéreux chimique et/ou un agent anti-cancéreux utilisé en immunothérapie.

**9.** Anticorps dirigé contre la protéine CD303, pour son utilisation selon l'une quelconque des revendications 1 à 8, dans laquelle ledit agent anti-cancéreux chimique est choisi parmi les agents anti-métaboliques, les agents alkylants, les agents intercalants, ou les molécules ayant une action sur le fuseau mitotique, et de préférence :

- lesdits agents métaboliques sont choisis parmi :

  - les antifoliques, notamment méthotrexate, raltitrexed, et pemetrexed),
  - les antipuriques, notamment mercaptopurine, thioguanine, pentostatine, cladribine et fludarabine,
  - les antipyrimidiques, notamment 5-fluoro-uracile, tégafur uracile, capécitabine et cytarabine, et
  - les anti-métaboliques, notamment l'hydroxycarbamide, l'hydroxyurée et la gemcitabine,

- lesdits agents alkylants sont choisis parmi :

  - les moutardes à l'azote, notamment chlorambucil, melphelan, chlorméthine, métachloroéthamine, estramustine, ifosfamide et cyclophosphamide,
  - les nitroso-urées, notamment fotémustine, lomustine, carmustine, streptozocine,
  - les organoplatines, notamment carboplatine, cisplatine et oxaliplatine,
  - les ethylène imines, notamment thiotépa et altrétamine,
  - les triazènes, notamment procarbazine, témozolomide et dacarbazine,
  - les agents alkylants, notamment busulfan, mitomycine C et pipobroman,

- lesdits agents intercalants sont choisis parmi :

  - les dérivés de la camptothécine, notamment irinotécan et topotécan,
  - les antrhracyclines, notamment epirubicine, daunorubicine, doxorubicine, pirarubicine et idarubicine,
  - les agents intercalants, notamment mitoxantrone, amsacrine, elliptinium, actinomycine d, dactinomycine, etoposide et bléomycine,

- lesdites molécules ayant une action sur le fuseau mitotique sont choisies parmi :

  - les vinca-alcaloïdes ou poisons du fuseau, notamment vinorelbine, vindésine, vincristine et vinblastine,
  - les taxoïdes ou stabilisants du fuseau, notamment paclitaxel et docétaxel,
  - les inhibiteurs de tyrosine kinase, notamment dasatinib, erlotinib, imatinib, sorafénib et sunitinib.

**10.** Anticorps dirigé contre la protéine CD303, pour son utilisation selon la revendication 9, dans laquelle ledit agent anti-cancéreux utilisé en immunothérapie est un anticorps spécifique de la tumeur ciblée, un anticorps anti-CD123, ou un agoniste des TLR.

**11.** Anticorps dirigé contre la protéine CD303, pour son utilisation selon l'une quelconque des revendications 8 à 9, dans laquelle ladite utilisation dudit anticorps et ladite utilisation dudit agent anti-cancéreux sont simultanées, séparées ou étalées dans le temps.

**12.** Anticorps dirigé contre la protéine CD303, pour son utilisation selon l'une quelconque des revendications 1 à 11, dans laquelle ladite utilisation est couplée à une radiothérapie.

**13.** Anticorps dirigé contre la protéine CD303, pour son utilisation selon l'une quelconque des revendications 1 à 12, dans laquelle ladite prévention ou ledit traitement est effectué chez un patient présentant une déplétion en cellules dendritiques plasmacytoïdes, ou chez un patient ayant besoin ou nécessitant une déplétion en cellules dendritiques plasmacytoïdes.

**14.** Produit contenant :

- un anticorps, notamment monoclonal ou polyclonal, dirigé contre la protéine CD303 tel que défini dans l'une des revendications 5 à 7 ; et
- au moins un agent anti-cancéreux, notamment un agent anti-cancéreux chimique et/ou un agent anti-cancéreux utilisé en immunothérapie,

comme produit de combinaison pour une utilisation simultanée, séparée ou étalée dans le temps, pour son utilisation dans la prévention ou le traitement d'une tumeur impliquant une activation de cellules dendritiques plasmacytoïdes dans l'environnement de ladite tumeur, lesdites cellules dendritiques plasmacytoïdes ayant des propriétés immunosuppressives et/ou tolérogènes, et n'étant pas à l'origine de la tumeur et n'ayant pas le marqueur CD56+.

## Patentansprüche

**1.** Antikörper, insbesondere monoklonaler oder polyklonaler Antikörper, der gegen das CD303-Protein gerichtet ist, zur Verwendung bei der Prävention oder Behandlung eines Tumors, der ein Aktivierung von plasmazytoiden dendritischen Zellen in der Umgebung des Tumors beinhaltet, wobei die plasmazytoiden dendritischen Zellen immunsuppressive und/oder tolerogene Eigenschaften haben, nicht aus dem Tumor stammen und keinen CD56+-Marker aufweisen.

**2.** Antikörper, der gegen das CD303-Protein gerichtet ist, zur Verwendung nach Anspruch 1, wobei es sich bei den Tumoren, die eine Aktivierung von plasmazytoiden dendritischen Zellen beinhalten, um solide Tumoren oder hämatopoetische Tumoren handelt.

**3.** Antikörper, der gegen das CD303-Protein gerichtet ist, zur Verwendung nach Anspruch 2, wobei die soliden Tumoren eine Infiltration plasmazytoider dendritischer Zellen in der Umgebung des Tumors beinhalten und vorzugsweise zur Gruppe der Kopf- und Halstumore, der Melanome, der urogenitalen Tumore und Brustkrebs gehören.

**4.** Antikörper, der gegen das CD303-Protein gerichtet ist, zur Verwendung nach Anspruch 2, wobei die hämatopoetischen Tumoren zur Gruppe des multiplen Myeloms, des Lymphoms, der Leukämie, insbesondere der T-Zell-Leukämie, gehören.

**5.** Antikörper, der gegen das CD303-Protein gerichtet ist, zur Verwendung nach einem der Ansprüche 1 bis 4, wobei der Antikörper gewählt ist aus einem murinen Antikörper, einem chimären Antikörper, einem humanisierten Antikörper oder einem humanen Antikörper, insbesondere einem chimären Antikörper und vorzugsweise einem chimären Antikörper, gewählt aus einem murinen/humanen chimären Antikörper oder einem humanen/makaken chimären Antikörper.

**6.** Antikörper, der gegen das CD303-Protein gerichtet ist, zur Verwendung nach einem der Ansprüche 1 bis 5, wobei der Antikörper einen niedrigen Fucosegehalt von 65% oder mehr und/oder einen N-Glycan-Gehalt vom Oligomannose-Typ von 30% oder mehr und/oder einen Galactosegehalt von 50% oder mehr aufweist.

**7.** Antikörper, der gegen das CD303-Protein gerichtet ist, zur Verwendung nach einem der Ansprüche 1 bis 6, wobei der Antikörper an ein bioaktives Molekül gekoppelt ist, gewählt aus:

- Radioisotopen, insbesondere gewählt aus At211, I131, I125, Y90, Re186, Re188, Sm153, Bi212, P32,
- nicht-radioaktiven Metallen,
- Toxinen, insbesondere gewählt aus Ricin, Abrin, Diphtherietoxin,
- Nukleinsäuren, insbesondere gewählt aus Antisense-RNAs,

- Enzymen, insbesondere gewählt aus RNasen, Biotin, Avidin oder Streptavidin, zytotoxischen Mitteln, insbesondere gewählt aus

  - Antifolaten, und insbesondere Methotrexat, Pemetrexed, Raltitrexed;
  - Antipurinen, und insbesondere Cladribin, Fludarabin, Azathioprin, Azathioprin, Mercaptopurin, 5-Fluorouracil, Capecitabin, Cytarabin, Gemcitabin,
  - Inhibitoren der Topoisomerasen I und II,
  - Alkylierungsmitteln und verwandten Mitteln, insbesondere Chlormethin, Cyclophosphamid, Ifosfamid, Carmustin, Fotemustin, Mitomycin C, Cisplatin, Carboplatin, Oxaliplatin,
  - Interkalationsmitteln,
  - Anthracyclinen, und insbesondere gewählt aus Daunorubicin, Doxorubicin und Chlorid, Epirubicin, Idarubicin, Bleomycin,
  - Taxanen,
  - spezifischen Tyrosinkinaseinhibitoren, Imatinib, Erlotinib.

**8.** Antikörper, der gegen das CD303-Protein gerichtet ist, zur Verwendung nach einem der Ansprüche 1 bis 7, wobei der Antikörper in Kombination mit wenigstens einem Anti-Krebs-Mittel, insbesondere einem chemischen Anti-Krebs-Mittel und/oder einem in der Immuntherapie verwendeten Anti-Krebs-Mittel, verwendet wird.

**9.** Antikörper, der gegen das CD303-Protein gerichtet ist, zur Verwendung nach einem der Ansprüche 1 bis 8, wobei das chemische Anti-Krebsmittel gewählt ist aus anti-metabolischen Mitteln, alkylierenden Mitteln, interkalierenden Mitteln oder Molekülen mit einer Wirkung auf die mitotische Spindel, und vorzugsweise:

- wobei die genannten metabolischen Mittel gewählt sind aus:

  - Antifolaten, insbesondere Methotrexat, Raltitrexed und Pemetrexed),
  - Antipurinen, insbesondere Mercaptopurin, Thioguanin, Pentostatin, Cladribin und Fludarabin,
  - Antipyrimidinen, insbesondere 5-Fluorouracil, Tegafur-Uracil, Capecitabin und Cytarabin, und
  - anti-metabolischen Mitteln, insbesondere Hydroxycarbamid, Hydroxyharnstoff und Gemcitabin,

- wobei die Alkylierungsmittel gewählt sind aus:

  - Stickstoffsenf, insbesondere Chlorambucil, Melphelan, Chlormethin, Metachloroethamin, Estramustin, Ifosfamid und Cyclophosphamid,
  - Nitrosoharnstoffen, insbesondere Fotemustin, Lomustin, Carmustin, Streptozocin,
  - Organoplatinen, insbesondere Carboplatin, Cisplatin und Oxaliplatin,
  - Ethyleniminen, insbesondere Thiotepa und Altretamin,
  - Triazenen, insbesondere Procarbazin, Temozolomid und Dacarbazin,
  - Alkylierungsmittel, insbesondere Busulfan, Mitomycin C und 20 Pipobroman,

- wobei die Interkalierungsmittel gewählt sind aus:

  - Camptothecinderivaten, insbesondere Irinotecan und Topotecan,
  - Antrhracyclinen, insbesondere Epirubicin, Daunorubicin, Doxorubicin, Pirarubicin und Idarubicin,
  - Interkalationsmitteln, insbesondere Mitoxantron, Amsacrin, Elliptinium, Actinomycin d, Dactinomycin, Etoposid und Bleomycin,

- wobei die Moleküle, die eine Wirkung auf die mitotische Spindel haben, gewählt sind aus:

  - Vinca-Alkaloiden oder Spindelgiften, insbesondere Vinorelbin, Vindesin, Vincristin und Vinblastin,
  - Taxoiden oder Spindelstabilisatoren, insbesondere Paclitaxel und Docetaxel,
  - Tyrosinkinase-Inhibitoren, insbesondere Dasatinib, Erlotinib, Imatinib, Sorafenib und Sunitinib.

**10.** Antikörper, der gegen das CD303-Protein gerichtet ist, zur Verwendung gemäß Anspruch 9, wobei das Antikrebsmittel zur Verwendung in der Immuntherapie ein zieltumorspezifischer Antikörper, ein Anti-CD123-Antikörper oder ein TLR-Agonist ist.

**11.** Antikörper, der gegen das CD303-Protein gerichtet ist, zur Verwendung nach einem der Ansprüche 8 bis 9, wobei

die Verwendung des Antikörpers und die Verwendung des Antikrebsmittels gleichzeitig, getrennt oder zeitlich abgestimmt erfolgen.

**12.** Antikörper, der gegen das CD303-Protein gerichtet ist, zur Verwendung nach einem der Ansprüche 1 bis 11, wobei die Verwendung mit einer Strahlentherapie gekoppelt ist.

**13.** Antikörper, der gegen das CD303-Protein gerichtet ist, zur Verwendung nach einem der Ansprüche 1 bis 12, wobei die Vorbeugung oder Behandlung bei einem Patienten mit plasmazytoider dendritischer Zelldepletion oder bei einem Patienten, der eine plasmazytoide dendritische Zelldepletion benötigt oder erfordert, durchgeführt wird.

**14.** Produkt, umfassend:

- einen Antikörper, insbesondere einen monoklonalen oder polyklonalen Antikörper, der gegen das in einem der Ansprüche 5 bis 7 definierte CD303-Protein gerichtet ist; und
- wenigstens ein Anti-Krebs-Mittel, insbesondere ein chemisches Anti-Krebs-Mittel und/oder ein in der Immuntherapie verwendetes Anti-Krebs-Mittel,

als Kombinationsprodukt zur gleichzeitigen, getrennten oder zeitlich abgestimmten Verwendung, zur Verwendung bei der Prävention oder Behandlung eines Tumors, die eine Aktivierung von plasmazytoiden dendritischen Zellen in der Umgebung des Tumors beinhaltet, wobei die plasmazytoiden dendritischen Zellen immunsuppressive und/oder tolerogene Eigenschaften aufweisen, nicht aus dem Tumor stammen und keinen CD56+-Marker aufweisen.

**Claims**

**1.** Antibody, in particular monoclonal or polyclonal, directed against the CD303 protein for use in preventing or treating a tumour involving activation of plasmacytoid dendritic cells in the environment of said tumour, said plasmacytoid dendritic cells having immunosuppressive and/or tolerogenic properties, and not being the cause of the tumour and not having the CD56+ marker.

**2.** Antibody directed against the CD303 protein for use according to claim 1, wherein the tumours involving an activation of the plasmacytoid dendritic cells are solid tumours or haematopoietic tumours.

**3.** Antibody directed against the CD303 protein for use according to claim 2, wherein said solid tumours involve an infiltration of plasmacytoid dendritic cells in the environment of said tumour, and preferably belong to the group of tumours of the head or neck, melanoma, urogenital cancers or breast cancer.

**4.** Antibody directed against the CD303 protein for use according to claim 2, wherein said haematopoietic tumours belonging to the group of multiple myeloma, lymphoma, or leukaemia, in particular T-cell leukaemia.

**5.** Antibody directed against the CD303 protein for use according to any one of claims 1 to 4, wherein said antibody is selected from a murine antibody, a chimeric antibody, a humanised antibody or a human antibody, in particular a chimeric antibody and preferably a chimeric antibody selected from a murine/human chimeric antibody or a human/macaque chimeric antibody.

**6.** Antibody directed against the CD303 protein for use according to any one of claims 1 to 5, wherein the antibody has a low fucose content, less than or equal to 65%, and/or an N-glycan content of the oligomannose type greater than or equal to 30%, and/or a galactose content greater than or equal to 50%.

**7.** Antibody directed against the CD303 protein, for use according to any one of claims 1 to 6, wherein said antibody is coupled to a bioactive molecule selected from:

- radioisotopes, in particular selected from At211, 1131, 1125, Y90, Re186, Re188, Sm153, Bi212, P32,
- non-radioactive metals,
- toxins, in particular selected from ricin, abrin and diphtheric toxin,
- nucleic acids, in particular selected from anti-sens RNAs,
- enzymes, in particular selected from RNases, biotin, avidin or streptavidin,
- cytotoxic agents, in particular selected from:

- antifolates, more particularly methotrexate, pemetrexed and raltitrexed;
- antipurines, and more particularly cladribine, fludarabine, azathioprine, azathioprine, mercaptopurine, 5-fluorouracil, capecitabine, cytarabine, gemcitabine,
- topoisomerase I and II inhibitors,
- alkylising agents and similar agents, and more particularly chlormethine, cyclophosphamide, ifosfamide, carmustine, fotemustine, mitomycin C, cisplatin, carboplatin, oxaliplatin,
- intercalating agents,
- anthracyclines, and more particularly selected from daunorubicin, doxorubicin and chlorydrate, epirubicin, idarubicin, bleomycin,
- taxans,
- specific inhibitors of tyrosine-kinase, imatinib and erlotinib.

**8.** Antibody directed against the CD303 protein, for use according to any one of claims 1 to 7, wherein said antibody is used in combination with at least one anticancer agent, in particular a chemical anticancer agent and/or an anticancer agent used in immunotherapy.

**9.** Antibody directed against the CD303 protein, for use according to any one of claims 1 to 8, wherein said chemical anticancer agent is selected from antimetabolic agents, alkylising agents, intercalating agents, or molecules having an action on the mitotic spindle, and preferably:

- said metabolic agents are selected from:

  - antifolics, in particular methotrexate, raltitrexed, and pemetrexed),
  - antipurics, in particular mercaptopurine, thioguanine, pentostatin, cladribine and fludarabine,
  - antipyrimidics, in particular 5-fluoro-uracil, tegafur uracil, capecitabine and cytarabine, and
  - antimetabolics, in particular hydroxycarbamide, hydroxyurea and gemcitabine,

- said alkylising agents being selected from:

  - nitrogen mustards, in particular chlorambucil, melphelan, chlormethine, metachloroethamine, estramustine, ifosfamide and cyclophosphamide,
  - nitrosoureas, in particular fotemustine, lomustine, carmustine, streptozocin,
  - organoplatinums, in particular carboplatinum, cisplatinum and oxaliplatinum,
  - ethylene imines, in particular thiotepa and altretamine,
  - trazenes, in particular procarbazine, temozolomide and dacarbazine,
  - alkylising agents, in particular busulfan, mitomycin C and pipobroman,

- said intercalating agents are selected from:

  - derivatives of camptothecin, in particular irinotecan and topotecan,
  - anthracyclines, in particular epirubicin, daunorubicin, doxorubicin, pirarubicin and idarubicin,
  - intercalating agents, in particular mitoxantrone, amsacrine, elliptinium, actinomycin d, dactinomycin, etoposide and bleomycin,

- said molecules having an action on the mitotic spindle are selected from:

  - vinca alkaloids or spindle poisons, in particular vinorelbine, vindesine, vincristine and vinblastine,
  - taxoids or spindle stabilisers, in particular paclitaxel and docetaxel,
  - tyrosine kinase inhibitors, in particular dasatinib, erlotinib, imatinib, sorafenib and sunitinib.

**10.** Antibody directed against the CD303 protein, for use according to claim 9, wherein said anticancer agent used in immunotherapy is a specific antibody of the targeted tumour, an anti-CD123 antibody or a TLR agonist.

**11.** Antibody directed against the CD303 protein, for use according to either one of claims 8 to 9, wherein said use of said antibody and said use of said anticancer agent are simultaneous, separate or spread over time.

**12.** Antibody directed against the CD303 protein, for use according to any one of claims 1 to 11, wherein said use is coupled with radiotherapy.

**13.** Antibody directed against the CD303 protein, for use according to any one of claims 1 to 12, wherein said prevention or said treatment is implemented in a patient having plasmacytoid dendritic cell depletion, or in a patient needing or requiring plasmacytoid dendritic cell depletion.

**14.** Product containing:

- an antibody, in particular monoclonal or polyclonal, directed against the CD303 protein as defined in one of claims 5 to 7; and
- at least one anticancer agent, in particular a chemical anticancer agent and/or an anticancer agent used in immunotherapy,

as combination product for use that is simultaneous, separate or spread over time, for use in preventing or treating a tumour involving activation of plasmacytoid dendritic cells in the environment of said tumour, said plasmacytoid dendritic cells having immunosuppressive and/or tolerogenic properties, and not being the cause of the tumour and not having the CD56+ marker.

A

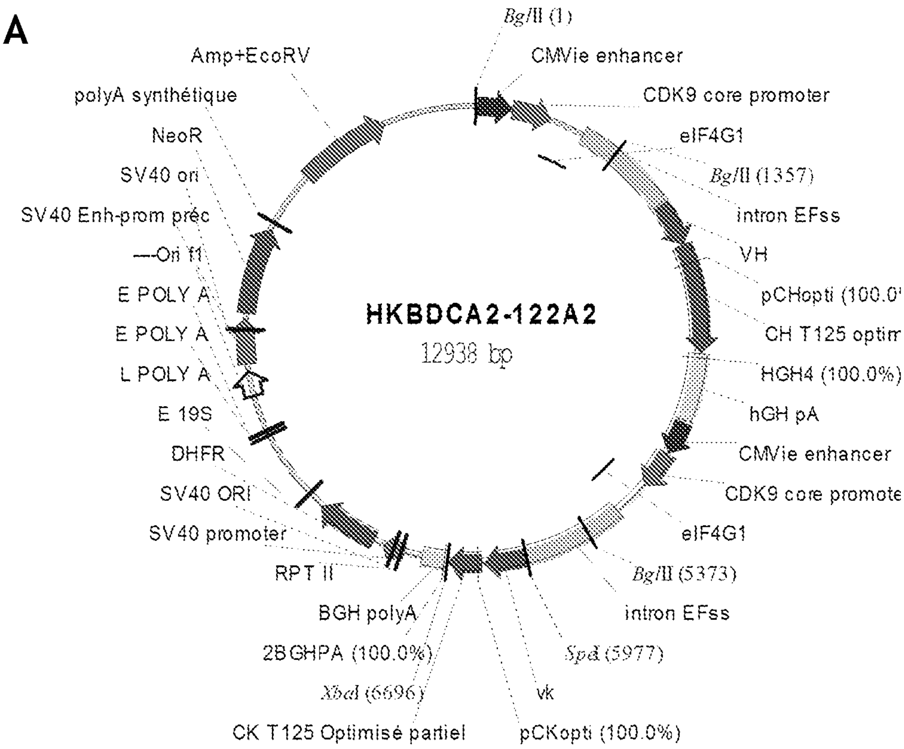


B

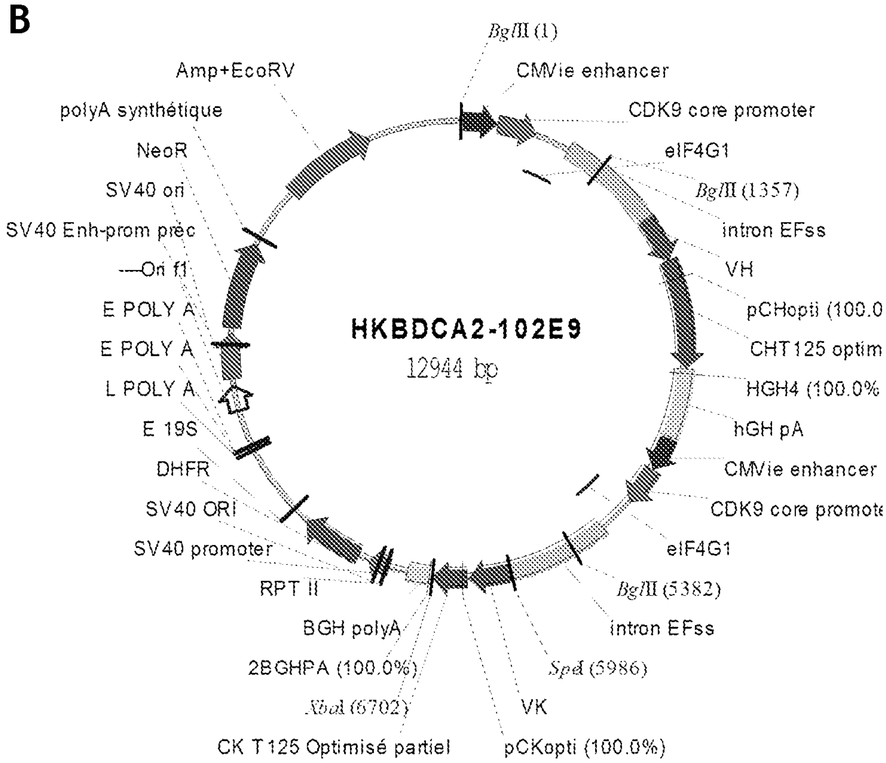


**Figures 1A et 1B**

C

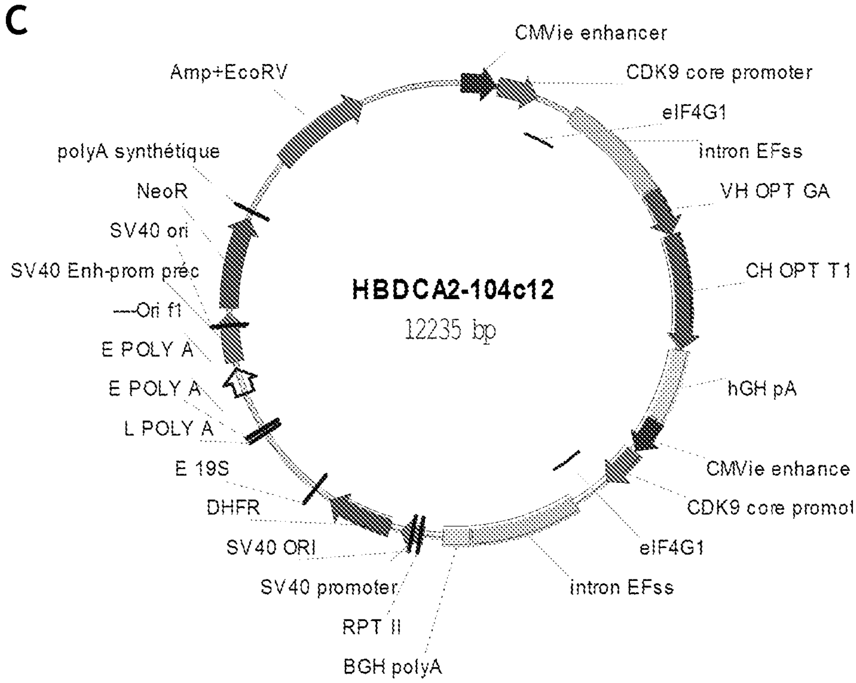


D

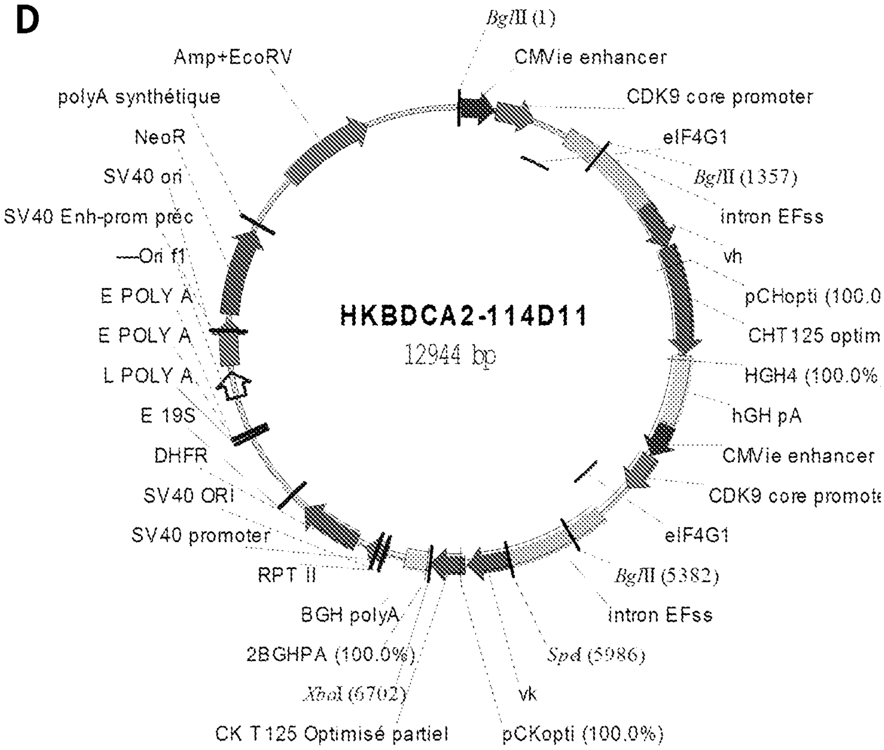


**Figures 1C et 1D**

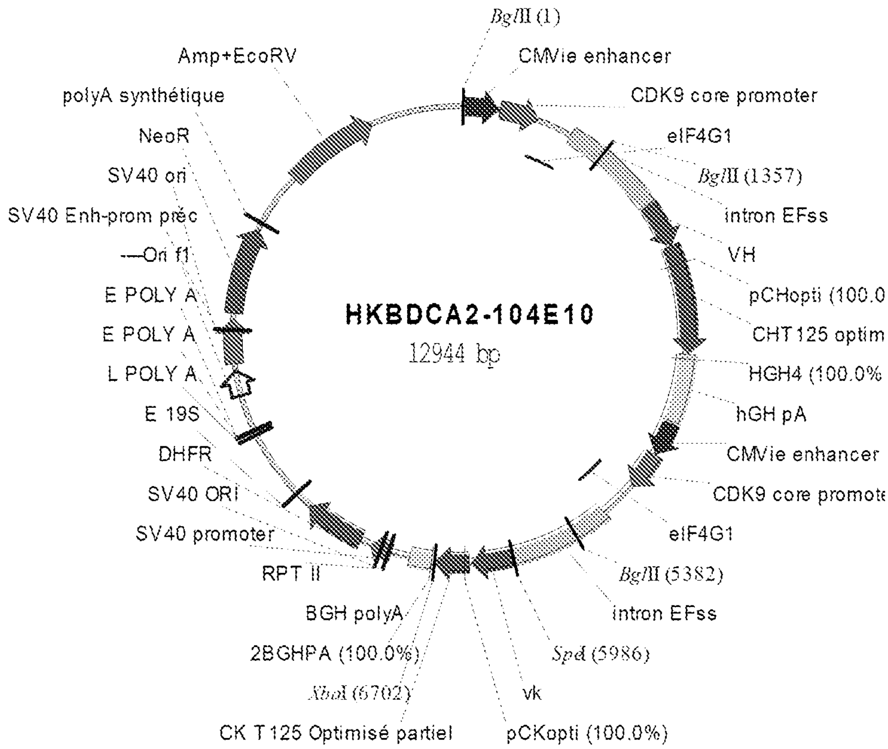
BglII (1)
CMVie enhancer
CDK9 core promoter
eIF4G1
BglII (1357)
intron EFss
VH
pCHopti (100.0
CHT125 optim
HGH4 (100.0%
hGH pA
CMVie enhancer
CDK9 core promote
eIF4G1
BglII (5382)
intron EFss
SpeI (5986)
vk
pCKopti (100.0%)
CK T125 Optimisé partiel
XbaI (6702)
2BGHPA (100.0%)
BGH polyA
RPT II
SV40 promoter
SV40 ORI
DHFR
E 19S
L POLY A
E POLY A
E POLY A
Ori f1
SV40 Enh-prom préc
SV40 ori
NeoR
polyA synthétique
Amp+EcoRV
HKBDCA2-104E10
12944 bp

**Figure 1E**

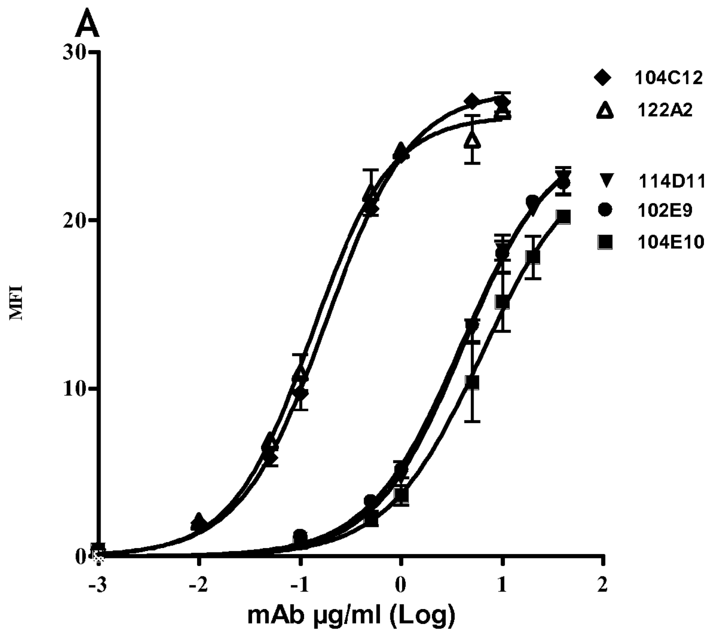


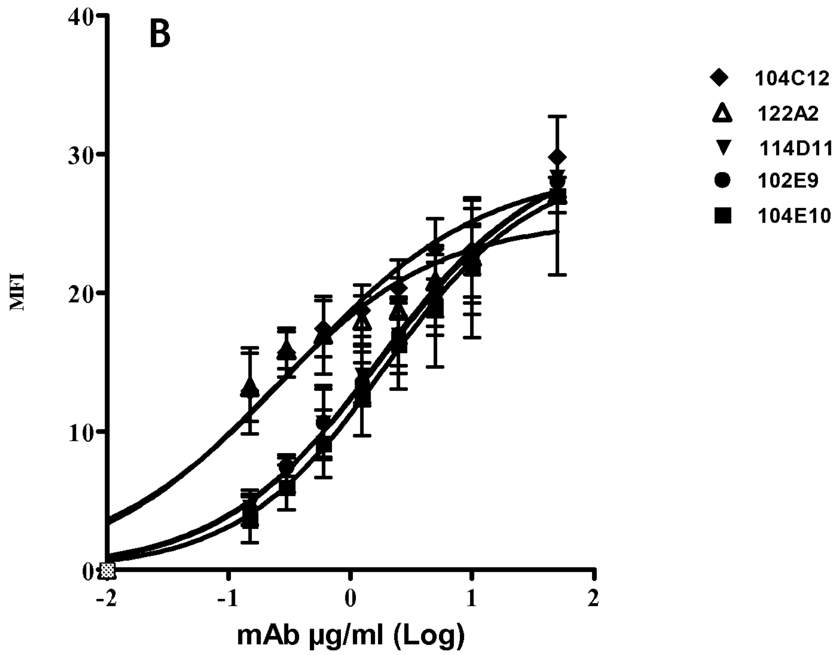


Figures 2A et 2B

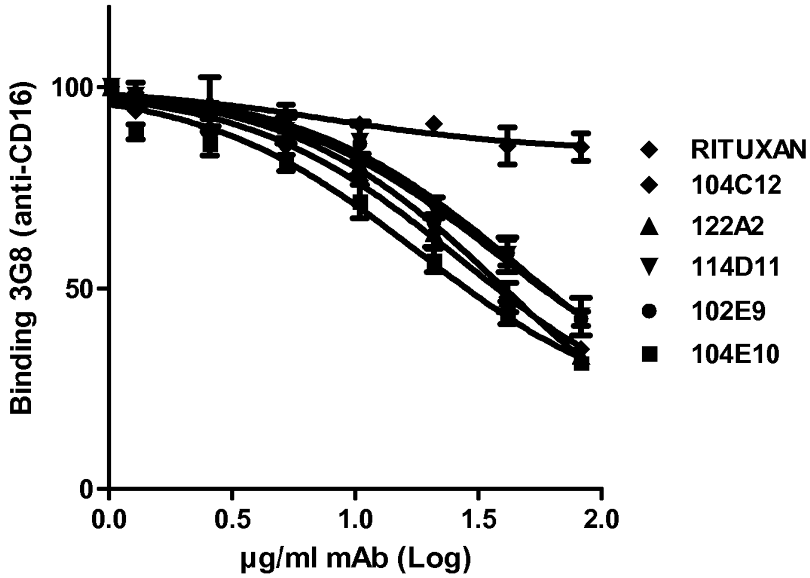


Figure 3

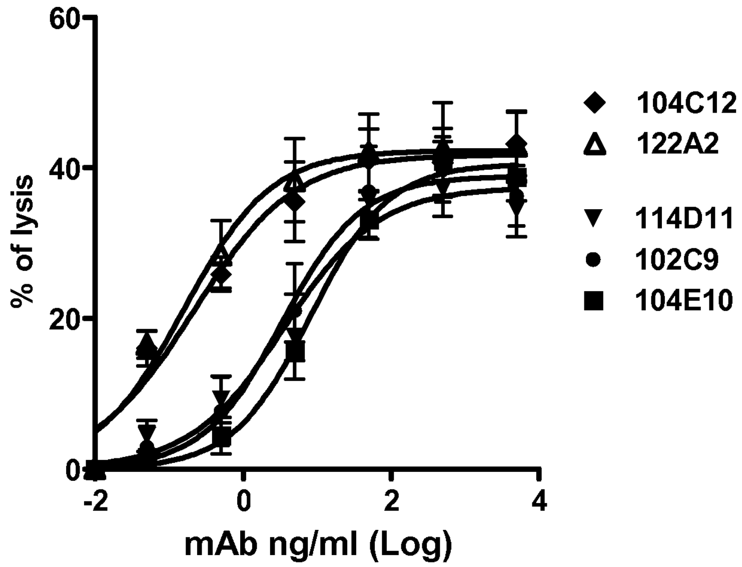


Figure 4

Figure 5

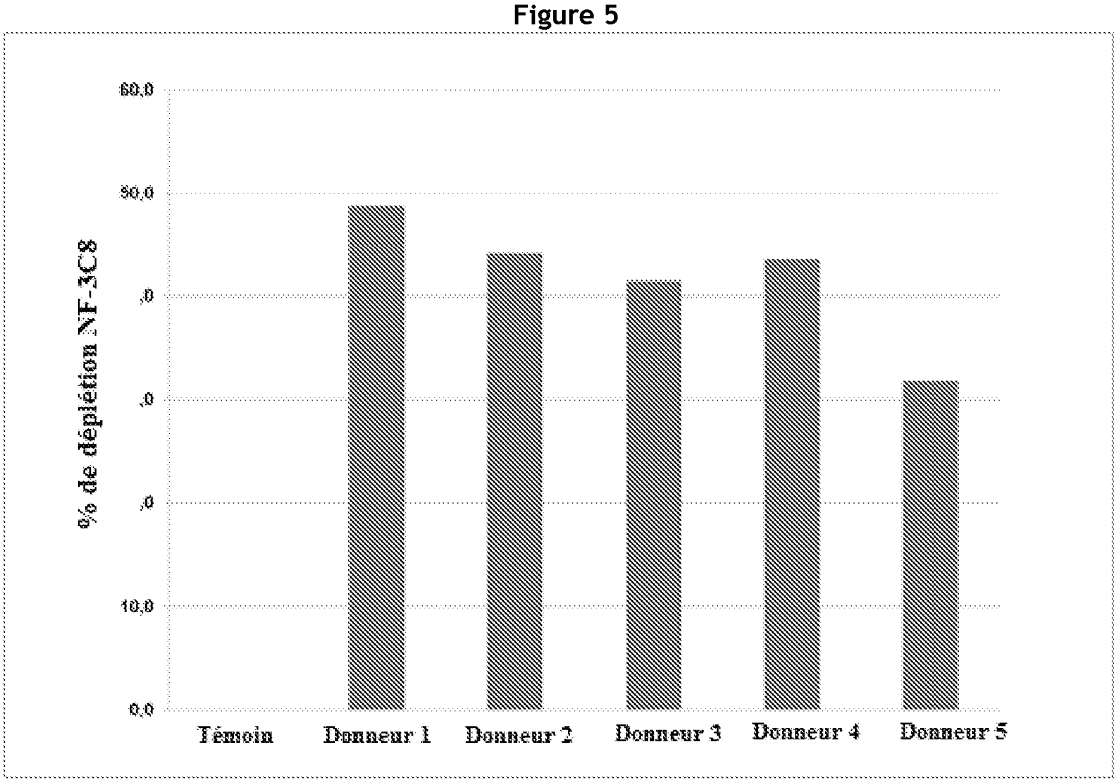
60,0
50,0
,0
,0
,0
10,0
0,0
% de déplétion NF-3C8
Témoin
Donneur 1
Donneur 2
Donneur 3
Donneur 4
Donneur 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1783141 A
- WO 2006037247 A
- WO 0136487 A
- WO 2012080642 A
- WO 0042072 A
- WO 2004029207 A
- WO 2004063351 A
- WO 2004074455 A
- WO 02060919 A
- WO 2010045193 A
- WO 2010106180 A
- WO 9951642 A
- WO 2011114063 A
- WO 2007048077 A
- EP 1176195 A1
- WO 0177181 A
- WO 2012041768 A
- WO 2008028686 A
- WO 0026357 A
- WO 9004036 A
- WO 9517085 A
- WO 0126455 A
- WO 2004050847 A
- WO 2005033281 A
- WO 2007106078 A
- FR 1562545


### Littérature non-brevet citée dans la description


- **NESTLE et al.** *J. Exp. Med,* 2005, vol. 202 (1), 135-143
- **BLOMBERG et al.** *Arthritis and Rheumatism,* 2003, vol. 48 (9), 2524-2532
- **KABAT et al.** Sequences of Proteins of Immunological Interest. Public Health Service, National Institutes of Health, 1991
- Manipulating the Mouse Embryo, A Laboratory Manual. Cold Spring Harbor Laboratory Press, 1994
- Gene Targeting, A Practical Approach. IRL Press at Oxford University Press, 1993
- **MAEDA T et al.** *Int J Hematol.,* Février 2005, vol. 81 (2), 148-54
- **MAEDA T et al.** *Int J Hematol.,* 08 Février 2005, vol. 1 (2), 148-54